# FASCICULE DE BREVET EUROPEEN

(11) **EP 1 263 722 B1**
(45) Date de publication et mention de la délivrance du brevet: **25.08.2010**
(21) Numéro de dépôt: 01909939.9
(22) Date de dépôt: 01.03.2001
(51) Int. Cl.: C07D 205/04, C07D 401/12, C07D 403/12, C07D 417/12, A61K 31/397, A61P 25/00

(54) **COMPOSITIONS PHARMACEUTIQUES CONTENANT DES DERIVES D'AZETIDINE, LES NOUVEAUX DERIVES D'AZETIDINE ET LEUR PREPARATION**
AZETIDINDERIVATE ENTHALTENDE PHARMAZEUTISCHE ZUSAMMENSETZUNGEN, DIE AZETIDINDERIVATE UND DEREN HERSTELLUNG
PHARMACEUTICAL COMPOSITIONS CONTAINING AZETIDINE DERIVATIVES, NOVEL AZETIDINE DERIVATIVES AND PREPARATION THEREOF

(30) Priorité: 03.03.2000 FR 0002776
(43) Date de publication de la demande: 11.12.2002
(73) Titulaire: Aventis Pharma S.A., 92160 Antony (FR)
(72) Inventeur: ACHARD, Daniel, F-94320 Thiais (FR); BOUCHARD, Hervé, F-94320 Thiais (FR); BOUQUEREL, Jean, F-93700 Drancy (FR); FILOCHE, Bruno, F-94000 Créteil (FR); GRISONI, Serge, F-94600 Choisy le Roi (FR); HITTINGER, Augustin, F-91430 Igny (FR); MYERS, Michael, Fishers, Indiana 46038 (US)
(74) Mandataire: Le Coupanec, Pascale A.M.P.
(86) Numéro de dépôt international: PCT/FR2001/000602
(87) Numéro de publication internationale: WO 2001/064634

(56) Documents cités:
- EP-A- 0 406 112
- WO-A-97/01556
- WO-A-99/01451
- US-A- 4 242 261

## Description

La présente invention concerne des compositions pharmaceutiques contenant comme principe actif au moins un composé de formule : 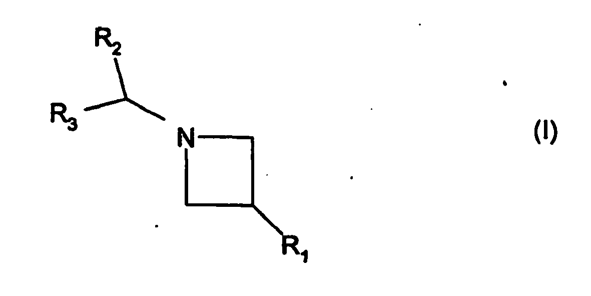 ou un de ses sels pharmaceutiquement acceptables, les nouveaux dérivés de formule (I), leurs sels pharmaceutiquement acceptables et leur préparation.

Le composé de formule (I) pour lequel R₂ et R₃ représentent des radicaux phényle, R₁ représente un radical -N(R₄)SO₂R₆, R₄ représente un radical phényle et R₆ représente un radical méthyle est décrit comme intermédiaire de synthèse dans le brevet WO99/01451. Les autres composés et leurs sels pharmaceutiquement acceptables sont nouveaux et en tant que tels font partie de l'invention.

Dans la formule (I)
R₁ représente un radical -N(R₄)R₅, -N(R₄)CO-R₅, -N(R₄)-SO₂R₆,
R₂ et R₃, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, COOalk, -CONR₇R₈, -CO-NH-NR₉R₁₀, alkylsulfanyle, alkylsulfinyle, alkylsulfonyle, alkylsulfanylalkyle, alkylsulfinylalkyle, alkylsulfonylalkyle, hydroxyalkyle ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle, isochromannyle, isoquinolyle, pyrrolyle, pyridyle, pyrimidyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, COOalk, -CO-NH-NR₉R₁₀, -CONR₇R₈, -alk-NR₉R₁₀, alkylsulfanyle, alkylsulfinyle, alkylsulfonyle, alkylsulfanylalkyle, alkylsulfinylalkyle, alkylsulfonylalkyle ou hydroxyalkyle ,
R₄ représente un radical -C(R₁₁)(R₁₂)-Het, -Het, -(CR₁₁)(R₁₂)-Ar, Ar, cycloalkyle ou norbornyle,
R₅ représente un atome d'hydrogène ou un radical hydroxyalkyle, -alk-COOalk, -alk-CONR₇R₈, -alk-NR₇R₈, alcoxy, Ar, Het, -CH₂Ar, -CH₂Het ou alkyle éventuellement substitué par un ou plusieurs halogène,
R₆ représente un radical hydroxyalkyle, -alk-COOalk, -alk-CONR₇R₈, -alk-NR₇R₈, alcoxy, Ar, Het, -CH₂Ar, -CH₂Het ou alkyle éventuellement substitué par 1 ou plusieurs halogène,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R_{10'} identiques ou différents, représentent un atome d'hydrogène ou un radical alkyle, -COOalk, cycloalkyle, alkylcycloalkyle, -alk-O-alk, hydroxyalkyle
ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₁₁ représente un atome d'hydrogène ou un radical hydroxyalkyle, -alk-COOalk, -alk-CONR₇R₈, -alk-NR₇R₈, alcoxyalkyle, Ar, Het, -CH₂Ar, -CH₂Het ou alkyle éventuellement substitué par un ou plusieurs halogène, R₁₂ représente un atome d'hydrogène ou un radical hydroxyalkyle, -alk-COOalk, -alk-CONR₇R₈, -alk-NR₇R₈, alcoxyalkyle ou alkyle éventuellement substitué par un ou plusieurs halogène,
ou bien R₁₁ et R₁₂ forment ensemble avec l'atome de carbone auquel ils sont rattachés un cycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle, naphtyle ou indènyle, ces différents radicaux étant éventuellement substitués par un ou plusieurs halogène, alkyle, alcoxy, -CO-alk, cyano, -COOH, -COOalk, -CONR₁₃R₁₄, -CO-NH-NR₁₅R₁₆, alkylsulfanyle, alkylsulfinyle, alkylsulfonyle, -alk-NR₁₅R₁₆, -NR₁₅R₁₆, alkylthioalkyle, formyle, CF₃, OCF₃, Het, -O-alk-NH-cycloalkyle, SO₂NH₂, hydroxy, hydroxyalkyle, -NHCOalk, NHCOOalk ou sur 2 atomes de carbone adjacents par dioxyméthylène,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, halogène, alcoxycarbonyle, oxo, hydroxy, les hétérocycles azotés étant éventuellement sous leur forme N-oxydée,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
alk représente un radical alkyle ou alkylène.

Dans les définitions précédentes et celles qui suivent, sauf mention contraire, les radicaux et portions alkyle et alkylène et les radicaux et portions alcoxy sont en chaîne droite ou ramifiée et contiennent 1 à 6 atomes de carbone et les radicaux cycloalkyle contiennent 3 à 10 atomes de carbone.

Parmi les radicaux alkyle on peut citer les radicaux méthyle, éthyle, n-propyle, isopropyle, n-butyle, sec-butyle, iso-butyle, tert-butyle, pentyle, hexyle. Parmi les radicaux alcoxy on peut citer les radicaux méthoxy, éthoxy, n-propoxy, iso-propoxy, n-butoxy, iso-butoxy, sec-butoxy, tert-butoxy, pentyloxy.

Parmi les radicaux cycloalkyle, on peut citer les radicaux cyclopropyle, cyclobutyle, cyclopentyle, cyclohexyle.

Le terme halogène comprend chlore, fluor, brome et iode.

Parmi les hétérocycles représentés par Het, on peut citer les hétérocycles suivants : benzimidazole, benzoxazole, benzothiazole, benzothiophène, cinnoline, thiophène, quinazoline, quinoxaline, quinoline, pyrazole, pyrrole, pyridine, imidazole, indole, isoquinoline, pyrimidine, thiazole, thiadiazole, pipéridine, pipérazine, triazole, furane, tétrahydroisoquinoline, tétrahydroquinoline, ces hétérocycles étant éventuellement substitués par un ou plusieurs alkyle, alcoxy, halogène, alcoxycarbonyle, oxo, hydroxy, OCF₃ ou CF₃.

Les composés de formule (I) peuvent se présenter sous forme d'énantiomères et de diastéréoisomères. Ces isomères et leurs mélanges font également partie de l'invention.

De façon préférentielle, les composés de formule (I) sont ceux pour lesquels
R₁ représente un radical -N(R₄)R₅, -N(R₄)-SO₂R₆,
R₂ représente soit un phényle non substitué ou substitué par un ou plusieurs halogène, alkyle, alcoxy, trifluorométhyle, trifluorométhoxy, -CO-alk, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle ,
R₃ représente soit un phényle non substitué ou substitué par un ou plusieurs halogène, alkyle, alcoxy, trifluorométhyle, trifluorométhoxy, -CO-alk, cyano, -CONR₇R₈, hydroxyalkyle ou -alk-NR₇R₆; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle ,
R₄ représente un radical -C(R₁₁)(R₁₂)-Het, -Het, -C(R₁₁)(R₁₂)-Ar, Ar ou norbomyle,
R₅ représente un atome d'hydrogène ou un radical hydroxyalkyle, -alk-COOalk, -alk-CONR₇R₈, -alk-NR₇R₈, alcoxy, -CH₂Ar, -CH₂Het ou alkyle,
R₆ représente un radical hydroxyalkyle, -alk-COOalk, -alk-CONR₇R₈, -alk-NR₇R₈, alcoxy, -CH₂Ar, -CH₂Het ou alkyle,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, cycloalkyle, alkylcycloalkyle, -alk-O-alk ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, oxo, hydroxyalkyle ou -CO-NH₂,
R₁₁ représente un atome d'hydrogène ou un radical hydroxyalkyle, -alk-COOalk, -alk-CONR₇R₈, -alk-NR₇R₈, alcoxyalkyle, Ar, Het, -CH₂Ar, -CH₂Het ou alkyle éventuellement substitué par un ou plusieurs halogène,
R₁₂ représente un atome d'hydrogène ou un radical hydroxyalkyle, -alk-COOalk, -alk-CONR₇R₈, -alk-NR₇R₈, alcoxyalkyle ou alkyle éventuellement substitué par un ou plusieurs halogène,
ou bien R₁₁ et R₁₂ forment ensemble avec l'atome de carbone auquel ils sont rattachés un cycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle ou naphtyle, ces différents radicaux étant éventuellement substitués par un ou plusieurs halogène, alkyle, alcoxy, -CO-alk, cyano, -CONR₁₃R₁₄, alkylsulfonyle, -alk-NR₁₅R₁₆, -NR₁₅R₁₆, CF₃, OCF₃, SO₂NH₂, hydroxy, hydroxyalkyle ou sur 2 atomes de carbone adjacents par dioxyméthylène,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, halogène, oxo, hydroxy, les hétérocycles azotés étant éventuellement sous leur forme N-oxydée et, plus particulièrement, Het représente un hétérocycle choisi parmi benzimidazole, benzoxazole, benzothiazole, benzothiophène, thiophène, quinazoline, quinoxaline, quinoline, pyrrole, pyridine, imidazole, indole, isoquinoline, pyrimidine, thiazole, thiadiazole, furane, tétrahydroisaquinoline et tétrahydroquinoline, ces hétérocycles étant éventuellement substitués par un ou plusieurs alkyle, alcoxy, halogène, alcoxycarbonyle, oxo, hydroxy, OCF₃ ou CF₃.
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Encore plus préférentiellement, les composés de formule (I) sont choisis parmi les composés suivants :
R₁ représente un radical -N(R₄)-SO₂R₆,
R₂ représente soit un phényle non substitué ou substitué par un ou plusieurs halogène, alkyle, alcoxy, trifiuorométhyle, trifluorométhoxy, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈ ou hydroxyalkyle ,
R₃ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈ ou hydroxyalkyle ,
R₄ représente -Het ou Ar,
R₆ représente un radical hydroxyalkyle ou alkyle,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle ou naphtyle, ces différents radicaux étant éventuellement substitués par un ou plusieurs halogène, alkyle, alcoxy, -CO-alk, cyano, -CONR₁₃R₁₄, -alk-NR₁₅R₁₆, -NR₁₅R₁₆, CF₃, OCF₃, SO₂NH₂, hydroxy ou hydroxyalkyle,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, halogène, oxo, hydroxy et plus particulièrement, Het représente un hétérocycle choisi parmi benzimidazole, benzoxazole, benzothiazole, benzothiophène, thiophène, quinazoline, quinoxaline, quinoline, pyrrole, pyridine, imidazole, indole, isoquinoline, thiazole, thiadiazole, furane, tétrahydroisoquinoline et tétrahydroquinoline, ces hétérocycles étant éventuellement substitués par un ou plusieurs alkyle, alcoxy, halogène, alcoxycarbonyle, oxo, hydroxy, OCF₃ ou CF₃,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, Identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle.

Parmi les composés préférés, on peut citer les composés suivants :
N-{1-[bis-(4-chlorophényl)méthyl]-azétidin-3-yl}-N-(6-chloropyrid-2-yl)-méthylsulfonamide,
N-{1-[bis-(4-chlorophényl)méthyl]azétidin-3-yl}-N-(6-éthylpyrid-2-yl)-méthylsulfonamide,
N-{1-[bis-(4-chlorophényl)méthyl]azétidin-3-yl}-N-quinol-6-yl-méthylsulfonamide,
N-{1-[bis-(4-chlorophényl)méthyl]azétidin-3-yl}-N-quinol-5-yl-méthylsulfonamide,
N-{1-[bis-(4-chlorophényl)méthyl]azétidin-3-yl}-N-isoquinol-5-yl-méthylsulfonamide,
N-{1-[bis-(4-chlorophényl)méthyl]azétidin-3-yl}-N-pyrid-3-yl-méthylsulfonamide,
N-{1-[bis-(4-chlomphényl)méthyl]azétidin-3-yl}-N-(1-oxyde-pyrid-3-yl)-méthylsulfonamide,
N-(1R,2S,4S)-bicyclo[2,2,1]hept-2-yl-N-{1-[bis-(4-chlorophényl)méthyl] azétidin-3-yl}-méthylsulfonamide,
N-(1R,2R,4S)-bicyclo[2,2,1]hept-2-yl-N-{1-[bis-(4-chlorophényl)méthyl] azétidin-3-yl}-méthylsulfonamide,
N-{1-[bis-(4-chlorophényl)méthyl]azétidin-3-yl}-N-(3,5-difluorophényl)-méthylsulfonamide,
N-{1-[bis-(4-chlorophényl)méthyl]azétdin-3-yl}-N-(thiazol-2-yl)-méthyl sulfonamide,
N-{1-[bis-(4-chlorophényl)-méthyl]-azétidin-3-yl}-N-(3-méthoxyphényl)-méthylsulfonamide,
N-{1-[bis-(4-chiorophényl)-méthyl]-azétidin-3-yl}-N-(3-hydroxyphényl)-méthylsulfonamide,
N-{1-[bis-(4-chlorophényl)-méthyl]-azétidin-3-yl}-N-(3-hydroxyméthyl-phényl)-méthylsulfonamide,
N-{1-[bis-(4-chlorophényl)-méthyl]-azétidin-3-yl}-N-(méthylsulfonyl)-3-aminobenzoate d'éthyle,
N-{1-[bis-(4-chlorophényl)méthyl]azétidin-3-yl}-N-(1-isobutyl-pipérid-4-yl)-méthylsulfonamide,
N-benzyl-N-{1-[bis(4-chlorophényl)méthyl]azétidin-3-yl}amine,
N-{1-[bis(4-chlorophényl)méthyl]azétidin-3-yl}-N-(3,5-difluorobenzyl)amine,
N-{1-[bis(4-chlorophényl)méthyl]azétidin-3-yl}-N-(3,5-difluorobenzyl)méthylsulfonamide,
N-{1-[bis(4-chlorophényl)méthyl]azétidin-3-yl}-N-(pyrid-3-yl-méthyl)-méthylsulfonamide,
N-{1-[bis-(4-fluoro-phényl)-méthyl]-azétidin-3-yl}-N-(3,5-difluorophényl)-méthylsulfonamide,
(RS)-N-{1-[(4-chlorophényl)-pyrid-3-yl-méthyl]-azétidin-3-yl}-N-(3,5-difluorophényl)-méthylsulfonamide,
(R)-N-{1-[(4-chlorophényl)-pyrid-3-yl-méthyl]-azétidin-3-yl}-N-3,5-difluorophényl)-méthylsulfonamide,
(S)-N-{1-[(4-chlorophényl)-pyrid-3-yl-méthyl]-azétidin-3-yl}-N-(3,5-difluorophényl)-méthylsulfonamide,
(RS)-N-{1-[(4-chlorophényl)-pyrid-4-yl-méthyl]-azétidin-3-yl}-N-(3,5-difluorophényl)-méthylsulfonamide,
(R)-N-{1-[(4-chlorophényl)-pyrid-4-yl-méthyl]-azétidin-3-yl}-N-(3,5-difluorophényl)-méthylsulfonamide,
(S)-N-{1-[(4-chlorophényl)-pyrid-4-yl-méthyl]-azétidin-3-yl}-N-(3,5-difluorophényl)-méthylsulfonamide,
(RS)-N-{1-[(4-chlorophényl)-pyrimidin-5-yl-méthyl]-azétidin-3-yl}-N-(3,5-difluorophényl)-méthylsulfonamide,
(R)-N-{1-[(4-chlorophényl)-pyrimidin-5-yl-méthyl]-azétidin-3-yl}-N-(3,5-difluorophényl)-méthylsulfonamide,
(S)-N-{1-[(4-chlorophényl)-pyrimidin-5-yl-méthyl]-azétidin-3-yl}-N-(3,5-difluorophényl)-méthylsulfonamide,
N-{1-[bis-(4-chlorophényl)méthyl]azétidin-3-yl}-N-(3,5-difluorophényl)-benzylsulfonamide,
leurs isomères optiques et leurs sels pharmaceutiquement acceptables.

Les composés de formule (I) pour lesquels R₁ représente un radical -N(R₄)R₅ dans lequel R₅ est un atome d'hydrogène, -N(R₄)-CO-R₅, -N(R₄)-SO₂R₆, R₄ est un radical -C(R₁₁)(R₁₂)-Ar ou -C(R₁₁)(R₁₂)-Het et R₁₂ est un atome d'hydrogène peuvent être préparés selon le schéma réactionnel suivant :

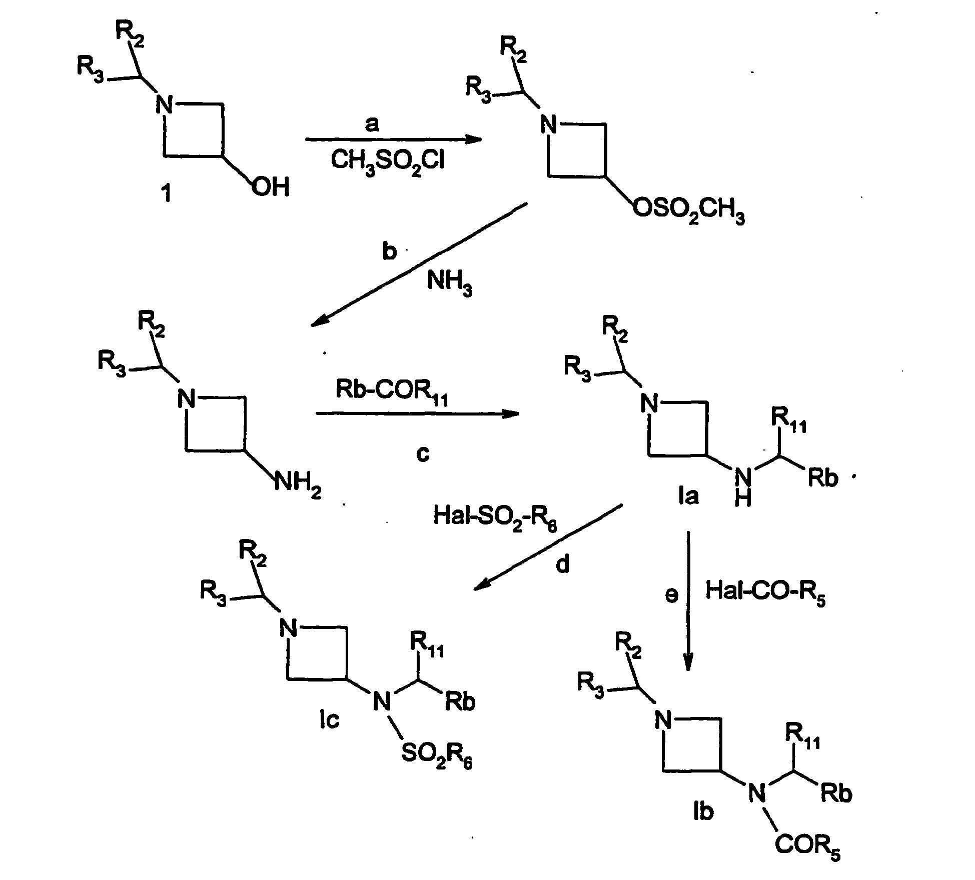

Dans ces formules R₂, R₃, R₆ et R₁₁ ont les mêmes significations que dans la formule (I), Rb représente radical Ar ou Het, Ar et Het ayant les mêmes significations que dans la formule (I) et Hal représente un atome d'halogène et de préférence chlore ou brome.

L'étape a s'effectue généralement au sein d'un solvant inerte tel que le tétrahydrofuranne, le dioxanne, un solvant chloré (dichlorométhane, chloroforme par exemple), à une température comprise entre 15 et 30°C, en présence d'une base telle qu'une trialkylamine (triéthylamine, dipropyléthylamine par exemple) ou au sein de la pyridine, à une température entre 0 et 30°C

L'étape b s'effectue de préférence au sein du méthanol, en autoclave, à une température comprise entre 50 et 70°C.

L'étape c s'effectue généralement au sein d'un solvant inerte tel qu'un solvant chloré (dichlorométhane par exemple), en présence de triacétoxyborohydrure de sodium et d'acide acétique, à une température voisine de 20°C

Les étapes d et e s'effectuent généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

Les dérivés Rb-COR₁₁ sont commercialisés ou peuvent être obtenus selon les méthodes décrites par exemple par R.C. LAROCK, Coprehensive Organic Transformations, VCH editor.

Les dérivés Hal-SO₂R₆ sont commercialisés ou peuvent être obtenus par halogénation des acides sulfoniques correspondants, notamment in situ en présence de chlorosulfonylisocyanate et d'alcool, au sein d'un solvant halogéné (dichlorométhane, chloroforme par exemple).

Les dérivés Hal-COR₅ sont commercialisés ou peuvent être préparés par halogénation des acides carboxyliques correspondants, notamment in situ en présence de chlorure de thionyle au sein d'un solvant halogéné (dichlorométhane, chloroforme par exemple).

Les azétidinols 1 peuvent être obtenus par application ou adaptation des méthodes décrites par KATRITZKY A.R et coll., J. Heterocycl. Chem., 271 (1994) ou DAVE P.R., J. Org. Chem., 61, 5453 (1996) et dans les exemples. On opère généralement selon le schéma réactionnel suivant : 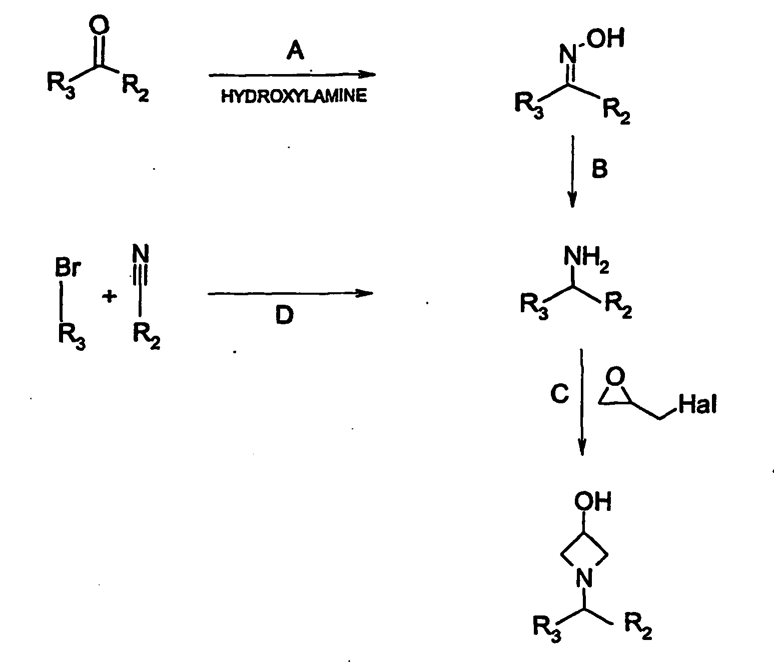 dans ces formules R₂ et R₃ ont les mêmes significations que dans la formule (I) et Hal représente un atome de chlore ou de brome.

Dans l'étape A, on opère de préférence au sein d'un solvant inerte tel qu'un alcool aliphatique 1-4C (éthanol, méthanol par exemple), éventuellement en présence d'un hydroxyde de métal alcalin, à la température d'ébullition du milieu réactionnel.

Dans l'étape B, la réduction s'effectue généralement, au moyen d'hydrure de lithium et d'aluminium, au sein du tétrahydrofuranne à la température d'ébullition du milieu réactionnel.

Dans l'étape C, on opère de préférence au sein d'un solvant inerte tel qu'un alcool aliphatique 1-4C (éthanol, méthanol par exemple), en présence d'hydrogénocarbonate de sodium, à une température comprise entre 20°C et la température d'ébullition du milieu réactionnel.

Dans l'étape D, on opère selon la méthode décrite par GRISAR M. et coll. dans J. Med. Chem., 885 (1973). On forme le magnésien du dérivé bromé puis on fait réagir le nitrile, au sein d'un éther tel que l'éther éthylique, à une température comprise entre 0°C et la température d'ébullition du milieu réactionnel. Après hydrolyse avec un alcool, l'imine intermédiaire est réduite *in situ* par du borohydrure de sodium à une température comprise entre 0°C et la température d'ébullition du milieu réactionnel.

Les dérivés R₂-CO-R₃ sont commercialisés ou peuvent être obtenus par application ou adaptation des méthodes décrites par KUNDER N.G. et coll. J. Chem. Soc. Perkin Trans 1, 2815 (1997); MORENO-MARRAS M., Eur. J. Med. Chem., 23 (5) 477 (1988); SKINNER et coll., J. Med. Chem., 14 (6) 546 (1971); HURN N.K., Tet. Lett., 36 (52) 9453 (1995); MEDICI A. et coll., Tet. Lett., 24 (28) 2901 (1983); RIECKE R.D. et coll., J. Org. Chem., 62 (20) 6921 (1997); KNABE J. et coll., Arch. Pharm., 306 (9) 648 (1973); CONSONNI R. et coll., J. Chem. Soc. Perkin Trans 1, 1809 (1996); FR-96-2481 et JP-94-261393.

Les dérivés R₃Br sont commercialisés ou peuvent être obtenus par application ou adaptation des méthodes décrites par BRANDSMA L. et coll., Synth. Comm., 20 (11) 1697 et 3153 (1990); LEMAIRE M. et coll., Synth. Comm., 24 (1) 95 (1994); GODA H. et coll., Synthesis, 9 849 (1992); BAEUERLE P. et coll., J. Chem. Soc. Perkin Trans 2, 489 (1993).

Les dérivés R₂CN sont commercialisés ou peuvent être obtenus par application ou adaptation des méthodes décrites par BOUYSSOU P. et coll., J. Het. Chem., 29 (4) 895 (1992); SUZUKI N. et coll., J. Chem. Soc. Chem. Comm., 1523 (1984); MARBURG S. et coll., J. Het. Chem., 17 1333 (1980); PERCEC V. et coll., J. Org. Chem., 60 (21) 6895 (1995).

Les composés de formule (I) pour lesquels R₁ représente un radical -N(R₄)R₅ peuvent être préparés selon le schéma réactionnel suivant :

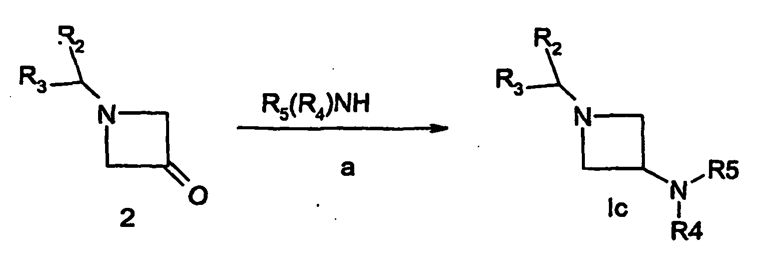

Dans ces formules R₂, R₃, R₄ et R₅ ont les mêmes significations que dans la formule (I).

Cette réaction s'effectue généralement au sein d'un solvant inerte tel qu'un solvant chloré (dichlorométhane par exemple), en présence de triacétoxyborohydrure de sodium et d'acide acétique, à une température voisine de 20°C.

Les composés HN(R₄)R₅ sont commercialisés ou peuvent être préparés selon les méthodes classiques connues de l'homme de l'art ou par application ou adaptation des méthodes décrites par Park K.K. et coll., J. Org. Chem., 60 (19) 6202 (1995); Kalir A. Et coll., J. Med. Chem., 12 (3) 473 (1969); Sarges R., J. Org. Chem., 40 (9) 1216 (1975); Zaugg H.E., J. Org. Chem., 33 (5) 2167 (1968); Med. Chem., 10, 128 (1967); J. Am. Chem. Soc., 2244 (1955); Chem. Ber., 106, 2890 (1973); Chem. Pharm. Bull., 16 (10) 1953 (1968); Bull. Soc. Chim. Fr., 835 (1962).

Les azétidinones 2 peuvent être obtenus par oxydation des azétidinoles correspondants, de préférence au sein de diméthylsulfoxyde, au moyen du complexe trioxyde de soufre-pyridine, à une température voisine de 20°C ou au moyen de diméthylsulfoxyde, en présence de chlorure d'oxalyle et de triéthylamine, à une température comprise entre -70°C et -50°C.

Les composés de formule (I) pour lesquels R₁ représente un radical -N(R₄)COR₅ ou -N(R₄)SO₂R₆ peuvent être préparés selon le schéma réactionnel suivant :

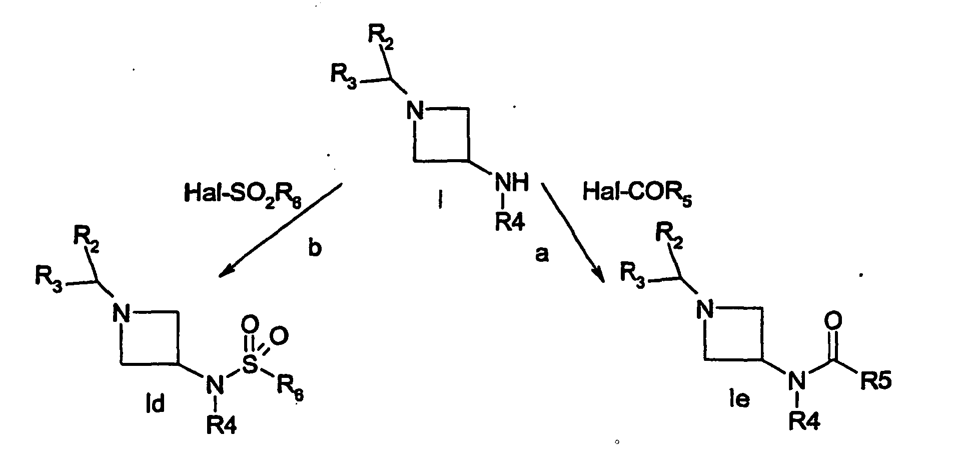

Dans ces formules, R₂, R₃, R₄, R₅ et R₆ ont les mêmes significations que dans la formule (I) et Hal représente un atome d'halogène et de préférence chlore.

Les étapes a et b s'effectuent généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

Les composés de formule (I) pour lesquels R₁ représente un radical -N(R₄)-SO₂-R₆ pour lequel R₄ est un radical Het ou Ar peuvent être préparés selon le schéma réactionnel suivant :

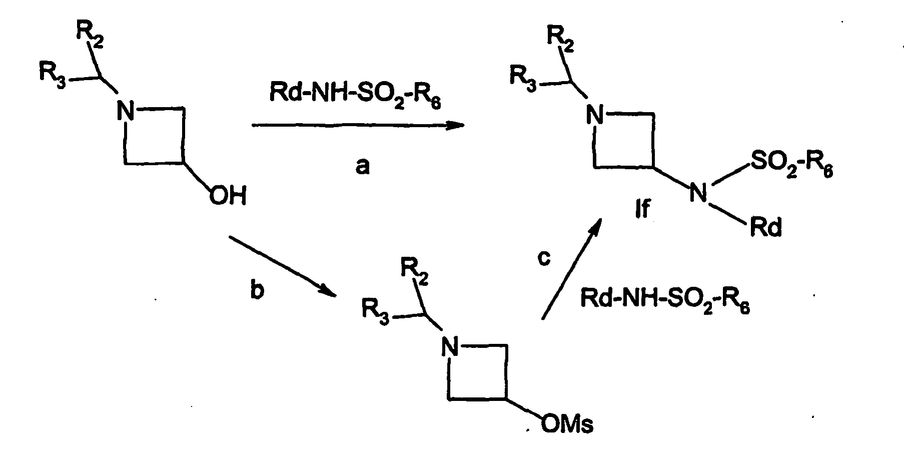

Dans ces formules R₂, R₃ et R₆ ont les mêmes significations que dans la formule (I), Rd représente un radical Ar ou Het (Het et Ar ayant les mêmes significations que dans la formule (I)) et Ms représente un radical méthylsulfonyloxy.

L'étape a s'effectue généralement au sein d'un solvant inerte tel que le tétrahydrofuranne, en présence de triphénylphosphine et de diéthylazodicarboxylate, à une tempéraure comprise entre 0°C et la température d'ébullition du milieu réactionnel.

L'étape b s'effectue généralement au sein d'un solvant inerte tel que le tétrahydrofuranne, le dioxanne, un solvant chloré (dichlorométhane, chloroforme par exemple), à une température comprise entre 15°C et 30°C, en présence d'une base telle qu'une trialkyamine (triéthylamine, dipropyléthylamine par exemple) ou au sein de la pyridine, à une température entre 0°C et 30°C.

L'étape c s'effectue de préférence, au sein d'un solvant inerte tel que le dioxanne, en présence de CsCO₃, au reflux du mélange réactionnel.

Les dérivés pour lesquels Rd représente un hétérocycle azoté N-oxydé peuvent être réduits composé non oxydé selon la méthode décrite par SANGHANEL E. Et coll., Synthesis 1375 (1996).

Les dérivés Rd-NH-SO₂R₆ peuvent être obtenus selon le schéma réactionnel suivant :

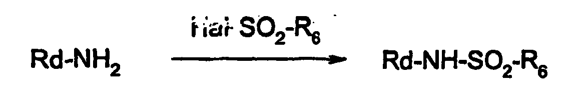

Dans ces formules Hal représente un atome d'halogène et Rd représente un radical Het ou Ar. La réaction s'effectue au sein d'un solvant inerte tel que le tétrahydrofuranne, le dioxanne, un solvant chloré (dichlorométhane, chloroforme par exemple), à une température comprise entre 15°C et 30°C, en présence d'une base telle qu'une trialkyamine (triéthylamine, dipropyléthylamine par exemple) ou au sein de la pyridine, à une température comprise entre 0°C et 30°C.

Les dérivés pour lesquels Rd représente un hétérocycle azoté N-oxydé peuvent être obtenus selon la méthode décrite par RHIE R., Heterocycles, 41 (2) 323 (1995).

Les composés de formule (I) peuvent également être préparés selon le schéma réactionnel suivant :

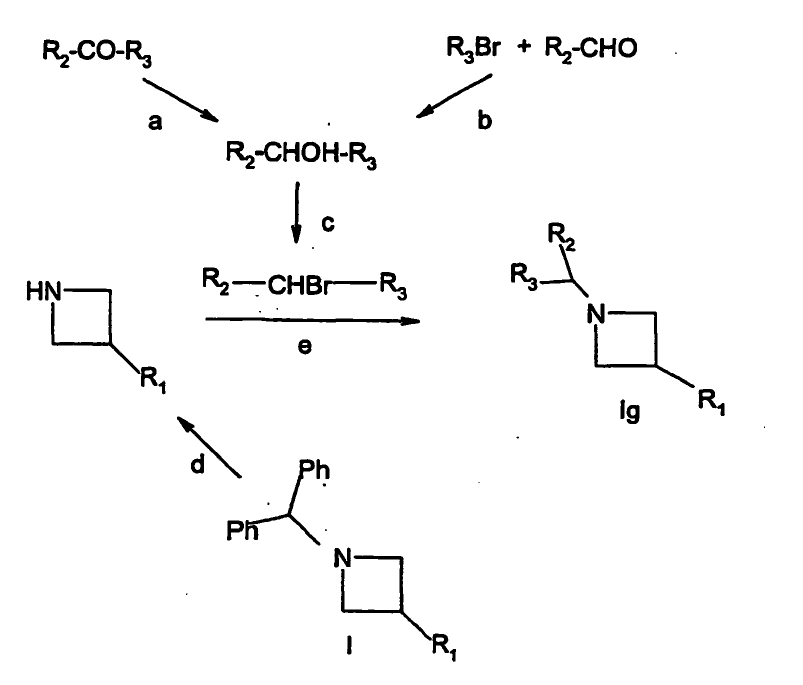

Dans ces formules R₁, R₂ et R₃ ont les mêmes significations que dans la formule (I) et Ph représente un phényle.

L'étape a s'effectue généralement au sein d'un alcool tel que le méthanol, en présence de borohydrure de sodium, à une température voisine de 20°C.

Dans l'étape b, on prépare le magnésien du dérivé bromé et le fait réagir, au sein d'un solvant inerte tel que l'éther éthylique ou le tétrahydrofuranne, à une température comprise entre 0°C et la température d'ébullition du milieu réactionnel.

L'étape c s'effectue au moyen d'un agent d'halogénation tel que l'acide bromhydrique, le bromure de thionyle, le chlorure de thionyle, un mélange de triphénylphosphine et de tétrabromure ou tétrachlorure de carbone, au sein de l'acide acétique ou un solvant inerte tel que le dichlorométhane, le chloroforme, le tétrachlorure de carbone ou le toluène, à une température comprise entre 0°C et la température d'ébullition du milieu réactionnel.

L'étape d s'effectue au moyen d'hydrogène, en présence de charbon palladié, au sein d'un alcool tel que le méthanol, à une température voisine de 20°C.

L'étape e s'effectue au sein d'un solvant inerte tel que l'acétonitrile, en présence d'un carbonate de métal alcalin (carbonate de potassium par exemple) et d'iodure de potassium, à une température comprise entre 20°C et la température d'ébullition du milieu réactionnel.

Les dérivés R₃Br et les dérivés R₂-CHO sont commercialisés ou peuvent être obtenus selon les méthodes décrites par exemple par R.C. LAROCK, Comprehensive Organic Transformations, VCH editor.

Les composés de formule (I) pour lesquels R₁ représente un radical -N(R₄)-SO₂-R₆ pour lequel R₄ est un radical pipérid-4-yle éventuellement substitué sur l'azote par un radical alkyle peuvent également être préparés selon le schéma réactionnel suivant :

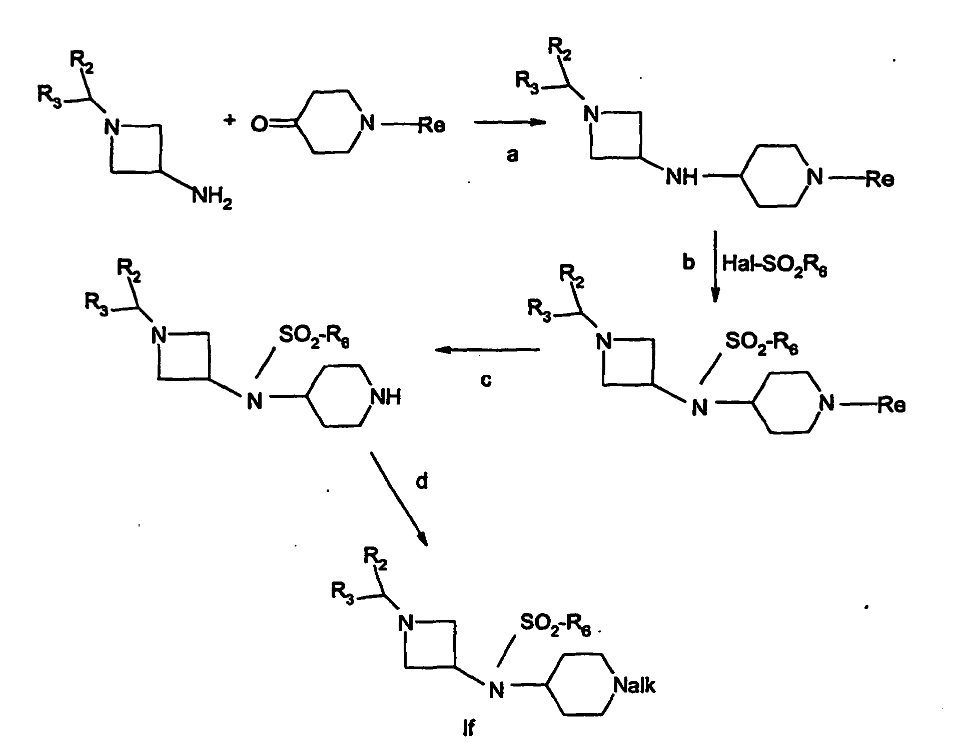

Dans ces formules R₂, R₃ et R₆ ont les mêmes significations que dans la formule (I), alk représente un radical alkyle et Re représente un radical tert-butylcarbonyloxy.

L'étape a s'effectue au sein d'un solvant inerte tel qu'un solvant chloré (dichlorométhane par exemple), en présence d'un hydrure tel que le triacétoxyborohydrure de sodium et d'acide acétique, à une température comprise entre 0°C et la température d'ébullition du milieu réactionnel.

L'étape b s'effectue généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C. L'étape c s'effectue au moyen d'acide chlorhydrique, au sein du dioxanne, à une température comprise entre 0°C et la température d'ébullition du milieu réactionnel.

L'étape d s'effectue par tout moyen connu de l'homme de l'art pour alkyler une amine sans toucher au reste de la molécule. On peut par exemple utiliser un halogènure d'alkyle, en présence d'une base organique telle que la triéthylamine, un hydroxyde de métal alcalin (soude, potasse par exemple), éventuellement en présence de bromure de tétrabutylammonium, au sein d'un solvant inerte tel que le diméthylsulfoxyde, le diméthylformamide ou la pyridine, à une température comprise entre 20 et 50°C.

Les composés de formule (I) pour lesquels R₁ représente un radical -N(R₄)-SO₂-R₆ pour lequel R₄ est un radical phényle substitué par un radical pyrrolid-1-yle peuvent également être préparés par action de pyrrolidine sur un composé de formule (I) correspondant pour lequel R₁ représente un radical -N(R₄)SO₂R₆ pour lequel R₄ est un radical phényle substitué par un atome d'halogène.

Cette réaction s'effectue de préférence, au sein du diméthylsulfoxyde, à une température comprise entre 50 et 95°C.

II est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane ou d'allyle au moyen de catalyseurs du palladium. Comme exemples de groupes protecteurs de la fonction hydroxy, on peut citer les triéthylsilyle, tert-butyldiméthylsilyle qui peuvent être régénérés au moyen de fluorure de tétrabutylammonium ou bien les acétals dissymétriques (méthoxyméthyle, tétrahydropyranyle par exemple) avec régénération au moyen d'acide chlorhydrique. Comme groupes protecteurs des fonctions carboxy, on peut citer les esters (allyle, benzyle par exemple), les oxazoles et les 2-alkyl-1,3-oxazolines. D'autres groupes protecteurs utilisables sont décrits par GREENE T.W. et coll., Protecting Groups in Organic Synthesis, second édition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) présentent des propriétés pharmacologiques intéressante. Ces composés possèdent une forte affinité pour les récepteurs cannabinoïdes et particulièrement ceux de type CB1. Ce sont des antagonistes du récepteur CB1 et sont donc utiles dans le traitement et la prévention des désordres touchant au système nerveux central, au système immunitaire, au système cardio-vasculaire ou endocrinien, au système respiratoire, à l'appareil gastrointestinal et aux désordres de la reproduction (Hollister, Pharm. Rev.; 38, 1986, 1-20, Reny et Sinha, Prog. Drug Res., 36, 71-114 (1991), Consroe et Sandyk, in Marijuana/Cannabinoids, Neurobiology and Neurophysiology, 459, Murphy L. and Barthe A. Eds, CRC Press, 1992).

C'est ainsi que ces composés peuvent être utilisés pour le traitement ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques. Ils peuvent également être utilisés pour le traitement ou la prévention du transit intestinal.

L'affinité des composés de formule (I) pour les récepteurs du cannabis a été déterminée selon la méthode décrite par KUSTER J.E., STEVENSON J.I., WARD S.J., D'AMBRA T.E., HAYCOCK D.A. dans J. Pharmacol. Exp. Ther., 264 1352-1363 (1993).

Dans ce test, la Cl₅₀ des composés de formule (I) est inférieure ou égale à 1000 nM.

Leur activité antagonistique a été montrée au moyen du modèle d'hypothermie induite par un agoniste des récepteurs du cannabis (CP-55940) chez la souris, selon la méthode décrite par Pertwee R.G. dans Marijuana, Harvey D.J. eds, 84 Oxford IRL Press, 263-277 (1985).

Dans ce test, la DE50 des composés de formule (I) est inférieure ou égale à 50 mg/kg.

Les composés de formule (I) présentent une toxicité faible. Leur DL₅₀ est supérieure à 40 mg/kg par voie sous cutanée chez la souris.

Les exemples suivants illustrent l'invention.

### Exemple 1

Le N-{1-[bis-(4-chlorophényl)méthyl]-azétidin-3-yl}-N-(6-chloropyrid-2-yl)-méthylsulfonamide peut être préparé en opérant de la façon suivante : A une solution de 1,54 g de 1-[bis-(4-chlorophényl)méthyl]azétidin-3-ol et de 1,22 g de N-(6-chloropyrid-2-yl)méthylsulfonamide, dans 120 cm³ de tétrahydrofuranne anhydre, on ajoute sous argon 2,4 cm³ d'azodicarboxylate de diéthyle et 1,44 g de triphénylphosphine. Après 20 heures d'agitation à 20°C, le mélange réactionnel est concentré à sec sous pression réduite (2,7 kPa). Le résidu est chromatographié sur une colonne de gel de silice (granulométrie 0,040-0,063 mm, hauteur 30 cm, diamètre 4,5 cm), en éluant sous une pression de 0,5 bar d'argon avec un mélange de cyclohexane et d'acétate d'éthyle (80/20 en volumes) et en recueillant des fractions de 60 cm³. Les fractions 6 à 9 sont réunies et concentrées à sec sous pression réduite (2,7 kPa). On obtient 1,75 g de N-{1-[bis-(4-chlorophényl)méthyl]-azétidin-3-yl}-N-(6-chloropyrid-2-yl)-méthylsulfonamide, sous la forme d'une meringue blanche [Spectre de R.M.N ¹H (300 MHz, CDCl₃, 8 en ppm) : de 2,85 à 3,00 (mt : 2H); 2,91 (s : 3H); 3,57 (t dédoublé, J = 7 et 2 Hz : 2H); 4,25 (s : 1H); 4,64 (mt : 1H); de 7,20 à 7,35 (mt : 9H); 7,36 (dd, J = 8 et 1 Hz : 1 H); 7,71 (t, J = 8 Hz : 1H)].

Le 1-[bis(4-chiorophényl)méthyl]azétidin-3-ol peut être préparé selon le mode opératoire décrit par KATRITZKY A.R. et coll., J. Heterocycl. Chem., 271 (1994), en partant de 35,5 g de chlorhydrate de [bis(4-chlorophényl)méthyl]amine et 11,0 cm³ d'épichlorhydrine. On isole 9,0 g de 1-[bis(4-chlorophényl)méthyl]azétidin-3-ol.

Le chlorhydrate de [bis(4-chlorophényl)méthyl]amine peut être préparé selon la méthode décrite par GRISAR M. et coll., J. Med. Chem., 885 (1973).

Le N-(6-chloropyrid-2-yl)méthylsulfonamide peut être préparé en opérant de la façon suivante : A une solution refroidie à +5°C de 2-amino-6-chloropyridine dans 12,5 cm³ de pyridine, on coule goutte à goutte en 1 heure 7,8 cm³ de chlorure de méthylsulfonyle. Après retour à température ordinaire et 20 heures d'agitation, le mélange réactionnel noir est additionné de 140 cm³ d'eau et extrait par 200 cm³ de dichlorométhane. La phase organique est décantée, séchée sur sulfate de magnésium, filtrée et concentrée à sec sous pression réduite (2,7 kPa). Le résidu huileux obtenu est chromatographié sur une colonne de gel de silice (granulométrie 0,063-0,200 mm, hauteur 30 cm, diamètre 4 cm), en éluant sous une pression de 0,5 bar d'argon avec un mélange de cyclohexane et d'acétate d'éthyle (70/30 en volumes) et en recueillant des fractions de 60 cm³. Les fractions 5 à 11 sont réunies et concentrées à sec sous pression réduite (2,7 kPa). On obtient 17g de N-(6-chloropyrid-2-yl)méthylsulfonamide, sous la forme d'une huile jaune.

### Exemple 2

Le N-{1-[bis-(4-chlorophényl)méthyl]azétidin-3-yl}-N-(6-éthylpyrid-2-yl)-méthylsulfonamide peut être préparé en opérant comme il est décrit dans l'exemple 1, à partir de 0,61 g de 1-[bis-(4-chlorophényl)méthyl]azétidin-3-ol, de 0,40 g de N-(6-éthylpyrid-2-yl)méthylsulfonamide, de 50 cm³ de tétrahydrofuranne anhydre, de 0,96 cm³ d'azodicarboxylate de diéthyle et de 0,577 g de triphénylphosphine. Le produit brut est chromatographié sur une colonne de gel de silice (granulométrie 0,040-0,063 mm, hauteur 20 cm, diamètre 2 cm), en éluant sous une pression de 0,5 bar d'argon avec un mélange de cyclohexane et d'acétate d'éthyle (70/30 en volumes) et en recueillant des fractions de 30 cm³. Les fractions 6 à 9 sont réunies et concentrées à sec sous pression réduite (2,7 kPa). On obtient 0,3 g d'une huile que l'on triture dans un mélange de 5 cm³ d'oxyde de diéthyle et 5 cm³ d'oxyde de diisopropyle. La suspension est filtrée, le solide essoré puis séché sous pression réduite (2,7 kPa). On obtient 0,11 g de N-{1-[bis-(4-chlorophényl)méthyl]azétidin-3-yl}-N-(6-éthylpyrid-2-yl)-méthylsutfonamide, sous la forme d'un solide blanc [Spectre de R.M.N ¹H (300 MHz, CDCl₃, δ en ppm) : 1,26 (t, J = 7,5 Hz : 3H); 2,76 (q, J = 7,5 Hz : 2H); de 2,85 à 2,95 (mt : 2H); 2,90 (s : 3H); 3,53 (t dédoublé, J = 7 et 2 Hz : 2H); 4,22 (s : 1 H); 4,69 (mt : 1H); 7,07 (d, J = 7,5 Hz : 1 H); de 7,15 à 7,30 (mt : 9H); 7,64 (t, J = 7,5 Hz : 1H)].

Le N-(6-éthylpyrid-2-yl)méthylsulfonamide peut être préparé en opérant de la façon suivante : A une solution refroidie à +5°C de 2,50 g de 2-amino-6-éthylpyridine dans 2,50 cm³ de pyridine on coule goutte à goutte 1,56 cm³ de chlorure de méthylsulfonyle. Après 20 heures d'agitation à 20°C, le mélange réactionnel est additionné de 8 cm³ d'eau et filtré. Le filtrat est concentré à sec à 50°C sous pression réduite (2,7 kPa). Le résidu est chromatographié sur une colonne de gel de silice (granulométrie 0,040-0,063 mm, hauteur 30 cm, diamètre 4 cm), en éluant sous une pression de 0,5 bar d'argon avec 1,5 litres de dichlorométhane puis avec un mélange de dichlorométhane et de méthanol (98/2 en volumes) et en recueillant des fractions de 60 cm³. Les fractions 8 à 12 sont réunies et concentrées à sec sous pression réduite (2,7 kPa). On obtient 2,8 g de N-(6-éthylpyrid-2-yl)méthylsulfonamide, sous la forme d'une huile jaune.

### Exemple 3

Le N-{1-[bis-(4-chlorophényl)méthyl]azétidin-3-yl}-N-quinol-6-yl-méthylsulfonamide peut être préparé en opérant de la façon suivante : A une solution de 0,50 g de N-quinol-6-yl-méthylsulfonamlde dans 50 cm³ de tétrahydrofuranne anhydre, on ajoute sous argon 0,70 g de 1-[bis-(4-chlorophényl)méthyl]azétidin-3-ol, 0,597 g de triphénylphosphine puis coule 0,40 cm³ d'azodicarboxylate de diéthyle. Après 20 heures d'agitation à 20°C, le mélange réactionnel est chauffé à la température du reflux pendant 4 heures puis additionné de 2,98 g de triphénylphosphine et de 2,0 cm³ d'azodicarboxylate de diéthyle. Après 48 heures d'agitation à 20°C, le mélange est concentré à sec sous pression réduite (2,7 kPa). Le résidu est repris par 30 cm³ d'oxyde de diéthyle, la suspension obtenue est filtrée, le filtrat concentré à sec. Une fraction du résidu obtenu (0,90 g) est purifiée sur une colonne Bond Elut de résine SCX acide sulfonique échangeuse de cations, (granulométrie 0,054 mm, hauteur 4 cm, diamètre 3 cm), en éluant d'abord avec du méthanol puis avec une solution d'ammoniac 2M dans le méthanol pour éluer le produit attendu, en recueillant des fractions de 5 cm³. Les fractions 16 à 19 sont réunies et concentrées à sec sous pression réduite (2,7 kPa). On obtient 0,33 g d'une huile que l'on agite dans 10 cm³ d'oxyde de diisopropyle. La suspension résultante est filtrée. Le filtrat, filtré à nouveau, donne après 15 minutes, un solide que l'on sèche à 50°C sous pression réduite (2,7 kPa). On obtient 83 mg de N-{1-[bis-(4-chlorophényl)méthyl]azétidin-3-yl}-N-quinol-6-yl-méthylsulfonamide, sous la forme d'un solide blanc [Spectre de R.M.N ¹H (300 MHz, CDCl₃, δ en ppm) : 2,87 (s : 3H); 2,89 (mt : 2H); 3,55 (t dédoublé, J = 7 et 1 Hz : 2H); 4,18 (s : 1H); 4,69 (mt : 1H); de 7,15 à 7,30 (mt : 8H); 7,47 (dd, J = 8,5 et 4 Hz : 1H); 7,58 (dd, J = 9 et 2,5 Hz : 1H); 7,73 (d, J = 2,5 Hz : 1H); 8,10 à 8,20 (mt : 2H); 8,97 (dd, J = 4 et 1,5 Hz : 1H)]

Le N-quinol-6-yl-méthylsulfonamide peut être préparé en opérant de la façon suivante : A une solution refroidie à +3°C de 1,98 g de 6-aminoquinoléine dans 1,75 cm³ de pyridine on coule goutte à goutte en 1 heure 1,1 cm³ de chlorure de méthylsulfonyle. Après 20 heures d'agitation à 20°C, le mélange réactionnel est additionné de 10 cm³ d'eau et de 50 cm³ de dichlorométhane, puis filtré. Le filtrat est décanté, la phase organique est séchée sur sulfate de magnésium, puis filtrée et concentrée à sec sous pression réduite (2,7 kPa). On obtient 1,15 g de N-quinol-6-yl-méthylsulfonamide, sous la forme d'un solide jaune crème.

### Exemple 4

Le N-{1-[bis-(4-chlorophényl)méthyl]azétidin-3-yt}-N-quinol-5-yl-méthylsulfonamide peut être préparé en opérant de la façon suivante : A une solution de 0,50 g de N-(quinol-5-yl)méthytsulfonamide dans 70 cm³ de tétrahydrofuranne anhydre, on ajoute sous argon 0,70 g de 1-[bis-(4-chlorophényl)méthyl]azétidin-3-ol, 0,597 g de triphénylphosphine puis coule 0,40 cm³ d'azodicarboxylate de diéthyle et 0,45 g de 1,2-bis-(diphénylphosphine)éthane. Après 20 heures d'agitation à 20°C, le mélange réactionnel est concentré à sec sous pression réduite (2,7 kPa). Le résidu est repris par 70 cm³ d'acétate d'éthyle, la solution résultante est lavée par 30 cm³ de saumure, séchée sur sulfate de magnésium, filtrée puis concentrée à sec à 50°C sous pression réduite (2,7 kPa). L'huile violette obtenue est purifiée par chromatographie sur une colonne de gel de silice (granulométrie 0,063-0,200 mm, hauteur 35 cm, diamètre 3,9 cm), en éluant sous une pression de 0,5 bar d'argon avec un mélange de cyclohexane et d'acétate d'éthyle (40/60 puis 30/70 et 20/80 en volumes) et en recueillant des fractions de 50 cm³. Les fractions 6 à 12 sont réunies et concentrées à sec sous pression réduite (2,7 kPa). Le résidu est repris par 15 cm³ de méthanol, la suspension blanche résultante est filtrée, le solide essoré, puis séché à 50°C sous pression réduite (2,7 kPa). On obtient 0,35 g de N-{1-[bis-(4-chlorophényl)méthyl]azétidin-3-yl}N-quinol-5-yl-méthylsulfonamide, sous la forme d'un solide blanc [Spectre de R.M.N ¹H (300 MHz, CDCl₃, δ en ppm) : 2,60 (t, J = 7 Hz : 1H); 2,84 (t, J = 7 Hz : 1H); 2,99 (s : 3H); 3,36 (t dédoublé, J = 7 et 2, 5 Hz : 1 H); 3,56 (t dédoublé, J = 7 et 2,5 Hz : 1H); 4,01 (s : 1H); 4,85 (mt : 1H); de 7,10 à 7,25 (mt : 8H); 7,40 (dd, J = 7,5 et 1 Hz : 1H); 7,54 (dd, J = 8,5 et 4 Hz : 1H); 7,74 (dd, J = 8 et 7,5 Hz : 1H); 8,20 (d large, J = 8 Hz : 1H); 8,54 (d large, J = 9 Hz : 1H); 8,99 (dd, J = 4 et 1,5 Hz : 1H)].

Le N-(quinol-5-yl)méthylsulfonamide peut être préparé en opérant comme il est décrit dans l'exemple 3, à partir de 2,0 g de 5-aminoquinoléine, 3,0 cm³ de pyridine, 1,1 cm³ de chlorure de méthylsulfonyle. On obtient 2,47 g de N-(quinol-5-yl)méthylsulfonamide, sous la forme d'un solide jaune brun.

### Exemple 5

Le N-{1-[bis-(4-chlorophényl)méthyl]azétidin-3-yl}-N-isoquinol-5-yl-méthylsulfonamide peut être préparé en opérant comme il est décrit dans l'exemple 4, à partir de 0,497 g de N-(isoquinol-5-yl)méthylsuffonamide, 70 cm³ de tétrahydrofuranne anhydre, 0,712 g de 1-[bis-(4-chlorophényl)méthflazétidin-3-ol, 0,597 g de triphénylphosphine, de 0,40 cm³ d'azodicarboxylate de diéthyle et de 0,45 g de 1,2-bis-(diphénylphosphine)éthane. L'huile brune brute obtenue est purifiée par chromatographie sur une colonne de gel de silice (granulométrie 0,063-0,200 mm, hauteur 38 cm, diamètre 3 cm), en éluant avec un mélange de cyclohexane et d'acétate d'éthyle (30/70 en volumes) et en recueillant des fractions de 40 cm³. Les fractions 8 à 23 sont réunies et concentrées à sec sous pression réduite (2,7 kPa). Le résidu est agité dans 15 cm³ d'oxyde de diéthyle, la suspension est filtrée et l'insoluble est chromatographié sur une colonne de résine SCX (hauteur 4 cm, diamètre 3 cm), en lavant d'abord avec un mélange de méthanol et de dichlorométhane (50/50 en volumes) puis en éluant avec une solution d'ammoniac 2M dans le méthanol et en recueillant des fractions de 20 cm³. Les fractions 1 à 6 sont réunies et l'insoluble blanc qui apparaît est filtré, le solide est essoré, puis séché à 50°C sous pression réduite (2,7 kPa). On obtient 0,169 g de N-{1-[bis-(4-chlorophényl)méthyl]azétidin-3-yl}-N-isoquinol-5-yl-méthyisulfonamide, sous la forme d'un solide blanc [Spectre de R.M.N ¹H (300 MHz, CDCl₃, δ en ppm) : 2,64 (t, J = 7 Hz : 1H); 2,81 (t, J = 7 Hz : 1H); 2,98 (s : 3H); 3,36 (t dédoublé, J = 7 et 2 Hz : 1H); 3,55 (t dédoublé, J = 7 et 2 Hz : 1H); 4,02 (s : 1H); 4,86 (mt : 1H); de 7,10 à 7,25 (mt : 8H); 7,60 (dd, J = 8 et 1 Hz : 1H); 7,66 (t, J = 8 Hz : 1H); 7,93 (d large, J = 6 Hz : 1H); 8,06 (d large, J = 8 Hz : 1H); 8,66 (d, J = 6 Hz : 1H); 9,32 (s large : 1H)].

Le N-(isoquinol-5-yl)méthylsulfonamide peut être préparé en opérant comme il est décrit dans l'exemple 4, à partir de 2,0 g de 5-aminoisoquinoléine, 3,0 cm³ de pyridine et de 1,1 cm³ de chlorure de méthylsulfonyle. On obtient 2,3 g de N-(isoquinol-5-yl)méthylsulfonamide, sous la forme d'un solide beige.

### Exemple 6

Le N-{1-[bis-(4-chlorophényl)méthyl]azétidin-3-yl}-N-pyrid-3-yl-méthylsulfonamide peut être préparé en opérant de la façon suivante : A une solution de 0,144 g de N-{1-[bis-(4-chlorophényl)méthyl]azétidin-3-yl}-N-(1-oxyde-pyrid-3-yl)-méthylsulfonamide dans 5 cm³ de chloroforme, on coule 0,042 cm³ de trichlorure de phosphore, puis chauffe le mélange à la température du reflux. Après 1 heure et 30 minutes d'agitation, le mélange réactionnel est laissé revenir à température ordinaire, puis est additionné de 5 cm³ d'acide chlorhydrique 0,1N, puis agité et décanté. La phase organique est diluée avec 20 cm³ de chloroforme, séchée sur sulfate de magnésium, filtrée, puis concentrée à sec sous pression réduite (2,7 kPa). Le résidu est chromatographié sur une colonne de gel de silice (granulométrie 0,063-0,200 mm, hauteur 9 cm, diamètre 1,8 cm), en éluant sous une pression de 0,1 bar d'argon avec un mélange de dichlorométhane et de méthanol (95/5 en volumes) et en recueillant des fractions de 15 cm³. Les fractions 2 à 4 sont réunies et concentrées à sec sous pression réduite (2,7 kPa). Le résidu est agité avec 15 cm³ d'oxyde de diéthyle, la suspension est filtrée, le solide essoré puis séché sous pression réduite (2,7 kPa). On obtient 35 mg de N-{1-[bis-(4-chlorophényl)méthyl]azétidin-3-yl}-N-pyrid-3-yl-méthylsulfonamide, sous la forme d'un solide crème [Spectre de R.M.N. ¹H (300 MHz, CDCl₃, δ en ppm) : de 2,80 à 2,95 (mt : 2H); 2,87 (s : 3H); 3,51 (t dédoublé, J = 7 et 1,5 Hz : 2H); 4,18 (s : 1 H); 4,65 (mt : 1H); de 7,15 à 7,35 (mt : 8H); 7,37 (dd large, J = 8 et 5 Hz : 1 H); 7,64 (d démultiplié, J = 8 Hz : 1 H); 8,52 (d large, J = 2 Hz : 1 H); 8,61 (d large, J = 5 Hz : 1 H)].

### Exemple 7

Le N-{1-[bis-(4-chlorophényl)méthyl]azétidin-3-yl}-N-(1-oxyde-pyrid-3-yl)-méthylsulfonamide peut être préparé en opérant de la façon suivante : A une solution de 0,265 g de 1-[bis-(4-chlorophényl)méthyl]azétidin-3-ol et de 0,162 g de N-(1-oxyde-pyrid-3-yl)méthylsulfonamide, dans 25 cm³ de tétrahydrofuranne anhydre, on ajoute sous argon 0,16 cm³ d'azodicarboxylate de diéthyle et 0,226 g de triphénylphosphine. Après 20 heures d'agitation à 20°C, puis 24 heures à la température du reflux, le mélange réactionnel est concentré à sec sous pression réduite (2,7 kPa). Le résidu est chromatographié sur une colonne de gel de silice (granulométrie 0,063-0,2 mm, hauteur 20 cm, diamètre 1,5 cm), en éluant sous une pression de 0,5 bar d'argon avec un mélange de dichlorométhane et de méthanol (98/2 en volumes) et en recueillant des fractions de 40 cm³. Les fractions 26 à 64 sont réunies et concentrées à sec sous pression réduite (2,7 kPa). le résidu est agité dans 10 cm³ d'oxyde de diéthyle, la suspension est filtrée, l'insoluble est essoré, puis séché sous pression réduite (2,7 kPa). On obtient 0,10 g de N-{1-[bis-(4-chlorophényl)méthyl]azétidin-3-yl}-N-(1-oxyde-pyridin-3-yl)-méthylsulfonylamide, sous la forme d'un solide blanc [Spectre de R.M.N ¹H (400 MHz, (CD₃)₂SO d6, δ en ppm) : 2,78 (t, J = 7 Hz : 2H); 3,06 (s : 3H); 3,37 (t, J = 7 Hz : 2H); 4,45 (s : 1H); 4,71 (mt : 1H): de 7,30 à 7,50 (mt : 10H); 8,21 (d large, J = 6,5 Hz : 1H); 8,27 (s large : 1H)].

Le N-(1-oxyde-pyrid-3-yl)méthylsulfonamide peut être préparé en opérant de la façon suivante: A une solution de 1,81 g de N-pyrid-3-yl-méthylsulfonamide dans 71 cm³ de N,N-diméthylformamide et 3 cm³ de méthanol, on ajoute par fractions 7,1 g d'acide 3-chloroperoxybenzoïque à 50-55% puis 0,56 cm³ d'acide fluorhydrique à 40%. Après 1 heure d'agitation à 20°C, le mélange réactionnel est versé dans 500 g de glace, agité, puis filtré. Le filtrat est concentré à sec à 60°C sous pression déduite (2,7 kPa). Le résidu est repris par 50 cm³ d'un mélange de dichlorométhane et de méthanol (98/2 en volumes) puis filtré. Le filtrat est chromatographié sur une colonne de gel de silice (granulométrie 0,063-0,2 mm, hauteur 27 cm, diamètre 4 cm), en éluant sous une pression de 0,5 bar d'argon avec un mélange de dichlorométhane et de méthanol (98/2, 97/3 puis 50/50 en volumes) et en recueillant des fractions de 60 cm³. La fraction 62 est concentrée à sec sous pression réduite (2,7 kPa). On obtient 0,96 g de N-(1-oxyde-pyrid-3-yl)méthylsulfonamide, sous la forme d'un solide jaunâtre.

Le N-pyrid-3-yl-méthylsulfonamide peut être préparé en opérant comme il est décrit dans l'exemple 1, à partir de 2 g de 3-aminopyridine, 5 cm³ de pyridine et de 1,8 cm³ de chlorure de méthylsulfonyle. Le produit brut obtenu est agité dans 40 cm³ d'oxyde de diéthyle, la suspension est filtrée puis le solide est essoré et séché sous pression réduite (2,7 kPa). On obtient 2,47 g de N-pyrid-3-yl-méthylsulfonamide, sous la forme d'un solide rosâtre.

### Exemple 8

Le N-{1-[bis-(4-chlorophényl)-méthyl]azétidin-3-yl}-N-cyclohexyl-méthylsulfonamide peut être préparé en opérant de la façon suivante : A une solution de 1,8 g de N-{1-[bis-(4-chlorophényl)méthyl]azétidin-3-yl}-N-cyclohexylamine, de 0,7 cm³ de triéthylamine et de 20 mg de 4-diméthylaminopyridine dans 25 cm³ de dichlorométhane, on ajoute sous agitation 0,4 cm³ de chlorure de méthylsulfonyle. Après 48 heures d'agitation à 20°C, on ajoute au mélange réactionnel 20 cm³ de dichlorométhane, 20 cm³ d'eau et on agite et décante. La phase organique est séchée sur sulfate de magnésium et concentrée à 50°C sous pression réduite (2,7 kPa). Le résidu huileux brun est chromatographié sur une colonne de gel de silice (granulométrie 0,063-0,2 mm, hauteur 20 cm, diamètre 2,0 cm), en éluant sous une pression de 0,1 bar d'argon avec un mélange de dichlorométhane et de méthanol (96/4 en volumes) et en recueillant des fractions de 10 cm³. Les fractions 2 à 4 et 5 à 10 sont réunies et concentrées à sec sous pression réduite (2,7 kPa). Le résidu est chromatographié sur une colonne de gel de silice (granulométrie 0,063-0,2 mm, hauteur 30 cm, diamètre 1,5 cm), en éluant sous une pression de 0,1 bar d'argon avec un mélange de cyclohexane et d'acétate d'éthyle (70/30 en volumes) et en recueillant des fractions de 5 cm³. Les fractions 7 à 10 sont réunies et concentrées à sec sous pression réduite (2,7 kPa). On obtient 0,10 g de N-{1-[bis-(4-chlorophényl)-méthyl]azétidin-3-yl}-N-cyclohexyl-méthylsulfonamide, sous la forme d'une meringue crème [Spectre de R.M.N. ¹H (300 MHz, CDCl₃, δ en ppm) : de 0,80 à 1,90 (mt : 10H); 2,82 (s : 3H); 3,36 (t large, J = 7,5 Hz : 2H); 3,46 (t large, J = 7,5 Hz : 2H); 3,59 (mt : 1H); 4,08 (mt : 1 H); 4,42 (s : 1H); de 7,20 à 7,40 (mt : 8H)].

La N-{1-[bis-(4-chtorophényl)méthyl]azétidin-3-yl}-N-cyclohexylamine peut être préparée en opérant de la façon suivante : A une solution de 1,5 g de 1-[bis-(4-chlorophényl)méthyl]azétidin-3-one dans 25 cm³ de dichloro-1,2-éthane, on ajoute 0,5 g de cyclohexylamine, 1 g de triacétoxyborohydrure de sodium et 0,3 cm³ d'acide acétique à 100%. Après 20 heures d'agitation à 20°C, on ajoute au mélange réactionnel en agitant 20 cm³ de dichlorométhane et 10 cm³ d'eau puis neutralise jusqu'à pH 7 à 8 avec une solution aqueuse d'hydroxyde de sodium 1 N. Le mélange est décanté, la phase organique est séchée sur sulfate de magnésium et concentrée à sec à 50°C sous pression réduite (2,7 kPa). On obtient 1,8 g de N-{1-[bis-(4-chlorophényl)méthyl]azétidin-3-yl}-N-cyclohexylamine, sous la forme d'une pâte crème qui sera utilisée telle quelle à l'étape suivante.

La 1-[bis(4-chlorophényl)méthyl]azétidin-3-one peut être préparée selon le mode opératoire suivant : à une solution de 5,0 cm³ de chlorure d'oxalyle dans 73 cm³ de dichlorométhane refroidie à -78°C, on additionne une solution de 8,1 cm³ de diméthylsulfoxyde dans 17,6 cm³ de dichlorométhane. Après 0,5 heure à -78°C, on coule une solution de 16,0 g de 1-[bis(4-chlorophényl)méthyl]azétidin-3-ol dissous dans 50 cm³ de dichlorométhane. Après 5 heures à -78°C, 26,6 cm³ de triéthylamine sont ajoutés goutte à goutte et on laisse le mélange réactionnel revenir à température ambiante. Après 16 heures, le mélange réactionnel est lavé par 4 fois 200 cm³ d'eau puis par 200 cm³ d'une solution saturée de chlorure de sodium, séché sur sulfate de magnésium, filtré et concentré à sec sous pression réduite (2,7 kPa). Le résidu obtenu est chromatographié sur colonne de gel de silice (granulométrie 0,04-0,06 mm, diamètre 9,2 cm, hauteur 21 cm), sous une pression de 0,5 bar d'argon avec un mélange d'acétate d'éthyle et cyclohexane (40/60 en volumes) comme éluants et en recueillant des fractions de 200 cm³. Les fractions 15 à 25 sont réunies puis concentrées à sec sous pression réduite (2,7 kPa). On obtient 8,9 g de 1-[bis(4-chlorophényl)méthyl]azétidin-3-one sous forme de cristaux jaunes pâle fondants à 111°C.

### Exemple 9

Le N-{1-[bis-(4-chlorophényl)-méthyl]azétidin-3-yl}-N-cyclopropyl-méthylsulfonamide peut être préparé en opérant comme il est décrit dans l'exemple 8, à partir de 1,6 g de N-{1-[bis-(4-chlorophényl)méthyl]azétidin-3-yl}-N-cyclopropylamine, de 25 cm³ de dichlorométhane, de 0,7 cm³ de triéthylamine, de 20 mg de 4-diméthylaminopyridine et de 0,4 cm³ de chlorure de méthylsulfonyle, en agitant le mélange pendant 20 heures à 20°C. Le produit brut est chromatographié sur une colonne de gel de silice (granulométrie 0,063-0,2 mm, hauteur 30 cm, diamètre 2,0 cm), en éluant sous une pression de 0,1 bar d'argon avec un mélange de dichlorométhane et de méthanol (97/3 en volumes) et en recueillant des fractions de 10 cm³. Les fractions 6 à 9 et 10 à 20 sont réunies et concentrées à sec sous pression réduite (2,7 Kpa). Le résidu obtenu est chromatographié sur une colonne de gel de silice (granulométrie 0,063-0,2 mm, hauteur 30 cm, diamètre 2,0 cm), en éluant sous une pression de 0,1 bar d'argon avec un mélange de cyclohexane et d'acétate d'éthyle (70/30 en volumes) et en recueillant des fractions de 10 cm³. Les fractions 6 à 11 sont réunies et concentrées à sec sous pression réduite (2,7 kPa). On obtient 0,14 g de N-{1-[bis-(4-hlorophényl)-méthyl]azétidin-3-yl}-N-cyclopropyl-méthylsulfonamide, sous la forme d'une meringue crème [Spectre de R.M.N ¹H (300 MHz, CDCl₃, δ en ppm) : 0,79 (mt : 2H); 0,95 (mt : 2H); 2,11 (mt : 1H); 2,84 (s : 3H); 3,17 (t large, J = 7 Hz : 2H); 3,50 (mt : 2H); 4,18 (mt : 1H); 4,29 (s : 1H); de 7,20 à 7,40 (mt : 8H)].

La N-{1-[bis-(4-chlorophényl)méthyl]azétidin-3-yl}-N-cyclopropylamine peut être préparée en opérant comme il est décrit dans l'exemple 8, à partir de 1,5 g de 1-[bis-(4-chlorophényl)méthyl]azétidin-3-one, de 25 cm³ de dichloro-1,2-éthane, de 0,37 cm³ de cyclopropylamine, de 1 g de triacétoxyborohydrure de sodium et de 0,3 cm³ d'acide acétique à 100%. On obtient, 1,6 g de N-{1-[bis-(4-chlorophényl)méthyl]azétidin-3-yl}-N-cyclopropylamine, sous la forme d'une huile brune qui sera utilisée telle quelle à l'étape suivante.

### Exemple 10

Le N-(1R,2S,4S)-bicyclo[2,2,1]hept-2-yl-N-{1-[bis-(4-chlorophényl)méthyl]azétidin-3-yl}-méthylsulfonamide, peut être préparé en opérant comme il est décrit dans l'exemple 8, à partir de 2,0 g de N-(1R,2S,4S)-bicyclo[2,2,1]hept-2-yl-N-{1-[bis-(4-chlorophényl)méthyl]azétidin-3-yl}amine, de 25 cm³ de dichlorométhane, de 0,7 cm³ de triéthylamine, de 20 mg de 4-diméthylaminopyridine et de 0,4 cm³ de chlorure de méthylsulfonyle, en agitant pendant 20 heures. Le résidu huileux brun est chromatographié sur une colonne de gel de silice (granulométrie 0,063-0,2 mm, hauteur 30 cm, diamètre 2,0 cm), en éluant sous une pression de 0,1 bar d'argon avec un mélange de dichlorométhane et de méthanol (97/3 en volumes) et en recueillant des fractions de 10 cm³. Les fractions 6 à 18 sont réunies et concentrées à sec sous pression réduite (2,7 kPa). Le résidu est chromatographié sur une colonne de gel de silice (granulométrie 0,063-0,2 mm, hauteur 30 cm, diamètre 2,0 cm), en éluant sous une pression de 0,1 bar d'argon avec un mélange de cyclohexane et d'acétate d'éthyle (70/30 en volumes) et en recueillant des fractions de 10 cm³. Les fractions 8 à 14 sont réunies et concentrées à sec sous pression réduite (2,7 kPa). On obtient 0,70 g de N-(1R,2S,4S)-bicyclo[2,2,1]hept-2-yl-N-{1-[bis-(4-chlorophényl)-méthyl]azétidin-3-yl}-méthylsulfonamide, sous la forme d'une meringue crème [Spectre de R.M.N ¹H (300 MHz, CDCl₃, δ en ppm) : de 1,20 à 1,75 (mt : 7H); 1,84 (t large, J = 12,5 Hz : 1H); 2,29 (mt : 1H); 2,35 (mt : 1H); 2,82 (s : 3H); de 3,35 à 3,55 (mt : 3H); 3,66 (mt : 1H); de 3,90 à 4,05 (mt : 2H); 4,51 (s : 1H); de 7,20 à 7,45 (mt : 8H)].

La N-(1R,2S,4S)-bicyclo[2,2,1]hept-2-yl-N-{1-[bis-(4-chlorophényl)méthyl] azétidin-3-yl}amine peut être préparée en opérant comme il est décrit dans l'exemple 8, à partir de 1,5 g de 1-[bis-(4-chlorophényl)méthy]azétdin-3-one, de 25 cm³ de dichloro-1,2-éthane, de 1,5 g de (1R,2S,4S)-bicyclo[2,2,1]heptyl-2-amine, de 1 g de triacétoxyborohydrure de sodium et de 0,3 cm³ d'acide acétique à 100%. On obtient 2 g de N-(1R,2S,4S)-bicyclo[2,2,1]hept-2-yl-N-{1-[bis-(4-chlorophényl)méthyl]azétidin-3-yl}amine, sous la forme d'une huile brune qui sera utilisée telle quelle à l'étape suivante.

### Exemple 11

Le N-(1R,2R,4S)-bicyclo[2,2,1]hept-2-yl-N-{1-[bis-(4-chlorophényl)méthyl] azétidin-3-yl}-méthylsulfonamide peut être préparé en opérant comme il est décrit dans l'exemple 8, à partir de 1,8 g de N-(1R,2R,4S)-bicyclo[2,2,1]hept-2-yl-N-{1-[bis-(4-chlorophényl)méthyl]azétidin-3-yl}amine, de 25 cm³ de dichlorométhane, de 0,7 cm³ de triéthylamine, de 20 mg de 4-diméthylaminopyridine et de 0,4 cm³ de chlorure de méthylsulfonyle, en agitant pendant 20 heures. Le résidu huileux brun est chromatographié sur une colonne de gel de silice (granulométrie 0,063-0,2 mm, hauteur 30 cm, diamètre 2,0 cm), en éluant sous une pression de 0,1 bar d'argon avec un mélange de cyclohexane et d'acétate d'éthyle (60/40 en volumes) et en recueillant des fractions de 10 cm³. Les fractions 3 à 12 sont réunies et concentrées à sec sous pression réduite (2,7 Kpa). Le résidu est chromatographié sur une colonne de gel de silice (granulométrie 0,063-0,2 mm, hauteur 30 cm, diamètre 2,0 cm), en éluant sous une pression de 0,1 bar d'argon avec un mélange de cyclohexane et d'acétate d'éthyle (70/30 en volumes) et en recueillant des fractions de 10 cm³. Les fractions 4 à 10 sont réunies et concentrées à sec sous pression réduite (2,7 kPa). On obtient 0,10g de N-(1R,2R,4S)-bicyclo[2,2,1]hept-2-yl-N-{1-[bis-(4-chlorophényl) méthyt]azétidin-3-yl}-méthylsutfonamide, sous la forme d'une meringue jaune [Spectre de R.M.N¹H (300 MHz, CDCl₃, δ en ppm) : de 1,00 à 1,85 (mt : 8H); 2,14 (mt : 1H); 2,33 (mt : 1H); 2,82 (s : 3H); de 3,40 à 3,60 (mt : 4H); 3,71 (dd large, J = 8 et 6 Hz : 1H); 4,10 (mt : 1H); 4,47 (s : 1H); de 7,20 à 7,40 (mt : 8H)].

La N-(1 R,2R,4S)-bicyclo[2,2, 1]hept-2-yl-N-{1-[bis-(4-chlorophényl)méthyl] azétidin-3-yl}amine peut être préparée en opérant comme il est décrit dans l'exemple 8, à partir de 1,5 g de 1-[bis-(4-chlorophényl)méthyl]azétidin-3-one, de 25 cm³ de dichloro-1,2-éthane, de 0,6 g de (1R,2R,4S)-bicyclo[2,2,1]heptyl-2-amine, de 1,0 g de triacétoxyborohydrure de sodium et de 0,3 cm³ d'acide acétique à 100%. On obtient 1,8 g de N-(1R,2R,4S)-bicyclo[2,2,1]hept-2-yl-N-(1-[bis-(4-chlorophényl)méthyl]azétdin-3-yl)amine, sous la forme d'une pâte crème qui sera utilisée telle quelle à l'étape suivante.

### Exemple 12

Le N-[(1-benzhydryl)azétidin-3-yl]-N-phényl-méthylsulfonamide peut être préparé en opérant de la façon suivante : A une solution de 2 g de 1-benzhydryl 3-anilino azétidine, dans 40 cm³ de dichlorométhane, on coule 0,7 cm³ de chlorure de méthylsulfonyle puis ajoute 1,34 cm³ de triéthylamine. Après 4 heures et 15 minutes d'agitation à 20°C, le mélange réactionnel est lavé par 2 fois 20 cm³ d'eau, la phase organique est séchée sur sulfate de magnésium, puis concentrée à sec à 50°C sous pression réduite (2,7 kPa). L'huile marron obtenue est chromatographiée sur une colonne de gel de silice (granulométrie 0,063-0,2 mm, hauteur 26 cm, diamètre 3,6 cm), en éluant sous une pression de 0,5 bar d'argon avec un mélange de cyclohexane et d'acétate d'éthyle (70/30 en volumes) et en recueillant des fractions de 50 cm³. Les fractions 10 à 15 sont réunies et concentrées à sec sous pression réduite (2,7 kPa), le résidu est trituré dans de l'oxyde de diéthyle, la suspension est filtrée, le solide essoré, puis séché sous pression réduite (2,7 kPa). On obtient 35 mg de N-[(1-benzhydryl)azétidin-3-yl]-N-phényl-méthylsulfonamide, sous la forme d'un solide blanc [Spectre de R.M.N. ¹H (300 MHz, (CD₃)₂SO d6 avec ajout de quelques gouttes de CD₃COOD d4, δ en ppm) : 2,72 (mt : 2H); 2,92 (s : 3H); 3,36 (mt : 2H); 4,32 (s : 1H); 4,73 (mt : 1H); de 7,10 à 7,45 (mt : 15H)].

La 1-benzhydryl 3-anilino azétidine peut être préparée en opérant comme il est décrit dans l'exemple 8, à partir de 5 g de 1-benzhydryl azétidin-3-one, de 1,92 cm³ d'aniline, de 74 cm³ de dichloro-1,2-éthane, de 6,3 g de triacétoxyborohydrure de sodium et de 1,2 cm³ d'acide acétique à 100%. On obtient 8,81 g de 1-benzhydryl 3-anilino azétidine, sous la forme d'une gomme marron qui sera utilisée telle quelle à l'étape suivante.

### Exemple 13

Le N-{1-[bis-(4-chlorophényl)méthyl]azétidin-3-yl}-N-(3,5-difluorophényl)-méthylsulfonamide peut être préparé en opérant de la façon suivante : A un mélange de 1,23 g de méthylsulfonate de 1-[bis(4-chlorophényl)méthyl]azétidin-3-yle et de 0,66 g de N-(3,5-difluorophényl)méthylsulfonamide, dans 25 cm³ de dioxane, on ajoute 1,0 g de carbonate de césium. Après 5 heures d'agitation à la température du reflux puis 20 heures à 20°C, le mélange réactionnel est additionné de 50 cm³ d'oxyde de diéthyle et de 30 cm³ de saumure, puis est agité et décanté. La phase organique est séchée sur sulfate de magnésium, filtrée puis concentrée à sec à 50°C sous pression réduite (2,7 kPa). L'huile orange obtenue est chromatographiée sur une colonne de gel de silice (granulométrie 0,040-0,063 mm, hauteur 25 cm, diamètre 2,0 cm), en éluant sous une pression de 0,5 bar d'argon avec un mélange de cyclohexane et d'acétate d'éthyle (65/35 en volumes) et en recueillant des fractions de 10 cm³. Les fractions 6 à 10 sont réunies et concentrées à sec sous pression réduite (2,7 Kpa). Le résidu est chromatographié sur une colonne de gel de silice (granulométrie 0,040-0,063 mm, hauteur 15 cm, diamètre 1,0 cm), en éluant sous une pression de 0,5 bar d'argon avec un mélange de cyclohexane et d'acétate d'éthyle (65/35 en volumes) et en recueillant des fractions de 5 cm³. La fraction 7 est concentrée à sec sous pression réduite (2,7 kPa). On obtient 0,11 g de N-{1-[bis-(4-chlorophényl)méthyl]azétidin-3-yl}-N-(3,5-difluorophényl)-méthylsulfonamide, sous la forme d'une poudre blanche [Spectre de R.M.N ¹H (300 MHz, CDCl₃, δ en ppm) : 2,82 (s : 3H); 2,85 (mt : 2H); 3,52 (t dédoublé, J = 7 et 2 Hz : 2H); 4,22 (s : 1H); 4,47 (mt : 1H); de 6,75 à 6,90 (mt : 3H); de 7,20 à 7,35 (mt : 8H)].

### Méthode 2

A une solution de 1,41 g de 1-[bis-(4-chlorophényl)méthyl]azétidin-3-ol et de 0,95 g de N-(3,5-difluorophényl)méthylsulfonamide dans 100 cm³ de tétrahydrofuranne anhydre, on ajoute sous argon 0,78 cm³ d'azodicarboxylate de diéthyle et 1,31 g de triphénylphosphine. Après 16 heures d'agitation à 20°C, 300 cm³ d'acétate d'éthyle sont additionnés, le mélange réactionnel est lavé 2 fois avec 100 cm³ d'eau, séché sur sulfate de magnésium et concentré à sec sous pression réduite (2,7 kPa). Le résidu est chromatographié sur une colonne de gel de silice (granulométrie 0,20-0,063 mm, hauteur 50 cm, diamètre 4 cm), en éluant sous une pression de 0,6 bar d'argon avec un mélange de cyclohexane et d'acétate d'éthyle (75/25 en volumes) et en recueillant des fractions de 125 cm³. Les fractions 6 à 12 sont réunies et concentrées à sec sous pression réduite (2,7 kPa). On obtient 1,8 g d'un solide qui est dissous à chaud dans un mélange acétate d'éthyle/dilsopropyle éther (15/2 en volume), refroidi, dilué avec 100 cm³ de pentane pour amorcer la cristallisation. Après filtration et séchage, on obtient 1,0g de N-{1-[bis-(4-chlorophényl)méthyl]azétidin-3-yl}-N-(3,5-difluorophényl)-méthylsulfonamide sous la forme de cristaux blancs fondants à 154°C.

Le N-(3,5-difluorophényl)méthylsulfonamide, peut être préparé en opérant de la façon suivante : A une solution de 3,5 g de 3,5-difluoroaniline dans 75 cm³ de dichlorométhane, on ajoute lentement 2,0 cm³ de chlorure de méthylsulfonyle, 3,8 cm³ de triéthylamine et 20 mg de 4-diméthylaminopyridine. Après 20 heures d'agitation à 20°C, le mélange réactionnel, additionné de 20 cm³ de dichlorométhane et de 20 cm³ d'eau, est agité puis décanté. La phase organique est séchée sur sulfate de magnésium, filtrée, puis concentrée à sec sous pression réduite (2,7 kPa). Le résidu est chromatographié sur une colonne de gel de silice (granulométrie 0,063-0,200 mm, hauteur 20 cm, diamètre 2,0 cm), en éluant sous une pression de 0,1 bar d'argon avec du dichlorométhane et en recueillant des fractions de 25 cm³. Les fractions 14 à 20 sont réunies et concentrées à sec sous pression réduite (2,7 kPa). On obtient 0,66 g de N-(3,5-difluorophényl)méthylsulfonamide, sous la forme d'une poudre blanche.

Le méthylsulfonate de 1-[bis(4-chlorophényl)méthyl]azétidin-3-yle peut être préparé en opérant de la façon suivante : A une solution de 12 g de 1-[bis-(4-chlorophényl)méthyl]azéfldin-3-ol dans 200 cm³ de dichlorométhane, on ajoute sous argon en 10 minutes 3,5 cm³ de chlorure de méthylsulfonyle, puis refroidit à +5°C et coule en 10 minutes 3,8 cm³ de pyridine. Après 30 minutes d'agitation à +5°C puis 20 heures à 20°C, le mélange réactionnel est dilué avec 100 cm³ d'eau et 100 cm³ de dichlorométhane. Le mélange, d'abord filtré est décanté. La phase organique est lavée avec de l'eau, puis séchée sur sulfate de magnésium, filtrée et concentrée à sec sous pression réduite (2,7 kPa). L'huile obtenue est chromatographiée sur une colonne de gel de silice (granulométrie 0,063-0,200 mm, hauteur 40 cm, diamètre 3,0 cm), en éluant sous une pression de 0,5 bar d'argon avec un mélange de cyclohexane et d'acétate d'éthyle (70/30 en volumes) et en recueillant des fractions de 100 cm³. Les fractions 4 à 15 sont réunies et concentrées à sec sous pression réduite (2,7 kPa). On obtient 6,8 g de méthylsulfonate de 1-[bis(4-chlorophényl)méthyl]azétidin-3-yle, sous la forme d'une huile jaune.

Le 1-[bis(4-chlorophényl)méthyl]azétidin-3-ol peut être préparé selon le mode opératoire décrit par KATRITZKY A.R. et coll., J. Heterocycl. Chem., 271 (1994), en partant de 35,5 g de chlorhydrate de [bis(4-chlorophényl)méthyl]amine et 11,0cm³ d'épichlorhydrine. On isole 9,0 g de 1-[bis(4-chlorophényl)méthyl]azétidin-3-ol.

Le chlorhydrate de [bis(4-chlorophényl)méthyl]amine peut être préparé selon la méthode décrite par GRISAR M. et coll., J. Med. Chem., 885 (1973).

### Exemple 14

La N-{1-[bis-(4-chlorophényl)méthyl]azétidin-3-yl}-N-(4,6-diméthylpyrimid-2-yl)-méthylsulfonamide peut être préparée en opérant comme il est décrit dans l'exemple 13 (méthode 2), à partir de 0,20 g de N-(4,6-diméthylpyrimid-2-yl)-méthylsulfonamide et 0,308 g de 1-[bis-(4-chlorophényl)méthyl]azétidin-3-ol. Après chromatographie sur une colonne de gel de silice (granulométrie 0,06-0,04 mm, hauteur 50 cm, diamètre 2 cm), en éluant sous une pression de 0,6 bar d'argon avec du dichlorométhane puis un mélange de dichlorométhane + 1% de méthanol puis un mélange de dichlorométhane + 2% de méthanol et en recueillant des fractions de 200 cm³, les fractions 4 à 7 sont réunies et concentrées à sec sous pression réduite (2,7 kPa). Après cristallisation dans l'éther diisopropylique, filtration et séchage, on obtient 0,20 g de N-{1-[bis-(4-chlorophényl)métyl]azétidin-3-yl}-N-4,6-diméthylpyàmld-2-yl)-méthylsulfonamide sous la forme d'un solide blanc [Spectre de R.M.N. ¹H (300 MHz, CDCl₃, δ en ppm) : 2,39 (s : 6H); 2,89 (t large, J = 7,5 Hz : 2H); 3,51 (s : 3H); 3,77 (mt : 2H); 4,27 (s : 1H); 4,77 (mt : 1H); 6,73 (s : 1H); de 7,20 à 7,00 (mt : 8H)].

La N-(4,6-diméthylpyrimid-2-yl)-méthylsulfonamide peut être préparée en opérant de la façon suivante : à un mélange de 1,23 g de 2-amino-4,6-diméthylpyrimidine, 0,77 cm³ de chlorure de méthylsulfonyle et 50 mg de 4-diméthylaminopyridine dissous dans 50 cm³ de dichlorométhane, on ajoute 1,4 cm³ de triéthylamine à 0°C. Après 16 heures à température ambiante, le milieu réactionnel est lavé par 2 fois 100 cm³ d'eau, séché sur sulfate de magnésium, filtré puis évaporé à sec sous pression réduite (2,7 kPa). On obtient 1,0 g d'une poudre jaune qui est traitée avec 15 cm³ de soude à 10% à 100°C pendant 1 heure. Après refroidissement, le mélange réactionnel est extrait avec 2 fois 50 cm³ de dichlorométhane. La phase aqueuse est acidifiée à pH = 1 avec 5 cm³ d'acide chlorhydrique 10N et extraite avec 2 fois 50 cm³ de dichlorométhane. Les phases organiques obtenues sont réunies, lavées avec 50 cm³ d'eau, séchées sur sulfate de magnésium, filtrées et concentrées. On obtient 0,20 g de N-(4,6-diméthylpyrimid-2-yl)-méthylsulfonamide sous la forme d'une poudre jaune.

### Exemple 15

La N-{1-[bis-(4-chlorophényl)méthyl]azétidin-3-yl}-N-(1,3,4-thiadiazol-2-yl)-méthylsulfonamide peut être préparée en opérant comme il est décrit dans l'exemple 13 (méthode 2), à partir de 0,10 g de N-(1,3,4-thiadlazol-2-yl)-méthylsulfonamide et 0,215 g de 1-[bis-(4-chlorophényl)méthyl]azétidin-3-ol. Après chromatographie sur une colonne de gel de silice (granulométrie 0,06-0,04 mm, hauteur 25 cm, diamètre 1 cm), en éluant sous une pression de 0,8 bar d'argon avec un mélange acétate d'éthyle/cyclohexane 20/80 puis 40/60 en volume et en recueillant des fractions de 60 cm³, les fractions 26 à 31 sont réunies et concentrées à sec sous pression réduite (2,7 kPa). Après cristallisation dans l'éther diisopropylique, filtration et séchage, on obtient 40 mg de N-{1-[bis-(4-chlorophényl)méthyl]azétidin-3-yl}-N-(1,3,4-thiadiazol-2-yl)-méthylsulfonamide sous la forme d'un solide blanc [Spectre de R.M.N. ¹H (300 MHz, CDCl₃, δ en ppm) : 3,01 (s : 3H); 3,09 (t dédoublé, J = 7 et 1,5 Hz : 2H); 3,70 (t dédoublé, J = 7 et 1,5 Hz : 2H); 4,28 (s : 1H); 4,76 (mt : 1H); de 7,20 à 7,35 (mt : 8H); 9,01 (s : 1H)].

La N-(1,3,4-thiadiazol-2-yl)-méthylsulfonamide peut être préparée en opérant de la façon suivante : à un mélange de 2,02 g de 2-amino-1,3,4-thiadiazole dans 10 cm³ de pyridine, on ajoute 1,5 cm³ de chlorure de méthylsulfonyle. Après 2 heures à température ambiante, 60 cm³ d'eau sont additionnés, le milieu réactionnel est filtré. La phase aqueuse recueillie est acidifiée à pH = 2 avec de l'acide chlorhydrique 1 N, extraite avec 2 fois 50 cm³ d'acétate d'éthyle, la phase organique lavée par 2 fois 50 cm³ d'eau, séché sur sulfate de magnésium, filtré puis évaporé à sec sous pression réduite (2,7 kPa). On obtient 0,1 g d'une poudre jaune.

### Exemple 16

La N-{1-[bis-(4-chlorophényl)méthyl]azétidin-3-yl}-N-(thiazol-2-yl)-méthylsulfonamide peut être préparée en opérant comme il est décrit dans l'exemple 15, à partir de 0,50 g de N-(thiazol-2-yl)-méthylsulfonamide et 0,5 g de 1-[bis-(4-chlorophényl)méthyl]azétidin-3-ol. Après chromatographie sur une colonne de gel de silice (granulométrie 0,06-0,04 mm, hauteur 60 cm, diamètre 2 cm), en éluant sous une pression de 0,9 bar d'argon avec un mélange acétate d'éthyle/cyclohexane 20/80 puis 40/60 en volume et en recueillant des fractions de 30 cm³, les fractions 9 à 12 sont réunies et concentrées à sec sous pression réduite (2,7 kPa). Après cristallisation dans l'éther diisopropylique, filtration et séchage, on obtient 0,21 g de N-{1-[bis-(4-chlorophényl)méthyl]azétidin-3-yl}-N-(thiazol-2-yl)-méthylsulfonamide sous la forme d'un solide blanc [Spectre de R.M.N. ¹H (300 MHz, CDCl₃, δ en ppm) : de 2,95 à 3,10 (mt : 2H); 3,00 (s : 3H); 3,59 (mt : 2H); 4,22 (s large : 1H); 4,69 (mt : 1H); de 7,20 à 7,35 (mt : 9H); 7,60 (mt : 1H)].

La N-(thiazol-2-yl)-méthylsulfonamide peut être préparée en opérant de la façon suivante : à un mélange de 1,0 g de 2-aminothiazole dans 5 cm³ de pyridine, on ajoute 1,15 g de chlorure de méthylsulfonyle. Après 2 heures à température ambiante, 20 cm³ d'eau sont additionnés, le milieu réactionnel est filtré et le solide recueilli (0,35 g). La phase aqueuse recueillie est acidifiée à pH = 2 avec de l'acide chlorhydrique 1N, extraite avec 2 fois 40 cm³ d'acétate d'éthyle, la phase organique lavée par 2 fois 30 cm³ d'eau, séché sur sulfate de magnésium, filtré puis évaporé à sec sous pression réduite (2,7 kPa). On obtient 0,15 g d'un solide blanc aux caractéristiques spectrales voisines du solide filtré correspondant à un mélange N-(thiazol-2-yl)-méthylsulfonamide et N-(thiazol-2-yl)-di(méthylsulfonyl)imide que l'on utilise tel quel pour l'étape suivante.

### Exemple 17

La N-{1-[bis-(4-chlorophényl)-méthyl]-azétidin-3-yl}-N-(3-hydroxyphényl)-méthylsulfonamide peut être préparée en opérant de la façon suivante : à un mélange de 0,5 g de N-{1-[bis-(4-chlorophényl)-méthyl]-azétidin-3-yl}-N-(3-méthoxyphényl)-méthylsulfonamide dans 20 cm³ de dichlorométhane, on ajoute goutte à goutte à 2°C 7,63 cm³ d'une solution 1 M de tribromure de bore. Après 20 heures à température ambiante, le milieu réactionnel est versé sur de la glace et extrait avec 60 cm³ de dichlorométhane. La phase organique lavée par 3 fois 80 cm³ d'eau puis 2 fois par 80 cm³ d'une solution aqueuse saturée de chlorure de sodium, séché sur sulfate de magnésium, filtré puis évaporé à sec sous pression réduite (2,7 kPa). On obtient 0,33 g d'une meringue blanche qui est reprise dans de l'acétonitrile, filtrée et séchée pour obtenir 0,20 g d'un solide blanc [Spectre de R.M.N. ¹H (300 MHz, CDCl₃, δ en ppm) : 2,81 (s : 3H); 2,86 (t large, J = 7,5 Hz : 2H); 3,50 (t large, J = 7,5 Hz : 2H); 4,20 (s : 1H); 4,53 (mt : 1H); 5,36 (mf : 1H); de 6,70 à 6,85 (mt : 3H); de 7,15 à 7,35 (mt : 9H)].

### Exemple 18

La N-{1-[bis-(4-chlorophényl)-méthyl]-azétldin-3-yl}-N-(3-méthoxyphényl)-méthylsulfonamide peut être préparée en opérant comme il est décrit dans l'exemple 15, à partir de 1,58 g de N-(3-méthoxyphényl)méthylsulfonamide et 2,0 g de 1-[bis-(4-chlorophényl)méthyl]azétidin-3-ol. Après chromatographie sur une colonne de gel de silice (granulométrie 0,06-0,04 mm, hauteur 24 cm, diamètre 7,8 cm), en éluant sous une pression de 0,7 bar d'argon avec un mélange acétate d'éthyle/cyclohexane 50/50 puis 40/60 en volume et en recueillant des fractions de 100 cm³, les fractions 7 à 10 sont réunies et concentrées à sec sous pression réduite (2,7 kPa) (2,05 g). Après cristallisation dans l'éther diisopropylique, filtration et séchage, on obtient 0,21 g de N-{1-[bis-(4-chlorophényl)-méthyl]-azétidin-3-yl}-N-(3-méthoxyphényl)-méthylsulfonamide.

La N-(3-méthoxyphényl)méthylsulfonamide peut être préparée en opérant de la façon suivante : à un mélange de 5,0 g de 3-méthoxyaniline dans 150 cm³ de pyridine, on ajoute à 3°C, 3,14 cm³ de chlorure de méthylsulfonyle. Après 20 heures à température ambiante, 200 cm³ d'eau et 400 cm³ d'acétate d'éthyle sont additionnés et le milieu réactionnel décanté. La phase organique est lavée par 3 fois 400 cm³ d'eau et 400 cm³ d'une solution saturée de chlorure de sodium, séchée sur sulfate de magnésium, filtré puis évaporé à sec sous pression réduite (2,7 kPa). Après chromatographie sur une colonne de gel de silice (granulométrie 0,06-0,04 mm, hauteur 23 cm, diamètre 7,8 cm), en éluant sous une pression de 0,7 bar d'argon avec un mélange acétate d'éthyle/cyclohexane 25/75 en volume et en recueillant des fractions de 100 cm³, les fractions 24 à 36 sont réunies et concentrées à sec sous pression réduite (2,7 kPa), on obtient 6,21 g de N-(3-méthoxyphényl)méthylsulfonamide sous forme d'une huile orange.

### Exemple 19

La N-{1-[bis-(4-chlorophényl)-méthyl]-azétidin-3-yl}-N-(3-hydroxyméthylphényl)-méthylsuifonamide peut être préparée en opérant de la façon suivante : à un mélange de 0,5 g de N-{1-[bis-(4-chlorophényl)-méthyl]-azétidin-3-yl}-N-(méthytsulfonyl)-3-aminobenzoate d'éthyle dans 20 cm³ de toluène, on ajoute goutte à goutte à -50°C, 1,46 cm³ d'une solution toluènique à 20% d'hydrure de diisopropyl-aluminium. Après 1,5 heure à 0°C et 1,5 heure à 10°C, le milieu réactionnel est refroidi à 0°C et 20 cm³ d'eau sont additionnés lentement. Après filtration du précipité et extraction avec de l'acétate d'éthyle, la phase organique lavée par 2 fois 80 cm³ d'eau puis 80 cm³ d'une solution aqueuse saturée de chlorure de sodium, séché sur sulfate de magnésium, filtré puis évaporé à sec sous pression réduite (2,7 kPa). On obtient 0,46 g d'une huile qui est chromatographiée sur une colonne de gel de silice (granulométrie 0,06-0,04 mm, hauteur 16 cm, diamètre 4 cm), en éluant sous une pression de 0,7 bar d'argon avec un mélange acétate d'éthyle/cyclohexane 40/60 en volume et en recueillant des fractions de 20 cm³, les fractions 72 à 76 sont réunies et concentrées à sec sous pression réduite (2,7 kPa). On obtient 0,20 g de N-{1-[bis-(4-chlorophényl)-méthyl]-azétidin-3-yl}-N-(3-hydroxyméthyl-phényl)-méthylsulfonamide sous la forme d'un solide blanc [Spectre de R.M.N. ¹H (300 MHz, CDCl₃, δ en ppm) : 1,80 (mt : 1H); 2,83 (s : 3H); 2,87 (mt : 2H); 3,52 (mt : 2H); 4,21 (s large : 1H); 4,60 (mt : 1H); 4,74 (d large, J = 4 Hz : 2H); de 7,10 à 7,45 (mt : 12H)].

### Exemple 20

Le N-{1-[bis-(4-chlorophényl)-méthyl]-azétidin-3-yl}-N-(méthylsulfonyl)-3-aminobenzoate d'éthyle peut être préparée en opérant comme il est décrit dans l'exemple 15, à partir de 1,58 g de N-(méthylsuffonyl)-3-aminobenzoate d'éthyle et 2,0 g de 1-[bis-(4-chlorophényl)méthyl]azétidin-3-ol. Après chromatographie sur une colonne de gel de silice (granulométrie 0,06-0,04 mm, hauteur 24 cm, diamètre 7,8 cm), en éluant sous une pression de 0,7 bar d'argon avec un mélange acétate d'éthyle/clohexane 50/50 puis 40/60 en volume et en recueillant des fractions de 100 cm³, les fractions 7 à 10 sont réunies et concentrées à sec sous pression réduite (2,7 kPa) pour obtenir 2,0 g d'une huile jaune.

Le N-(méthylsuifonyl)-3-aminobenzoate d'éthyle peut être préparé en opérant de la façon suivante : à un mélange de 5,0 g de 3-aminobenzoate d'éthyle dans 150 cm³ de pyridine, on ajoute à 3°C, 2,35 cm³ de chlorure de méthylsulfonyle. Après 20 heures à température ambiante, 200 cm³ d'eau et 400 cm³ d'acétate d'éthyle sont additionnés et le milieu réactionnel décanté. La phase organique est lavée par 3 fois 400 cm³ d'eau et 400 cm³ d'une solution saturée de chlorure de sodium, séchée sur sulfate de magnésium, filtré puis évaporé à sec sous pression réduite (2,7 kPa). Après chromatographie sur une colonne de gel de silice (granulométrie 0,06-0,04 mm, hauteur 25 cm, diamètre 7,8 cm), en éluant sous une pression de 0,7 bar d'argon avec un mélange acétate d'éthyle/cyclohexane 25/75 en volume et en recueillant des fractions de 100 cm³, les fractions 27 à 36 sont réunies et concentrées à sec sous pression réduite (2,7 kPa), on obtient 5,24 g de N-(méthylsulfonyl)-3-aminobenzoate d'éthyle sous forme d'une huile orange.

### Exemple 21

Le N-{1-[bis-(4-chlorophényl)méthyl]azétidin-3-yl}-N-(1-isobutyl-pipérid-4-yl)-méthylsulfonamide peut être préparé en opérant de la façon suivante : A une solution de 0,47 g de N-{1-[bis-(4-chlorophényl)méthyl]azétidin-3-yl}-N-(pipérid-4-yl)-méthylsulfonamide dans 20 cm³ de dichlorométhane, on ajoute 0,11 cm³ d'isobutyraldéhyde, 0,057cm³ d'acide acétique à 100% et 320 mg de triacétoxyborohydrure de sodium. Après 20 heures d'agitation à 20°C, le mélange réactionnel est additionné de 50 cm³ d'une solution aqueuse saturée d'hydrogénocarbonate de sodium et décanté. La phase organique est séchée sur sulfate de magnésium, filtrée et concentrée à sec sous pression réduite (2,7 kPa). Le résidu est purifié par chromatographie sur une colonne de gel de silice (granulométrie 0,063-0,200 mm, hauteur 20 cm, diamètre 2 cm), en éluant sous une pression de 0,5 bar d'argon avec un mélange de cyclohexane et d'acétate d'éthyle (40/60 en volumes) et en recueillant des fractions de 30 cm³. Les fractions 3 à 15 sont réunies et concentrées à sec sous pression réduite (2,7 kPa). On obtient 0,22 g de N-{1-[bis-(4-chlorophényl)méthyl]azétidin-3-yl}-N-(1-isobutyl-pipérid-4-yl)-méthylsulfonamide, sous la forme d'une meringue blanche [Spectre de R.M.N ¹H (300 MHz, CDCl₃, δ en ppm) : 0,87 (d, J = 7 Hz : 6H); de 1,60 à 1,90 (mt : 5H); 1,93 (t large, J = 11,5 Hz : 2H); 2,03 (d, J = 7,5 Hz : 2H); 2,84 (s : 3H); 2,89 (d large, J = 11,5 Hz : 2H); 3,38 (t large, J = 7 Hz : 2H); 3,47 (t large, J = 7 Hz : 2H); 3,62 (mt : 1H); 4,08 (mt : 1 H); 4,43 (s : 1H); de 7,20 à 7,40 (mt : 8H)].

Le N-{1-[bis-(4-chlorophényl)méthyl]azétidin-3-yl}-N-(pipérid-4-yl)-méthylsulfonamide peut être préparé en opérant de la façon suivante : A une solution de 19 g de N-{1-[bis-(4-chlorophényl)méthyl]azétidin-3-yl}-N-(1-tert-butoxycarbonyl pipérid-4-yl)-méthylsulfonylamide dans 100 cm³ de dioxane, on coule lentement 50 cm³ d'une solution d'acide chlorhydrique 6N dans le dioxane. Après 20 heures d'agitation à 20°C, le mélange réactionnel est concentré à 50°C sous pression réduite (2,7 kPa). Le résidu, est repris par 200 cm⁹ d'acétate d'éthyle et par 200 cm³ d'eau. La phase aqueuse est alcalinisée avec une solution aqueuse d'hydroxyde de sodium 4N puis extraite par 200 cm³ d'acétate d'éthyle. Cette phase organique est séchée sur sulfate de magnésium, filtrée, puis concentrée à sec sous pression réduite (2,7 kPa). On obtient 15,5 g de N-{1-[bis-(4-chlorophényl)méthyl]azétidin-3-yl}-N-(pipéridin-4-yl)-méthylsulfonamide, sous la forme d'une meringue crème.

Le N-{1-[bis-(4-chlorophényl)méthyl]azétidin-3-yl}-N-(1-tert-butoxycarbonylpipérid-4-yl)-méthylsulfonylamide, peut être préparé en opérant de la façon suivante : A une solution de 14,7 g de 4-{1-[bis-(4-chlorophényt)méthyl]azétidin-3-ylamino}-(1-tert-butoxycarbonyl)-pipéridine dans 250 cm³ de dichlorométhane, on ajoute lentement 4,60 cm³ de chlorure de méthylsulfonyle puis 4,60 cm³ de triéthylamine et 100 mg de 4-diméthylaminopyridine. Après 20 heures d'agitation à 20°C, le mélange réactionnel est additionné de 200 cm³ d'une solution aqueuse saturée d'hydrogénocarbonate de sodium puis agité pendant 30 minutes et décanté. La phase organique est séchée sur sulfate de magnésium, filtrée, puis concentrée à sec sous pression réduite (2,7 kPa). La meringue obtenue reprise par 250 cm³ de dichlorométhane, est à nouveau additionnée lentement de 4,60 cm³ de chlorure de méthylsulfonyle puis de 4,60 cm³ de triéthylamine et de 100 mg de 4-diméthylaminopyridine. Après 20 heures d'agitation à 20°C, le mélange est additionné de 200 cm³ d'une solution aqueuse saturée d'hydrogénocarbonate de sodium puis agité pendant 30 minutes et décanté. La phase organique est séchée sur sulfate de magnésium, filtrée, puis concentrée à sec sous pression réduite (2,7 kPa). Le résidu est purifié par chromatographie sur une colonne de gel de silice (granulométrie 0,063-0,200 mm, hauteur 35 cm, diamètre 5 cm), en éluant sous une pression de 0,5 bar d'argon avec un mélange de cyclohexane et d'acétate d'éthyle (70/30 en volumes) et en recueillant des fractions de 250 cm³. Les fractions 4 à 18 sont réunies et concentrées à sec sous pression réduite (2,7 kPa). On obtient 19 g de N-{1-[bis-(4-chlorophényl)méthyl]azétidin-3-yl}-N-(1-tert-butoxycarbonylpipérid-4-yl)-méthylsulfonylamide sous la forme d'une meringue crème, qui sera utilisée telle quelle à l'étape suivante.

La 4-{1-[bis-(4-chlorophényl)méthyl]azétidin-3-ylamino}-(1-tert-butoxycarbonyl)-pipéridine peut être préparée en opérant de la façon suivante : A une solution de 9,22 gde 1-[bis(4-chlorophényl)méthyl]azétidin-3-ylamine, dans 300 cm³ de dichlorométhane, on ajoute 6,58 g 1-tert-butoxycarbonyl-pipéridin-4-one. Au mélange, refroidi à +5°C on ajoute en deux portions 9,54 g de triacétoxyborohydrure de sodium, puis coule 1,72 cm³d'acide acétique à 100%. Après 20 heures d'agitation à 20°C, le mélange réactionnel est additionné lentement de 500 cm³ d'une solution aqueuse saturée d'hydrogénocarbonate de sodium, puis bien agité et décanté. La phase organique est séchée sur sulfate de magnésium, filtrée et concentrée à sec à 50°C sous pression réduite (2,7 kPa). On obtient 15 g de 4-{1-[bis-(4-chlorophényl)rnéthyl]azétidin-3-ylamino}-(1-tert-butoxycarbonyl)-pipéridine sous la forme d'une meringue crème qui sera utilisée telle quelle à l'étape suivante.

### Exemple 22

N-benzyl-N-{1-[bis(4-chlorophényl)méthyl]azétidin-3-yl}amine : A une solution de 369 mg de 1-[bis(4-chlorophényl)méthyl]azétidin-3-ylamine dans 15 cm³ de dichlorométhane on ajoute, à température ambiante sous atmosphère d'argon, 0,134 cm³ de benzaldéhyde. Le mélange est refroidi vers 0°C, avant d'y ajouter progressivement 382 mg de triacétoxyborohydrure de sodium, puis 70 mm³ d'acide acétique. Après 16 heures d'agitation à température ambiante le mélange est versé sur 50 cm³ d'une solution aqueuse saturée d'hydrogénocarbonate de sodium, puis extrait par deux fois 25 cm³ de dichlorométhane. Les phases organiques réunies sont séchées sur sulfate de magnésium, filtrées et concentrées à sec sous pression réduite (2,7 kPa). Le résidu obtenu est purifié par chromatographie-flash sur gel de silice [éluant : dichlorométhane/méthanol (95/5 en volumes)]. On obtient 0,29 g de N-benzyl-N-{1-[bis(4-chlorophényl)méthyl]azétidin-3-yl}amine sous forme d'une huile incolore [Spectre de R.M.N. ¹H (300 MHz, CDCl₃, δ en ppm) : 2,71 (t large, J = 7 Hz : 2H); 3,42 (mt : 2H); 3,49 (mt : 1H); 3,70 (s : 2H); 4,25 (s : 1H); de 7,20 à 7,40 (mt : 13H)].

La 1-[bis(4-chlorophényl)méthyl]azétidin-3-yl-amine peut être obtenue de la manière suivante: A. 27 g de méthylsulfonate de 1-[bis(4-chlorophényl)méthyl]azétidin-3-yle contenus dans un autoclave préalablement refroidi vers -60°C on ajoute 400 cm³ d'un mélange de méthanol et d'ammoniac liquide (50/50 en volumes). Le milieu réactionnel est ensuite agité à 60°C pendant 24 heures, puis abandonné à l'air libre pour permettre l'évaporation de l'ammoniac et enfin concentré sous pression réduite (2,7 kPa). Le résidu est repris par 500 cm³ d'une solution aqueuse 0,37N d'hydroxyde de sodium et extrait par quatre fois 500 cm³ d'éther éthylique. Les phases organiques réunies sont lavées successivement avec deux fois 100 cm³ d'eau distillée et 100 cm³ d'une solution saturée de chlorure de sodium, séchées sur du sulfate de magnésium, filtrées et concentrées sous pression réduite (2,7 kPa). Le résidu obtenu est purifié par chromatographie-flash sur gel de silice [éluant : dichlorométhane/méthanol (95/5 en volumes)]. On obtient 14,2 g de 1-[bis(4-chlorophényl)méthyl]azétidin-3-yl-amine sous forme d'une huile, qui concrétise en un solide de couleur crème.

### Exemple 23

N-benzyl-N-{1-[bis(4-chlorophényl)méthyl]azétidin-3-yl}méthylsulfonamide : A une solution de 120 mg de N-benzyl-N-{1-[bis(4-chlorophényl)méthyl]azétidin-3-yl}amine dans 5 cm³ de dichlorométhane on ajoute, à température ambiante sous atmosphère d'argon, 104 mm³ de triéthylamine. Le mélange est refroidi vers 0°C, avant d'y ajouter 46,4 mm³ de chlorure de méthylsulfonyle, puis il est agité à température ambiante pendant 16 heures. Le mélange réactionnel est dilué avec 20 cm³ de dichlorométhane, puis est lavé avec deux fois 15 cm³ d'eau distillée. La phase organique est séchée sur sulfate de magnésium, filtrée et concentrée à sec sous pression réduite (2,7 kPa), fournissant une laque que l'on fait cristalliser par trituration dans le méthanol. On obtient ainsi 42 mg de N-benzyl-N-{1-[bis(4-chlorophényl)méthyl]azétidin-3-yl}méthylsulfonamide, sous forme d'une poudre crème fondant à 171°C.

### Exemple 24

La N-{1-[bis(4-chlorophényl)méthyl]azétidin-3-yl}-N-(3,5-difluorobenzyl)amine peut être préparée comme dans l'exemple 22 mais en utilisant 188 mg de 3,5-difluorobenzaldéhyde et 369 mg de 1-[bis(4-chlorophényl)méthyl]azétidin-3-yl-amine et de 382 mg de triacétoxyborohydrure de sodium, sans purification par chromatographie-flash. On obtient 0,48 g de N-{1-[bis(4-chlorophényl)méthyl]azétidin-3-yl}-N-(3,5-difluorobenzyl)amine sous forme d'une huile incolore [Spectre de R.M.N. ¹H (300 MHz, CDCl₃, δ en ppm) : 2,73 (mt : 2H); de 3,40 à 3,55 (mt : 3H); 3,70 (s : 2H); 4,26 (s : 1H); 6,69 (tt, J = 9 et 2 Hz : 1H); 6,83 (mt : 2H); de 7,20 à 7,35 (mt : 8H)].

### Exemple 25

N-{1-[bis(4-chlorophényl)méthyl]azétidin-3-yl}-N-(3,5-difluorobenzyl)méthylsulfonamide
A une solution de 433 mg de N-{1-[bis(4-chlorophényl)méthyl]azétidin-3-yl}-N-(3,5-difluorobenzyl)amine dans 30 cm³ de dichlorométhane on ajoute, à température ambiante sous atmosphère d'argon, 347 mm³ de triéthylamine. Le mélange est refroidi vers 0°C, avant d'y ajouter une solution de 46,4 mm³ de chlorure de méthylsulfonyle dans 5 cm³ de dichlorométhane, puis il est agité à température ambiante pendant 1 heure. Le mélange réactionnel est dilué avec 20 cm³ de dichlorométhane, puis est lavé avec deux fois 20 cm³ d'eau distillée. La phase organique est séchée sur sulfate de magnésium, filtrée et concentrée à sec sous pression réduite (2,7 kPa). Le résidu est introduit en solution dans le méthanol sur une cartouche Bond Elut^{®} SCX (10 g), en éluant successivement par du méthanol et par une solution 1 M d'ammoniac dans le méthanol. Les fractions ammoniacales sont jointes et concentrées à sec sous pression réduite (2,7 kPa). On obtient ainsi 0,44 g de N-{1-[bis(4-chlorophényl)méthyl]azétidin-3-yl}-N-(3,5-difluorobenzyl)méthylsulfonamide sous la forme d'une meringue de couleur crème [Spectre de R.M.N. ¹H (300 MHz, CDCl₃, δ en ppm) : 2,81 (s : 3H); 3,02 (t large, J = 7,5 Hz : 2H); 3,38 (t large, J = 7,5 Hz : 2H); 4,23 (s : 1H); 4,40 (mt : 1H); 4,54 (s : 2H); 6,75 (tt, J = 9 et 2 Hz : 1H); 6,95 (mt : 2H); 7,25 (mt : 8H)].

### Exemple 26

N-{1-[bis(4-chlorophényl)méthyl]azétidin-3-yl}-N-(3,5 difluorobenzyl)acétamide
A une solution de 2 g de N-{1-[bis(4-chlorophényl)méthl]azétidin-3-yl}-N-(3,5-difluorobenzyl)amine dans 75 cm³ de dichlorométhane on ajoute, à température ambiante sous atmosphère d'argon, 1,6 cm³ de triéthylamine. Le mélange est refroidi vers 0°C avant d'y ajouter goutte à goutte 0,66 cm³ de chlorure d'acétyle, puis il est agité à température ambiante pendant 16 heures. Le mélange réactionnel est dilué avec 50 cm³ de dichlorométhane, puis est lavé avec deux fois 20 cm³ d'eau distillée. La phase organique est séchée sur sulfate de magnésium, filtrée et concentrée à sec sous pression réduite (2,7 kPa). Le résidu obtenu est purifié par chromatographie-flash sur gel de silice [éluant : dichlorométhane/méthanol (98/2 en volumes)]. On obtient 1,2 g de N-{1-[bis(4-chlorophényl)méthyl]azétidin-3-yl}-N-(3,5 difluorobenzyl)acétamide sous forme d'une huile incolore [Spectre de R.M.N. ¹H (300 MHz, CDCl₃, δ en ppm). On observe un mélange de rotamères. * 2,06 et 2,14 (2s : 3H en totalité); 2,97 (mt : 2H); 3,43 (mt : 2H); 4,20 et 4,25 (2s : 1H en totalité); 4,54 et de 4,75 à 4,80 (mt : 1H en totalité); 4,68 et 4,78 (2s larges : 2H en totalité); 6,70 (mt : 3H); 7,24 (s large : 8H)].

### Exemple 27

N-{1-[bis(4-chlorophényl)méthyl]azétidin-3-yl}-N-(pyrid-4-yl-méthyl)-méthylsulfonamide
A une solution de 398 mg de N-{1-[bis(4-chlorophényl)méthyl]azétidin-3-yl}-N-(pyrid-4-ylméthyl)amine dans 8 cm³ de dichlorométhane on ajoute, à température ambiante sous atmosphère d'argon, 346 mm³ de triéthylamine. Le mélange est refroidi vers 0°C, avant d'y ajouter 155 mm³ de chlorure de méthylsulfonyle, puis il est agité à température ambiante pendant 3 heures. Le mélange réactionnel est dilué avec 35 cm³ de dichlorométhane, puis est lavé avec deux fois 20 cm³ d'eau distillée. La phase organique est séchée sur sulfate de magnésium, filtrée et concentrée à sec sous pression réduite (2,7 kPa). Le résidu obtenu est purifié par chromatographie-flash sur gel de silice [éluant : dichlorométhane/méthanol (97/3 en volumes)]. On obtient 288 mg de N-{1-[bis(4-chlorophényl)méthyl]azétidin-3-yl}-N-(pyrid-4-yl-méthyl)-méthylsulfonamide sous la forme d'une meringue de couleur crème [Spectre de R.M.N. ¹H (300 MHz, CDCl₃, δ en ppm) : 2,83 (s : 3H); 3,02 (t large, J = 7,5 Hz : 2H); 3,40 (t large, J = 7,5 Hz : 2H); 4,23 (s : 1H); 4,43 (mt : 1H); 4,57 (s : 2H); de 7,20 à 7,35 (mt : 8H); 7,32 (d large, J = 5,5 Hz : 2H); 8,60 (d large, J = 5,5 Hz : 2H)].

### Exemple 28

La N-{1-[bis(4-chlorophényl)méthyl]azétidin-3-yl}-N-(pyrid-4-yl-méthyl)amine peut être préparée de la manière suivante : A une solution de 369 mg de 1-[bis(4-chlorophényl)méthyl]azétidin-3-ylamine dans 15 cm³ de dichlorométhane on ajoute, à température ambiante sous atmosphère d'argon, 0,126 cm³ de pyrid-4-yl-carboxaldéhyde. Le mélange est refroidi vers 0°C, avant d'y ajouter progressivement 382 mg de triacétoxyborohydrure de sodium, puis 70 mm³ d'acide acétique. Après 72 heures d'agitation à température ambiante le mélange est versé sur 100 cm³ d'une solution aqueuse saturée d'hydrogénocarbonate de sodium, puis extrait par deux fois 100 cm³ de dichlorométhane. Les phases organiques réunies sont lavées avec 50 cm³ d'eau distillée, séchées sur sulfate de magnésium, filtrées et concentrées à sec sous pression réduite (2,7 kPa). Le résidu est introduit en solution dans 5 cm³ de méthanol sur une cartouche Bond Elut^{®} SCX (10 g), en éluant successivement par 50 cm³ de méthanol et par 60 cm³ d'une solution 1 M d'ammoniac dans le méthanol. Les fractions ammoniacales sont jointes et concentrées à sec sous pression réduite (2,7 kPa). On obtient ainsi 0,48 g de N-{1-[bis(4-chlorophényl)méthyl]azétidin-3-yl}-N-(pyrid-4-yl-méthyl)amine sous la forme d'une huile incolore.

### Exemple 29

N-(1-[bis(4-chlorophényi)méthy]azétidin-3-yl)-N-(pyrid-3-yl-méthyl)-méthylsulfonamide
En opérant selon le mode opératoire de l'exemple 27 mais à partir de 380 mg de N-{1-[bis(4-chtorophényl)méthyl]azétidin-3-yl}-N-(pyrid-3-yl-méthyl)amine, on obtient 319 mg de N-{1-[bis(4-chlorophényl)méthyl]azétidin-3-yl}-N-(pyrid-3-yl-méthyl)-méthylsulfonamide sous forme d'une meringue de couleur crème [Spectre de R.M.N. ¹H (300 MHz, CDCl₃, δ en ppm) : 2,80 (s : 3H); 3,02 (t dédoublé, J = 7 et 1,5 Hz : 2H); 3,38 (t dédoublé, J = 7 et 1,5 Hz : 2H); 4,22 (s : 1H); 4,35 (mt : 1H); 4,56 (s : 2H); 7,23 (s large : 8H); 7,31 (dd, J = 8 et 5 Hz : 1H); 7,80 (d large, J = 8 Hz : 1H); 8 ,57 (dd, J = 5 et 1,5 Hz : 1H); 8,63 (d large, J = 1,5 Hz : 1H)].

### Exemple 30

La N-{1-[bis(4-chlorophényl)méthyl]azétidin-3-yl}-N-(pyrid-3-yl-méthyl)amine peut être préparée comme dans l'exemple 28 mais à partir de 0,124 cm³ de pyrid-3-yl-carboxaldéhyde, 0,36 g de 1-[bis(4-chlorophényl)méthyl]azétidin-3-yi-amine et de 0,38 g de triacétoxyborohydrure de sodium. On obtient ainsi 0,44 g de N-{1-[bis(4-chlorophényl)méthyl]azétidin-3-yl}-N-(pyrid-3-yl-méthyl)amine sous la forme d'une huile incolore.

### Exemple 31

N-{1-[bis(4-chlorophényl)méthyl]azétidin-3-yl}-N-(1,1-dioxo-1H-1λ⁸-benzo[d]isothiazol-3-yl)-amine
A 386 mg de méthylsulfonate de 1-[bis(4-chlorophényl)méthyl]azétidin-3-yle en solution dans 10 cm³ de diméthylformamide on ajoute 182 mg de 1,1-dioxyde de 1,2-benzisothiazol-3-amine et 326 mg de carbonate de césium. Le milieu réactionnel est ensuite agité à 100°C pendant 9 heures, puis concentrée sous pression réduite (2,7 kPa). Le résidu est lavé quatre fois avec 5 cm³ d'eau distillée bouillante, désagrégé par agitation dans 5 cm³ d'eau distillée à température ambiante, puis recueilli par filtration et purifié par chromatographie-flash sur gel de silice [éluant : dichlorométhane/méthanol (98/2 en volumes)]. On obtient 53 mg de N-{1-[bis(4-chlorophényl)méthyl]azétidin-3-yl}-N-(1,1-dioxo-1H-1λ⁶-benzo[d]isothiazol-3-yl)-amine sous forme d'un produit pâteux [[Spectre de R.M.N. ¹H (300 MHz, CDCl₃, 8 en ppm) : 3,17 (mt : 2H); 3,61 (t large, J = 7,5 Hz : 2H); 4,37 (s : 1H); 4,75 (mt : 1H); de 6,30 à 6,40 (mf : 1H); de 7,20 à 7,35 (mt : 8H); 7,62 (d large, J = 7,5 Hz : 1H); 7,69 (t large, J = 7,5 Hz : 1H); 7,76 (t large, J = 7,5 Hz : 1H); 7,93 (d large, J = 7,5 Hz : 1H)].

Le 1,1-dioxyde de 1,2-benzisothiazol-3-amine peut être préparé selon la méthode décrite par Stoss, P. et coll., Chem. Ber. (1975), 108(12), 3855-63.

### Exemple 32

Le N-{1-[bis-(4-chlorophényl)méthyl]-azétidin-3-yl}-N-(3,5-difluorophényl)-N'-tert-butyloxycarbonylsulfamide peut être préparé de la manière suivante : A une solution de 0,095 cm³ d'alcool tertbutylique dans 2 cm³ de dichlorométhane anhydre, est ajouté 0,048 cm³ de chlorosulfonylisocyanate, après 2 minutes d'agitation sont ajoutés successivement, 0,21 g N-1-[bis-(4-chlorophényl)méthylj-azéfldin-3-yl}-N-(3,5-difluorophényl)-amine dans 1,25 cm³ de dichlorométhane anhydre, puis 0,084 cm³ de triéthylamine. Après 1 heure d'agitation à une température voisine de 20°C, est ajouté sous vive agitation 2 cm³ d'une solution saturée de bicarbonate de sodium. Le milieu réactionnel est décanté, séché sur sulfate de magnésium, filtré puis concentré à sec sous pression réduite (2,7 kPa). Le résidu obtenu est chromatographié sur une cartouche Varian (6 cm3) garnie avec 3 g de silice fine (0,040-0,063 mm), conditionnée puis éluée avec un mélange éther de pétrole-acétate d'éthyle à l'aide d'une pompe Duramat en recueillant des fractions de 2 cm3. Les fractions 6 à 17 sont réunies et concentrées à sec sous pression réduite (2,7 kPa) à 40°C pendant 2 heures. On obtient ainsi 61 mg de N-{1-[bis-(4-chlorophényl)méthyl]-azétidin-3-yl}-N-(3,5-difluorophényl)-N'-tert-butyloxycarbonylsulfamide sous forme de meringue blanche [Spectre de R.M.N ¹H (400 MHz, CDCl₃, δ en ppm) : 1,47 (s : 9H); 2,77 (t large, J = 8 Hz : 2H); 3,52 (mt : 2H); 4,19 (s : 1H); 5,06 (mt : 1H); de 6,75 à 6,90 (mt : 3H); de 7,15 à 7,35 (mt : 8H].

### Exemple 33

Le (RS)-N-{1-[(4-chlorophényl)-pyridin-3-yl-méthyl]-azétidin-3-yl}-N-(3,5-difluorophényl)-méthylsulfonamide peut être obtenu de la façon suivante : A un mélange de 0,2 g de 3-[bromo-(4-chlorophényl)-méthyl]-pyridine et de 0,22 g de chlorhydrate de N-azétidin-3-yl-N-(3,5-difluorophényl)-méthylsulfonamide dans 10 cm³ d'acétonitrile on ajoute 0,2 g de carbonate de potassium et 23 mg de iodure de potassium puis on chauffe le mélange à reflux pendant 3 heures. Après avoir rajouté 0,2 g de carbonate de potassium on chauffe au reflux pendant 15 heures supplémentaires. Après refroidissement à 21°C, on élimine les matières insolubles par filtration puis on concentre a sec à 40°C sous 2,7 kPa. On obtient 170 mg d'une laque incolore que l'on purifie par chromatographie sur cartouche de silice (référence SIL-020-005, FlashPack, Jones Chromatography Limited, New Road, Hengoed, Mid Glamorgan, CF82 8AU, Royaume Uni) en éluant avec un mélange de cyclohexane : d'acétate d'éthyle 1 :1 (6 cm³/min, fractions de 5 cm3). Les fractions de Rf=5/57 (cyclohexane:acétate d'éthyle 1:1, plaque de silice, Merck référence 1.05719, Merck KGaA, 64271 Darmstatd, Allemagne) sont réunies et concentrées sous 2,7 kPa à 40°C pour conduire à 100 mg de (RS)-N-{1-[(4-chlorophényl)-pyridin-3-yl-méthyl]-azétidin-3-yl}-N-(3,5-difluorophényl)-méthylsulfonamide fondant à 110°C [Spectre de R.M.N. ¹H (300 MHz, (CD₃)SO d6, δ en ppm) : 2,77 (mt : 2H); 2,98 (s : 3H); 3,38 (mt : 2H); 4,50 (s : 1H); 4,70 (mt : 1H); 7,11 (mt : 2H); de 7,20 à 7,40 (mt : 2H); 7,34 (d, J = 8 Hz : 2H); 7,41 (d, J = 8 Hz : 2H); 7,72 (d large, J = 8 Hz : 1H); 8,40 (dd, J = 5 et 1,5 Hz : 1H); 8,58 (d, J = 1,5 Hz : 1H)].

Les deux isomères du (RS)-N-{1-[(4-chloro-phényl)-pyridin-3-yl-méthyl]-azétidin-3-yl}-N-(3,5-difluoro-phényl)-méthanesulfonamide peuvent être séparés sur phase stationnaire chirale CHIRACEL OD.

Premier isomère : spectre de R.M.N. ¹H (300 MHz, CDCl₃, δ en ppm) : 2,82 (s : 3H); 2,87 (mt : 2H); 3,53 (mt : 2H); 4,29 (s : 1H ); 4,47 (mt : 1H); 6,80 (mt : 3H); 7,19 (dd, J = 8 et 5 Hz : 1H); de 7,20 à 7,35 (mt : 4H); 7,62 (d large, J = 8 Hz : 1H); 8,45 (d large, J = 5 Hz : 1H); 8,59 (s large : 1H).

Deuxième isomère : spectre de R.M.N. ¹H (300 MHz, CDCl₃, δ en ppm) : 2,82 (s : 3H); 2,87 (mt : 2H); 3,54 (mt : 2H); 4,29 (s : 1H); 4,48 (mt : 1H); 6,80 (mt : 3H); 7,19 (dd large, J = 8 et 5 Hz : 1H); de 7,25 à 7,35 (mt : 4H); 7,62 (dt, J = 8 et 2 Hz : 1H); 8,46 (dd, J = 5 et 2 Hz : 1H); 8,59 (d large, J = 2 Hz : 1H).

Le chlorhydrate de N-azétidin-3-yl-N-(3,5-difluorophényl)-méthylsulfonamide est obtenu de la façon suivante : Dans un hydrogénateur de 500 cm³, une solution de 1 g de N-(1-benzhydryl-azétidin-3-yl)-N-(3,5-difluorophényl)-méthylsulfonamide dans un mélange de 2,5 cm³ d'acide chlorhydrique 1 M et de 0,41 cm³ d'acide acétique est hydrogénée en présence de 0,161 g d'hydroxyde de palladium sous 30 bars d'hydrogène pendant 4 heures. Le catalyseur est éliminé par filtration sur un lit de célite puis le filtrat est concentré à sec à 40°C sous 2,7 kPa pour donner 630 mg de N-azétidin-3-yl-N-(3,5-difluorophényl)-méthylsulfonamide, fondant à 216°C.

Le N-(1-benzhydryl-azétidin-3-yl)-N-(3,5-difluorophényl)-méthylsulfonamide peut être obtenu en opérant comme dans l'exemple 13 (méthode 2) de la façon suivante : A une solution de 2 g de 1-benzhydryl-azétidin-3-ol dans 100 cm³ de tétrahydrofuranne on ajoute successivement 0,86 g de N-(3,5-difluorophényl) méthylsulfonamide, 3,28g de triphényl phosphine puis 2 ml de diéthyl azodicarboxylate. On observe une augmentation de la température qui passe de 22°C à 29°C, ainsi que la formation d'un précipité dès la fin de l'addition du diéthyl azodicarboxylate. Après 20h à 22°C, on élimine le précipité par filtration puis on concentre le filtrat à sec à 40°C sous 2,7 kPa. Le résidu est trituré avec 5 cm³ de méthanol pendant 20 minutes à 21°C fournissant 1,07g de N-(1-benzhydryl-azétidin-3-yl)-N-(3,5-difluorophényl)-méthylsulfonamide sous forme d'un solide amorphe blanc.

Le 1-benzhydryl-azétidin-3-ol peut être préparé selon le mode opératoire décrit par KATRITZKY A.R. et coll., J. Heterocycl. Chem., 271 (1994).

La 3-[bromo-(4-chlorophényi)-méthyl]-pyridine est obtenue de la façon suivante : A 1,5g de (4-Chlorophényl)-pyridin-3-yl-methanol on ajoute 3,5 cm³ d'une solution d'acide bromhydrique à 48% dans l'acide acétique et 1 cm³ de bromure d'acétyle. Le mélange de couleur ambrée ainsi obtenu est chauffé au reflux pendant 4 heures puis refroidi à 20°C, concentré à sec à 40°C sous 2,7 kPa conduisant à 1,53g de 3-[bromo-(4-chlorophényl)-méthyl]-pyridine (Rf=75/90, 254nm, Plaques de Silice, référence 1.05719, Merck KGaA, 64271 Darmstatd, Allemagne).

Le (4-chlorophényl)-pyridin-3-yl-methanol est obtenu de la façon suivante : A une solution de 3 g de pyridine-3-carboxaldéhyde dans le tétrahydrofuranne à 5°C, on ajoute 20 cm³ d'une solution molaire de bromure de 4-chlorophényl magnésium dans l'éther éthylique. Après réchauffement à 20°C, on laissé réagir pendant 15 heures sous agitation. On ajoute alors 20 cm³ d'une solution saturée de chlorure d'ammonium puis 20 cm³ d'acétate d'éthyle. Le mélange est décanté et les phases organiques sont extraites avec 20 cm³ d'acétate d'éthyle supplémentaires. Les extraits organiques sont réunis, séchés sur sulfate de magnésium puis concentrés à sec à 40°C sous 2,7 kPa. Le résidu obtenu est chromatographié sur silice (Amicon, 20-45 µm, 500 g silice, colonne de diamètre 5 cm) en éluant avec un mélange de cyclohexane : acétate d'éthyle de 80 : 20 à 50 : 50 sous une pression d'argon de 0,4 bar. Les fractions contenant le composé de Rf=13/53 (Plaques de Silice Merck, référence 1.05719, Merck KGaA, 64271 Darmstatd, Allemagne) sont réunies et évaporées à sec à 40°C sous 2,7 kPa pour donner 2,53 g de 4-Chlorophényl)-pyridin-3-yl-methanol.

### Exemple 34

Le N-{1-[bis-(4-fluoro-phényl)-méthyl]-azétidin-3-yl}-N-(3,5-difluorophényl)-méthylsulfonamide est obtenu de la façon suivante : A un mélange de 0,2 g de chlorure de 4,4'-difluorobenzhydryle et de 0,26 g de chlorhydrate de N-azétidin-3-yl-N-(3,5-difluorophényl)-méthylsulfonamide dans 10 cm³ d'acétonitrile on ajoute 0,36 g de carbonate de potassium et 27 mg de iodure de potassium puis on chauffe le mélange à reflux pendant 3 heures. Après refroidissement à 21 °C, on élimine les matières insolubles par filtration puis on concentre a sec à 40°C sous 2,7 kPa. On triture le résidu avec 30 cm³ d'acétate d'éthyle puis on élimine le solide par filtration. On concentre le filtrat à sec à 40°C sous 2,7 kPa et on obtient 90 mg de solide jaune pâle que l'on purifie par chromatographie sur cartouche BondElut SCX contenant 2 g de silice greffée (référence 1225-6019, Varian Associates, Inc. 24201 Frampton Avenue, Harbor City, CA90710, USA) en éluant avec une solution à 2M d'ammoniaque méthanolique. Les fractions de Rf=16/82 (cyclohexane-acétate d'éthyle 7/3), plaque de silice, référence 1.05719, Merck KGaA, 64271 Darmstatd, Allemagne) sont réunies et concentrées sous 2,7 kPa à 40°C pour conduire à 243 mg de N-{1-[bis-(4-fluoro-phényl)-méthyl]-azétidin-3-yl}-N-(3,5-difluorophényl)-méthylsulfonamide fondant à 98°C [Spectre de R.M.N. ¹H (300 MHz, (CD₃)₂SO d6, δ en ppm) : 2,74 (t large, J = 7 Hz : 2H); 3,00 (s : 3H); 3,37 (t large, J = 7 Hz : 2H); 4,43 (s : 1H); 4,69 (mt : 1H); de 7,05 à 7,20 (mt : 6H); 7,28 (tt, J = 9 et 2,5 Hz : 1H); 7,40 (mt : 4H)].

### Exemple 35

Le (RS)-N-{1-[(4-chlorophényl)-pyridin-4-yl-méthyl]-azétidin-3-yl}-N-(3,5-difluorophényl)-méthylsutfonamide peut être obtenu de la manière suivante : Un mélange d'environ 100 mg de (4-pyridyl)-(4-chlorophényl)-chlorométhane, 143 mg de chlorhydrate de N-azétidin-3-yl-N-(3,5-difluorophényl)-méthylsulfonamide, 17 mg d'iodure de potassium et 200 mg de carbonate de potassium dans 5 cm³ d'acétonitrile, est agité environ 18 heures à une température voisine de 20°C. Le mélange réactionnel est ensuite porté au reflux pendant 3 heures, additionné de 17 mg d'iodure de potassium et laissé au reflux pour 2 heures supplémentaires. Après refroidissement jusqu'à une température voisine de 20°C, le milieu réactionnel est filtré sur verre fritté. Le solide est rincé avec de l'acétonitrile, puis 2 fois 3 cm³ d'acétate d'éthyle. Les filtrats sont concentrés à sec sous pression réduite. On obtient 230 mg d'une pâte jaune pâle que l'on purifie par chromatographie préparative sur couche mince de silice [4 plaques préparatives Merck Kieselgel 60F254; 20x20 cm; épaisseur 0,5 mm], en éluant par un mélange méthanol-dichlorométhane (5-95 en volumes). Après élution de la zone correspondant au produit recherché, filtration sur verre fritté, puis évaporation des solvants sous pression réduite à une température voisine de 40°C, on obtient 12 mg de (RS)-N-{1-[(4-chlorophényl)-pyridin-4-yl-méthyl]-azétidin-3-yl}-N-(3,5-difluorophényl)-méthylsulfonamide [Spectre de R.M.N. ¹H (300 MHz, CDCl₃, δ en ppm) : 2,82 (s : 3H); 2,96 (mf : 2H); de 3,50 à 3,80 (mt : 2H); 4,33 (mf : 1H); 4,54 (mt : 1H); 6,82 (mt : 3H); de 7,20 à 7,45 (mt : 6H); 8,53 (d large, J = 5,5 Hz : 2H)].

Les deux isomères du (RS)-N-{1-[(4-chloro-phényl)-pyridin-4-yl-méthyl]-azétidin-3-yl}-N-(3,5-difluoro-phényl)-méthanesutfonamide peuvent être séparés sur phase stationnaire chirale CHIRACEL OJ.

Premier isomère : spectre de R.M.N. ¹H (300 MHz, CDCl₃, δ en ppm): 2,83 (s : 3H); 2,87 (t large, J = 7,5 Hz : 2H); 3,51 (mt : 1H); 3,60 (mt : 1H ); 4,24 (s : 1H); 4,50 (mt : 1H); 6,82 (mt : 3H); de 7,20 à 7,35 (mt : 6H); 8,50 (d large, J = 5,5 Hz : 2H).

Deuxième isomère : spectre de R.M.N. ¹H (300 MHz, CDCl₃, δ en ppm) : 2,83 (s : 3H); 2,88 (t, J = 7,5 Hz : 2H); 3,51 (mt : 1H); 3,61 (mt : 1H); 4,25 (s : 1H); 4,51 (mt : 1H); 6,81 (mt : 3H); de 7,20 à 7,35 (mt : 6H); 8,50 (d large, J = 5,5 Hz : 2H).

Le (4-pyridyl)(4-chlorophényl)-chlorométhane peut être préparé de la manière suivante : A une suspension de 100 mg de (4-pyridyl)-(4-chlorophényl)-méthanol dans 2 cm³ de toluène, refroidie à une température voisine de 0°C, on ajoute 0,0598 cm³ de chlorure de thionyle. Après 2 heures à une température voisine de 0°C et 1 heure à une température voisine de 20°C, le milieu réactionnel est concentré sous pression réduite. On obtient environ 100 mg de (4-pyridyl)-(4-chlorophényl)-chlorométhane sous forme d'un solide blanc.

Le (4-pyridyl)-(4-chtorophényl)-méthanol peut être préparé de la manière suivante : A une solution de 2 g de 4-(4-chlorobenzoyl)-pyridine dans 160 cm³ d'éthanol, sont ajoutés, à une température voisine de 20°C, 348 mg de tétrahydroborure de sodium. Après 2 heures d'agitation à une température voisine de 20°C, on ajoute 90 mg de tétrahydroborure de sodium. Après environ 1,5 heures à la même température, on dilue le milieu réactionnel avec 200 cm³ de dichlorométhane et 200 cm³ d'eau. Le pH de la phase aqueuse est ajusté à environ une valeur de 5 par ajout d'environ 13 cm³ d'une solution aqueuse d'acide chlorhydrique 1N. Après décantation, la phase aqueuse est extraite avec 3 fois 100 cm³ de dichlorométhane. Les phases organiques sont rassemblées, séchées sur sulfate de magnésium, filtrées et concentrées sous pression réduite. On obtient ainsi 2 g de (4-pyridyl)-(4-chlorophényl)-méthanol sous forme d'une poudre blanche.

### Exemple 36

A une solution de 330 mg de {1-[bis(4-chlorophényl)méthyl]azétidin-3-yl}(3,5-difluorobenzyl)amine dans 25 cm³ de tétrahydrofuranne on ajoute, à température ambiante sous atmosphère d'argon, 24,4 mg d'une dispersion d'hydrure de sodium à 75% dans l'huile minérale. Le mélange est agité à température ambiante pendant 1 heure avant d'y ajouter 59 mm³ de chloroformiate de méthyle, puis l'agitation est maintenue 18 heures dans les mêmes conditions. Le mélange réactionnel est additionné de 0,3 cm³ d'eau distillée et le tétrahydrofuranne est chassé au rotavapor. Le résidu obtenu est extrait au dichlorométhane, la phase organique est séchée sur sulfate de magnésium, filtrée et concentrée à sec sous pression réduite (2,7 kPa). Le résidu obtenu est purifié par chromatographie-flash sur gel de silice [éluant : dichlorométhane/méthanol (97,512,5 en volumes)]. On obtient 328 mg de {1-[bis(4-chlorophényl)méthyl]azétidin-3-yl}-(3,5-difluorobenryl)carbamate de méthyle sous forme d'une huile incolore [Spectre de R.M.N. ¹H (300 MHz, CDCl₃, δ en ppm) : 2,97 (mt : 2H); 3,39 (mt : 2H); 3,71 (s : 3H); 4,24 (s large : 1H); 4,45 (mf : 1H); 4,57 (s : 2H); de 6,65 à 6,80 (mt: 3H); de 7,15 à 7,30 (mt : 8H)].

### Exemple 37

Le (RS)-N-{1-[(4-chloro-phényl)-pyrimidin-5-yl-méthyl]-azétidin-3-yl}-N-(3,5-difluoro-phényl)-méthylsulfonamide peut être obtenu en opérant comme à l'exemple 33, à partir de 0,16 g de bromhydrate de (RS)-5-[bromo-(4-chlorophényl)-méthyl]-pyrimidine, de 0,131 g de chlorhydrate de N-azétidin-3-yl-N-(3,5-difluorophényl)-méthanesulfonamide dans 5 cm³ d'acétonitrile, de 303 mg de carbonate de potassium et de 95 mg de iodure de potassium, on obtient ainsi 26 mg de (RS)-N-{1-[(4-chloro-phényl)-pyrimidin-5-yl-méthyl]-azétidin-3-yl}-N-(3,5-difluoro-phényl)-méthylsulfonamide sous forme d'une meringue jaune [Spectre de R.M.N. ¹H (400 MHz, CDCl₃, δ en ppm) : 2,83 (s : 3H); 2,91 (mt : 2H); 3,57 (mt : 2H); 4,31 (s : 1H); 4,50 (mt : 1H); de 6,75 à 6,90 (mt : 3H); 7,29 (s : 4H); 8,71 (s : 2H); 9,08 (s : 1H)].

Les médicaments selon l'invention sont constitués par au moins un composé de formule (I) ou un isomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés.+ Dans ces compositions, le principe actif selon invention est mélangé à un ou plusieurs diluants inertes, tels que amidon, cellulose, saccharose, lactose ou silice , sous courant d'argon. Ces compositions peuvent également comprendre des substances autres que les diluants, par exemple un ou plusieurs lubrfiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, des troubles du transit intestinal, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques, .

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Composé de formule (I)..... | 50 mg |
| - Cellulose..... | 18 mg |
| - Lactose..... | 55 mg |
| - Silice colloïdale..... | 1 mg |
| - Carboxyméthylamidon sodique..... | 10 mg |
| - Talc..... | 10 mg |
| - Stéarate de magnésium..... | 1 mg |

### EXEMPLE B

On prépare selon la technique habituelle des comprimés dosés à 50 mg de produit actif ayant la composition suivante :

| | |
|---|---|
| - Composé de formule (I)..... | 50 mg |
| - Lactose..... | 104 mg |
| - Cellulose..... | 40 mg |
| - Polyvidone..... | 10 mg |
| - Carboxyméthylamidon sodique..... | 22 mg |
| - Talc..... | 10 mg |
| - Stéarate de magnésium..... | 2 mg |
| - Silice colloïdale..... | 2 mg |
| - Mélange d'hydroxyméthylcellulose, glycérine, oxyde de | |
| titane (72-3,5-24,5) q.s.p. 1 comprimé pelliculé terminé à 245 mg | |

### EXEMPLE C

On prépare une solution injectable contenant 10 mg de produit actif ayant la composition suivante :

| | |
|---|---|
| - Composé de formule (I)..... | 10 mg |
| - Acide benzoïque..... | 80 mg |
| - Alcool benzylique..... | 0,06 ml |
| - Benzoate de sodium..... | 80 mg |
| - Ethanol à 95 %..... | 0,4 ml |
| - Hydroxyde de sodium..... | 24 mg |
| - Propylène glycol..... | 1,6 ml |
| - Eau.....q.s.p. | 4 ml |

## Revendications

1. Composition pharmaceutique contenant en tant que principe actif au moins un composé de formule : 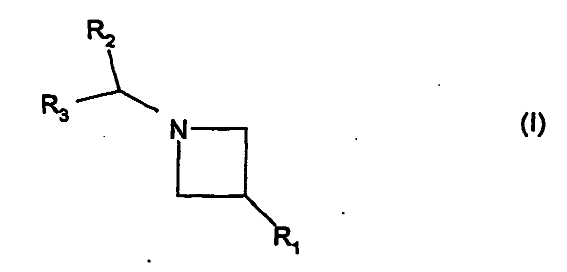 dans laquelle
R₁ représente un radical -N(R₄)R₅, -N(R₄)-CO-R₅, -N(R₄)-SO₂R₆,
R₂ et R₃, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, COOalk, -CONR₇R₈, -CO-NH-NR₉R₁₀, alkylsulfanyte, alkylsulfinyle, alkylsulfonyle, alkylsulfanylalkyle, alkylsulfinylalkyle, alkylsulfonylalkyle, hydroxyalkyle ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle,
isochromannyle, isoquinolyle, pyrrolyle, pyridyle, pyrimidyle, quinolyle, 1,2,3,4-tétrahydroisoquinotyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, Cooalk, -CO-NH-NR₉R₁₀, -CONR₇R₈, -alk-NR₉R₁₀, alkylsutfanyle, alkylsulfinyle, alkylsulfonyle, alkylsulfanylalkyle, alkylsulfinylalkyle, alkylsulfonylalkyle ou hydroxyalkyle ,
R₄ représente un radical-C(R₁₁)(R₁₂)-Het, -Het, -C(R₁₁)(R₁₂)-Ar, Ar, cycloalkyle ou norbornyle,
R₅ représente un atome d'hydrogène ou un radical hydroxyalkyle, -alk-COOalk, -alk-CONR₇R₈, -alk-NR₇R₈, alcoxy, Ar, Het, -CH₂Ar, -CH₂Het ou alkyle éventuellement substitué par un ou plusieurs halogène,
R₆ représente un radical hydroxyalkyle, -alk-COOalk, -alk-CONR₇R₈, -alk-NR₇R₈, alcoxy, Ar, Het, -CH₂Ar, -CH₂Het ou alkyle éventuellement substitué par 1 ou plusieurs halogène,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents, représentent un atome d'hydrogène ou un radical alkyle, -COOalk, cycloalkyle, alkylcycloalkyle, -alk-O-alk, hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₁₁ représente un atome d'hydrogène ou un radical hydroxyalkyle, -alk-COOalk, -alk-CONR₇R₈, -alk-NR₇R₈, alcoxyalkyle, Ar, Het, -CH₂Ar, -CH₂Het ou alkyle éventuellement substitué par un ou plusieurs halogène,
R₁₂ représente un atome d'hydrogène ou un radical hydroxyalkyle, -alk-COOalk, -alk-CONR₇R₈, -alk-NR₇R₈, alcoxyalkyle ou alkyle éventuellement substitué par un ou plusieurs halogène,
ou bien R₁₁ et R₁₂ forment ensemble avec l'atome de carbone auquel ils sont rattachés un cycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle, naphtyle ou indènyle, ces différents radicaux étant éventuellement substitués par un ou plusieurs halogène, alkyle, alcoxy, -CO-alk, cyano, -COOH, -COOalk, -CONR₁₃R₁₄, -CO-NH-NR₁₅R₁₆, alkylsulfanyle, alkylsulfinyle, alkylsulfonyl, -alk-NR₁₅R₁₆, -NR₁₅R₁₆, alkylthioalkyle, formyle, CF₃, OCF₃, Het, -O-alk-NH-cycloalkyle, SO₂NH₂, hydroxy, hydroxyalkyle, -NHCOalk, NHCOOalk ou sur 2 atomes de carbone adjacents par dioxyméthylène,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, halogène, alcoxycarbonyle, oxo, hydroxy, les hétérocycles azotés étant éventuellement sous leur forme N-oxydée,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
alk représente un radical alkyle ou alkylène,
les radicaux et portions alkyle et alkylène et les radicaux et portions alcoxy sont en chaîne droite ou ramifiée et contiennent 1 à 6 atomes de carbone et les radicaux cycloalkyle contiennent 3 à 10 atomes de carbone,
les isomères optiques de ces composés et leurs sels avec un acide minéral ou organique pharmaceutiquement acceptables.

2. Composition pharmaceutique selon la revendication 1 pour laquelle dans la formule (I) Het est choisi parmi benzimidazole, benzoxazole, benzothiazole, benzothiophène, cinnoline, thiophène, quinazoline, quinoxaline, quinoline, pyrazole, pyrrole, pyridine, imidazole, indole, isoquinoline, pyrimidine, thiazole, thiadiazole, pipéridine, pipérazine, triazole, furane, tétrahydroisoquinoline, tétrahydroquinoline, ces hétérocycles étant éventuellement substitués par un ou plusieurs alkyle, alcoxy, halogène, alcoxycarbonyle, oxo, hydroxy, OCF₃ ou CF₃.

3. Composition pharmaceutique selon la revendication 1 pour laquelle dans le composé de formule (I)
R₁ représente un radical -N(R₄)R₅, -N(R₄)-SO₂R₆,
R₂ représente soit un phényle non substitué ou substitué par un ou plusieurs halogène, alkyle, alcoxy, trifluorométhyle, trifluorométhoxy, -CO-alk, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, bifluorométhyle, trifluorométhoxy, -CONR₇R₈, -alk-NR₉R₁₀, alkylsulfanyle, alkylsulfinyle, alkylsulfonyle ou hydroxyalkyle ,
R₃ représente soit un phényle non substitué ou substitué par un ou plusieurs halogène, alkyle, alcoxy, trifluorométhyle, trifluorométhoxy, -CO-alk, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle ,
R₄ représente un radical -C(R₁₁)(R₁₂)-Het, -Het, -C(R₁₁)(R₁₂)-Ar, Ar ou norbomyle,
R₅ représente un atome d'hydrogène ou un radical hydroxyalkyle, -alk-COOalk, -alk-CONR₇R₈, -alk-NR₇R₈, alcoxy, -CH₂Ar, -CH₂Het ou alkyle,
R₆ représente un radical hydroxyalkyle, -alk-COOalk, -alk-CONR₇R₈, -alk-NR₇R₈, alcoxy, -CH₂Ar, -CH₂Het ou alkyle,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀ identiques ou différents représentent un atome d'hydrogène ou un radical alkyle, cycloalkyle, alkylcycloalkyle, -alk-O-alk ou hydroxyalkyle ou bien R₉ et R₁₀ forment ensemble avec l'atome d'azote auquel ils sont
rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, oxo, hydroxyalkyle ou -CO-NH₂,
R₁₁ représente un atome d'hydrogène ou un radical hydroxyalkyle, -alk-COOalk, -alk-CONR₇R₈, -alk-NR₇R₈, alcoxyalkyle, Ar, Het, -CH₂Ar, -CH₂Het ou alkyle éventuellement substitué par un ou plusieurs halogène,
R₁₂ représente un atome d'hydrogène ou un radical hydroxyalkyle, -alk-COOalk, -alk-CONR₇R₈, -alk-NR₇R₈, alcoxyalkyle ou alkyle éventuellement substitué par un ou plusieurs halogène,
ou bien R₁₁ et R₁₂ forment ensemble avec l'atome de carbone auquel ils sont rattachés un cycles mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle ou naphtyle, ces différents radicaux étant éventuellement substitués par un ou plusieurs halogène, alkyle, alcoxy, -CO-alk, cyano, -CONR₁₃R₁₄, alkylsulfonyle, -alk-NR₁₅R₁₆, -NR₁₅R₁₆, CF₃, OCF₃, SO₂NH₂, hydroxy, hydroxyalkyle ou sur 2 atomes de carbone adjacents par dioxyméthylène,
Het représente un hétérocycle choisi parmi benzimidazole, benzoxazole, benzothiazole, benzothiophène, thiophène, quinazoline, quinoxaline, quinoline, pyrrole, pyridine, imidazole, indole, isoquinoline, pyrimidine, thiazole, thiadiazole, furane, tétrahydroisoquinoline et tétrahydroquinoline, ces hétérocycles étant éventuellement substitués par un ou plusieurs alkyle, alcoxy, halogène, alcoxycarbonyle, oxo, hydroxy, OCF₃ ou CF₃,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₈, identiques ou différents, représentent un atome d'hydrogène ou un radical alkyle ou bien R₁₅ et R₁₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
les isomères optiques de ces composés et leurs sels avec un acide minéral ou organique pharmaceutiquement acceptables.

4. Composition pharmaceutique selon la revendication 1 pour laquelle dans le composé de formule (I)
R₁ représente un radical -N(R₄)-SO₂R₆,
R₂ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈ ou hydroxyalkyle ,
R₃ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈ ou hydroxyalkyle,
R₄ représente -Het ou Ar,
R₆ représente un radical hydroxyalkyle ou alkyle,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle ou naphtyle, ces différents radicaux étant éventuellement substitués par un ou plusieurs halogène, alkyle, alcoxy, -CO-alk, cyano, -CONR₁₃R₁₄, -alk-NR₁₅R₁₆, -NR₁₅R₁₆, CF₃, OCF₃, SO₂NH₂, hydroxy ou hydroxyalkyle,
Het représente représente un hétérocycle choisi parmi benzimidazole, benzoxazole, benzothiazole, benzothiophène, thiophène, quinazoline, quinoxaline, quinoline, pyrrole, pyridine, imidazole, indole, isoquinoline, thiazole, thiadiazole, furane, tétrahydroisoquinoline et tétrahydroquinoline, ces hétérocycles étant éventuellement substitués par un ou plusieurs alkyle, alcoxy, halogène, alcoxycarbonyle, oxo, hydroxy, OCF₃ ou CF₃,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
les isomères optiques de ces composés et leurs sels avec un acide minéral ou organique pharmaceutiquement acceptables.

5. Composition selon la revendication 1 pour laquelle le composé de formule (I) est choisi parmi les composés suivants :
N-{1-[bis-(4-chlorophényl)méthyl]-azétidin-3-yl}-N-(6-chloropyrid-2-yl)-méthylsulfonamide,
N-{1-[bis-(4-chlorophényl)méthyl]azétidin-3-yt}-N-(6-éthylpyrid-2-yl)-méthyl-sulfonamide,
N-{1-[bis-(4-chlorophényl)méthyl]azétidin-3-yl}-N-quinol-6-yl-méthyl-sulfonamide,
N-{1-[bis-(4-chlorophényl)méthyl]azétidin-3-yl}-N-quinol-5-yl-méthylsulfonamide,
N{1-[bis-(4-chlorophényl)méthyl]azétidin-3-y}-N-isoquinol-5-yl-méthylsulfonamide,
N-{1-[bis-(4-chlorophényl)méthyl]azétidin-3-yl}-N-pyrid-3-yl-méthylsulfonamide,
N-{1-[bis-(4-chlorophényl)méthyl]azétidin-3-yl}-N-(1-oxyde-pyrid-3-yl)-méthylsulfonamide,
N-(1R,2S,4S)-bicyclo[2,2,1]hept-2-yl-N-{1-[bis-(4-chlorophényl)méthyl] azétidin-3-yl}-méthylsulfonamide,
N-(1R,2R,4S)-bicyclo[2,2,1]hept-2-yl-N-{1-[bis-(4-chlorophényl)méthyl] azétidin-3-yl}-méthylsulfonamide,
N-{1-[bis-(4-chlorophényl)méthyl]azétidin-3-yl}-N-(3,5-difluorophényl)-méthylsulfonamide,
N-{1-[bis-(4-chlorophényl)méthyl]azétidin-3-yl}-N-(thiazol-2-yl)-méthyl sulfonamide,
N-{1-[bis-(4-chlorophényl)-méthyl]-azétidin-3-yl}-N-(3-méthoxyphényl)-méthylsulfonamide,
N-{1-[bis-(4-chlorophényl)-méthyl]-azétidin-3-yl}-N-(3-hydroxyphényl)-méthylsulfonamide,
N-{1-[bis-(4-chlorophényl)-méthyl]-azétidin-3-yl}-N-(3-hydroxyméthyl-phényl)-méthylsulfonamide,
N-{1-[bis-(4-chlorophényl)-méthyl]-azétidin-3-yl}-N-(méthylsulfonyl)-3-aminobenzoate d'éthyle,
N-{1-[bis-(4-chlorophényl)méthyl]azétidin-3-yl}-N-(1-isobutyl-pipérid-4-yl)-méthylsulfonamide,
N-benryl-N-{1-[bis(4-chlorophényl)méthyl]azétidin-3-yl}amine,
N-{1-[bis(4-chlorophényl)méthyl]azétidin-3-yl}-N-(3,5-difluorobenzyl)amine,
N-{1-[bis(4-chlorophényl)méthyl]azétidin-3-yl}-N-(3,5-difluorobenzyl)méthylsulfonamide,
N-{1-[bis(4-chlorophényl)méthyl]azétidin-3-yl}-N-(pyrid-3-yl-méthyl)-méthylsulfonamide,
N-{1-[bis-(4-fluoro-phényl)-méthyl]-azétidin-3-yl}-N-(3,5-difluorophényl)-méthylsulfonamide,
(RS)-N-{1-[(4-chlorophényl)-pyrid-3-yl-méthyl]-azétidin-3-yl}-N-(3.5-difluorophényl)-méthylsulfonamide,
(R)-N-{1-[(4-chlorophényl)-pyrid-3-y1-méthyl]-azétidin-3-yl}-N-(3,5-difluorophényl)-méthylsulfonamide,
(S)-N-{1-[(4-chlorophényl)-pyrid-3-yl-méthyl]-azétidin-3-yl}-N-(3,5-difluorophényl)-méthylsulfonamide,
(RS)-N-{1-[(4-chlorophényl)-pyrid-4-yl-méthyl]-azétidin-3-yl}-N-(3,5-difluorophényl)-méthylsuifonamide,
(R)-N-{1-[(4-chlorophényl)-pyrid-4-yl-méthyl]-azétidin-3-yl}-N-(3,5-difluorophényl)-méthylsulfonamide,
(S)-N-{1-[(4-chlorophényl)-pyrid-4-yl-méthyl]-azétidin-3-yl}-N-(3,5-difluorophényl)-méthylsulfonamide,
(RS)-N-{1-[(4-chlorophényl)-pyrimidin-5-yl-méthyl]-azétidin-3-yl}-N-(3,5-difluorophényl)-méthylsulfonamide,
(R)-N-{1-[(4-chlorophényl)-pyrimidin-5-yl-méthyl]-azétidin-3-yl}-N-(3,5-difluorophényl)-méthylsulfonamide,
(S)-N-{1-[(4-chlorophényl)-pyrimidin-5-yl-méthyl]-azétidin-3-yl}-N-(3,5-difluorophényl)-méthylsulfonamide,
N-{1-[bis-(4-chlorophényl)méthyl]azétidin-3-yl}-N-(3,5-difluorophényl)-benzylsulfonamide,
leurs isomères optiques et leurs sels avec un acide minéral ou organique pharmaceutiquement acceptables.

6. Composé de formule : 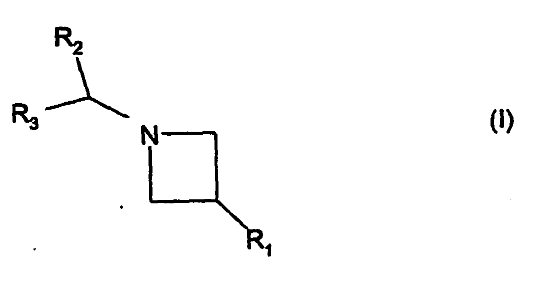 dans laquelle
R₁ représente un radical -N(R₄)R₅, -N(R₄)-CO-R₅. -N(R₄)-SO₂R₆,
R₂ et R₃, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, COOalk, -CONR₇R₈, -CO-NH-NR₉R₁₀, alkylsulfanyle, alkylsulfinyle, alkylsulfonyle, alkylsulfanylalkyle, alkylsulfinylalkyle, alkylsulfonylalkyle, hydroxyalkyle ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle, isochromannyle, isoquinolyle, pyrrolyle, pyridyle, pyrimidyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, COOalk, -CO-NH-NR₉R₁₀, -CONR₇R₈, -alk-NR₉R₁₀, alkylsulfanyle, alkylsulfinyle, alkylsulfonyle, alkylsulfanylalkyle, alkylsulfinylalkyle, alkylsulfonylalkyle ou hydroxyalkyle,
R₄ représente un radical -C(R₁₁)(R₁₂)-Het, -Het, -C(R₁₁)(R₁₂)-Ar, Ar, cycloalkyle ou norbomyle,
R₅ représente un atome d'hydrogène ou un radical hydroxyalkyle, -alk-COOalk, -alk-CONR₇R₈, -alk-NR₇R₈, alcoxy, Ar, Het, -CH₂Ar, -CH₂Het ou alkyle éventuellement substitué par un ou plusieurs halogène,
R₆ représente un radical hydroxyalkyle, -alk-COOalk, -alk-CONR₇R₆, -alk-NR₇R₈, alcoxy, Ar, Het, -CH₂Ar, -CH₂Het ou alkyle éventuellement substitué par 1 ou plusieurs halogène,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents, représentent un atome d'hydrogène ou un radical alkyle, -COOalk, cycloalkyle, alkylcycloalkyle, -alk-O-alk, hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₁₁ représente un atome d'hydrogène ou un radical hydroxyalkyle, -alk-COOalk, -alk-CONR₇R₈, -alk-NR₇R₈, alcoxyalkyle, Ar, Het, -CH₂Ar, -CH₂Het ou alkyle éventuellement substitué par un ou plusieurs halogène,
R₁₂ représente un atome d'hydrogène ou un radical hydroxyalkyle, -alk-COOalk, -alk-CONR₇R₈, -alk-NR₇R₈, alcoxyalkyle ou alkyle éventuellement substitué par un ou plusieurs halogène,
ou bien R₁₁ et R₁₂ forment ensemble avec l'atome de carbone auquel ils sont rattachés un cycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle, naphtyle ou indènyle, ces différents radicaux étant éventuellement substitués par un ou plusieurs halogène, alkyle, alcoxy, -CO-alk, cyano, -COOH, -COOalk, -CONR₁₃R₁₄, -CO-NH-NR₁₅R₁₆, alkylsulfanyle, alkylsulfinyle, alkylsulfonyle, -alk-NR₁₅R₁₆, -NR₁₅R₁₆, alkylthioalkyle, formyle, CF₃, OCF₃, Het, -O-alk-NH-cycloalkyle, SO₂NH₂, hydroxy, hydroxyalkyle, -NHCOalk, NHCOOalk ou sur 2 atomes de carbone adjacents par dioxyméthylène,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, halogène, alcoxycarbonyle, oxo, hydroxy, les hétérocycles azotés étant éventuellement sous leur forme N-oxydée,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
alk représente un radical alkyle ou alkylène,
les radicaux et portions alkyle et alkylène et les radicaux et portions alcoxy sont en chaîne droite ou ramifiée et contiennent 1 à 6 atomes de carbone et les radicaux cycloalkyle contiennent 3 à 10 atomes de carbone,
les isomères optiques de ces composés et leurs sels avec un acide minéral ou organique pharmaceutiquement acceptables
à l'exception du composé pour lequel R₂ et R₃ représentent des radicaux phényle, R₁ représente un radical -N(R₄)SO₂R₆ pour lequel R₄ représente un radical phényle et R₆ représente un radical méthyle.

7. Composé de formule (I) selon la revendication 6 pour lequel Het est choisi parmi benzimidazole, benzoxazole, benzothiazole, benzothiophène, cinnoline, thiophène, quinazoline, quinoxaline, quinoline, pyrazole, pyrrole, pyridine, imidazole, indole, isoquinoline, pyrimidine, thiazole, thiadiazole, pipéridine, pipérazine, triazole, furane, tétrahydroisoquinoline, tétrahydroquinoline, ces hétérocycles étant éventuellement substitués par un ou plusieurs alkyle, alcoxy, halogène, alcoxycarbonyle, oxo, hydroxy, OCF₃ ou CF₃.

8. Composé de formule (I) selon la revendication 6 pour lequel R₁ représente un radical -N(R₄)R₅, -N(R₄)-SO₂R₈,
R₂ représente soit un phényle non substitué ou substitué par un ou plusieurs halogène, alkyle, alcoxy, trifluorométhyle, trifluorométhoxy, -CO-alk, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle,
R₃ représente soit un phényle non substitué ou substitué par un ou plusieurs halogène, alkyle, alcoxy, trifluorométhyle, trifluorométhoxy, -CO-alk, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle ,
R₄ représente un radical -C(R₁₁)(R₁₂)-Het, -Het, -C(R₁₁)(R₁₂)-Ar, Ar ou norbornyle,
R₅ représente un atome d'hydrogène ou un radical hydroxyalkyle, -alk-COOalk, -alk-CONR₇R₈, -alk-NR₇R₈, alcoxy, -CH₂Ar, -CH₂Het ou alkyle,
R₆ représente un radical hydroxyalkyle, -alk-COOalk, -alk-CONR₇R₈, -alk-NR₇R₈, alcoxy, -CH₂Ar, -CH₂Het ou alkyle,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, cycloalkyle, alkylcycloalkyle, -alk-O-alk ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, oxo, hydroxyalkyle ou -CO-NH₂,
R₁₁ représente un atome d'hydrogène ou un radical hydroxyalkyle, -alk-COOalk, -alk-CONR₇R₈, -alk-NR₇R₈, alcoxyalkyle, Ar, Het, -CH₂Ar, -CH₂Het ou alkyle éventuellement substitué par un ou plusieurs halogène,
R₁₂ représente un atome d'hydrogène ou un radical hydroxyalkyle, -alk-COOalk, -alk-CONR₇R₈, -alk-NR₇R₈, alcoxyalkyle ou alkyle éventuellement substitué par un ou plusieurs halogène,
ou bien R₁₁ et R₁₂ forment ensemble avec l'atome de carbone auquel ils sont rattachés un cycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar représente un radical phényle ou naphtyle, ces différents radicaux étant éventuellement substitués par un ou plusieurs halogène, alkyle, alcoxy, -CO-alk, cyano, -CONR₁₃R₁₄, alkylsulfonyle, -alk-NR₁₅R₁₆, -NR₁₅R₁₆, CF₃, OCF₃, SO₂NH₂, hydroxy, hydroxyalkyle ou sur 2 atomes de carbone adjacents par dioxyméthylène,
Het représente un hétérocycle choisi parmi benzimidazole, benzoxazole, benzothiazole, benzothiophène, thiophène, quinazoline, quinoxaline, quinoline, pyrrole, pyridine, imidazole, indole, isoquinoline, pyrimidine, thiazole, thiadiazole, furane, tétrahydroisoquinoline et tétrahydroquinoline, ces hétérocycles étant éventuellement substitués par un ou plusieurs alkyle, alcoxy, halogène, alcoxycarbonyle, oxo, hydroxy, OCF₃ ou CF₃,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
les isomères optiques de ces composés et leurs sels avec un acide minéral ou organique pharmaceutiquement acceptables,
à l'exception du composé pour lequel R₂ et R₃ représentent des radicaux phényle, R₁ représente un radical -N(R₄)SO₂R₆ pour lequel R₄ représente un radical phényle et R₆ représente un radical méthyle.

9. Composé de formule (I) selon la revendication 6 dans laquelle
R₁ représente un radical -N(R₄)-SO₂R₅,
R₂ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈ ou hydroxyalkyle ,
R₃ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈ ou hydroxyalkyle ,
R₄ représente -Het ou Ar,
R₆ représente un radical hydroxyalkyle ou alkyle,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi
oxygène, soufre et azote et étant éventuellement substitué par un ou plusieurs alkyle,
Ar représente un radical phényle ou naphtyle, ces différents radicaux étant éventuellement substitués par un ou plusieurs halogène, alkyle, alcoxy, -CO-alk, cyano, -CONR₁₃R₁₄, -alk-NR₁₅R₁₆, -NR₁₅R₁₆, CF₃, OCF₃, SO₂NH₂, hydroxy ou hydroxyalkyle,
Het représente représente un hétérocycle choisi parmi benzimidazole, benzoxazole, benzothiazole, benzothiophène, thiophène, quinazoline,
quinoxaline, quinoline, pyrrole, pyridine, imidazole, indole, isoquinoline, thiazole, thiadiazole, furane, tétrahydroisoquinoline et tétrahydroquinoline, ces hétérocycles étant éventuellement substitués par un ou plusieurs alkyle, alcoxy, halogène, alcoxycarbonyle, oxo, hydroxy, OCF₃ ou CF₃,
R₁₃ et R₁₄, identiques ou différents, représentent un atome d'hydrogène ou un radical alkyle ou bien R₁₃ et R₁₄ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₁₅ et R₁₆, identiques ou différents, représentent un atome d'hydrogène ou un radical alkyle ou bien R₁₅ et R₁₆ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
les isomères optiques de ces composés et leurs sels avec un acide minéral ou organique pharmaceutiquement acceptables,
à l'exception du composé pour lequel R₂ et R₃ représentent des radicaux phényle, R₁ représente un radical -N(R₄)SO₂R₆ pour lequel R₄ représente un radical phényle et R₆ représente un radical méthyle.

10. Composé de formule (I) selon la revendication 6 choisi parmi les composés suivants :
N-{1-[bis-(4-chlorophényl)méthyl]-azétidin-3-yl}-N-(6-chioropyrid-2-yl)-méthylsulfonamide,
N-{1-[bis-(4-chlorophényl)méthyl]azétidin-3-yl}-N-(6-éthylpyrid-2-yl)-méthyl-sulfonamide,
N-{1-[bis-(4-chlorophényl)méthyl]azétidin-3-yl}-N-quinol-6-yl-méthylsulfonamide,
N-{1-[bis-(4-chlorophényl)méthyl]azétidin-3-yl}-N-quinol-5-yl-méthylsulfonamide,
N-{1-[bis-(4-chlorophényl)méthyl]azétidin-3-yl}-N-isoquinol-5-yl-méthylsulfonamide,
N-{1-[bis-(4-chlorophényl)méthyl]azétidin-3-yl}-N-pyrid-3-yl-méthylsulfonamide,
N-{1-[bis-(4-chlorophényl)méthyl]azétidin-3-yl}-N-(1-oxyde-pyrid-3-yl)-méthylsulfonamide,
N-(1R,2S,4S)-bicyclo[2,2,1]hept-2-yl-N-{1-[bis-(4-chlorophényl)méthyl] azétidin-3-yl}-méthylsulfonamide,
N-(1R,2R,4S)-bicyclo[2,2,1]hept-2-yl-N-{1-[bis-(4-chlorophényl)méthyl] azétidin-3-yl}-méthylsuifonamide,
N-{1-[bis-(4-chlorophényl)méthyl]azétidin-3-yl}-N-(3,5-difluorophényl)-méthylsulfonamide,
N-{1-[bis-(4-chlorophényl)méthyl]azétidin-3-yl}-N-(thiazol-2-yl)-méthyl sulfonamide,
N-{1-[bis-(4-chlorophényl)-méthyl]-azétidin-3-yl}-N-(3-méthoxyphényl)-méthylsulfonamide,
N-{1-[bis-(4-chlorophényl)-méthyl]-azétidin-3-yl}-N-(3-hydroxyphényl)-méthylsulfonamide,
N-{1-[bis-(4-chlorophényl)-méthyl]azétidin-3-yl}-N-3-hydroxyméthyl-phényl)-méthylsulfonamide,
N-{1-[bis-(4-chlorophényl)-méthyl]-azétidin-3-yl}-N-(méthylsulfonyl)-3-aminobenzoate d'éthyle,
N-{1-[bis-(4-chlorophényl)méthyl]azétidin-3-yl}-N-(1-isobutyl-pipérid-4-yl)-méthylsulfonamide,
N-benzyl-N-{1-[bis(4-chiorophényl)méthyl]azétidin-3-yl}amine, N-{1-[bis(4-chlorophényl)méthyl]azétidin-3-yl}-N-(3,5-difluorobenzyl)amine,
N-{1-[bis(4-chlorophényl)méthyl]azétidin-3-yl}-N-(3,5-difluorobenzyl)méthylsulfonamide,
N-{1-[bis(4-chlorophényl)méthyl]azétidin-3-yl}-N-(pyrid-3-yl-méthyl)-méthylsulfonamide,
N-{1-[bis-(4-fluoro-phényl)-méthyl]-azétidin-3-yl}-N-(3,5-difluorophényl)-méthylsulfonamide,
(RS)-N-{1-[(4-chlorophényl)-pyrid-3-yl-méthyl]-azétidin-3-yl}-N-(3,5-difluorophényl)-méthylsulfonamide,
(R)-N-{1-[(4-chlorophényl)-pyrid-3-yl-méthyl]-azétidin-3-yl}-N-(3,5-difluorophényl)-méthylsuifonamide,
(S)-N-{1-[(4-chlorophénylrpyrid-3-yl-méthyl]-azétidin-3-yl}-N-(3,5-difluorophényl)-méthylsulfonamide,
(RS)-N-{1-[(4-chrorophényl)-pyrid-4-yl-méthyl]-azétidin-3-yl}-N-(3,5-difluorophényl)-méthylsulfonamide,
(R)-N-{1-[(4-chlorophényl)-pyrid-4-yl-méthyl]-azétidin-3-yl}-N-(3,5-difluorophényl)-méthylsulfonamide.
(S)-N-{1-[(4-chlorophényl)-pyrid-4-yl-méthyl]-azétidin-3-yl}-N-(3,5-difluorophényl)-méthylsuifonamide,
(RS)-N-{1-[(4-chlorophényl)-pyrimidin-5-yl-méthyl]-azétidin-3-yl}-N-(3,5-difluorophényl)-méthylsulfonamide,
(R)-N-{1-[(4-chlorophényl)-pyrimidin-5-yl-méthyl]-azétidin-3-yl}-N-(3,5-difluorophényl)-méthylsulfonamide,
(S)-N-{1-[(4-chlorophényl)-pyrimidin-5-yl-méthyl]-azétidin-3-yl}-N-(3,5-difluorophényl)-méthylsulfonamide,
N-{1-[bis-(4-chlorophényl)méthyl]azétidin-3-yl}-N-(3,5-difluorophényl)-benzylsulfonamide,
leurs isomères optiques et leurs sels avec un acide minéral ou organique pharmaceutiquement acceptables.

11. Le N-{1-[bis-(4-chlorophényl)méthyl]azétidin-3-yl}-N-(3,5-difluorophényl)-méthylsulfonamide, ses isomères optiques et ses sels avec un acide minéral ou organique pharmaceutiquement acceptables.

12. Procédé de préparation des composés de formule (I) selon la revendication 6 pour lesquels R₁ représente un radical -N(R₄)R₅ dans lequel R₅ est un atome d'hydrogène, R₄ est un radical -CR₁₁R₁₂-Ar ou -CR₁₁R₁₂-Het et R₁₂ est un atome d'hydrogène **caractérisé en ce que** l'on fait réagir un dérivé Rb-COR₁₁ pour lequel R₁₁ a les mêmes significations que dans la revendication 6 avec un dérivé de formule : 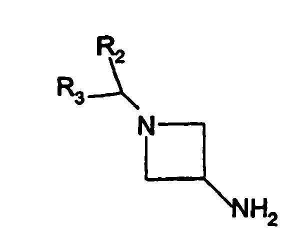 Rb représente radical Ar ou Het, R₂, R₃, R₁₁ Ar et Het ont les mêmes significations que dans la revendication 6, isole le produit et le transforme éventuellement en sel pharmaceutiquement acceptable.

13. Procédé de préparation des composés de formule (I) selon la revendication 6 pour lesquels R₁ représente un radical -N(F₄)-CO-R₅ dans lequel R₄ est un radical -C(R₁₁)(R₁₂)-Het ou -C(R₁₁)(R₁₂)-Ar et R₁₂ est un atome d'hydrogène **caractérisé en ce que** l'on fait réagir un dérivé Hal-COR₅ avec un dérivé de formule : 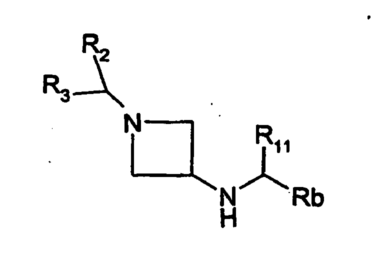 Hal représente un atome d'halogène Rb représente un radical Ar ou Het et R₂, R₃, R₅, R₁₁, Ar et Het ont les mêmes significations que dans la revendication 6, isole le produit et le transforme éventuellement en sel pharmaceutiquement acceptable.

14. Procédé de préparation des composés de formule (I) selon la revendication 6 pour lesquels R₁ représente un radical -N(R₄)-SO₂R₆ dans lequel R₄ est un radical -C(R₁₁)(R₁₂)-Ar ou -C(R₁₁)(R₁₂)-Het et R₁₂ est un atome d'hydrogène **caractérisé en ce que** l'on fait réagir un dérivé Hal-SO₂R₆, avec un dérivé de formule : 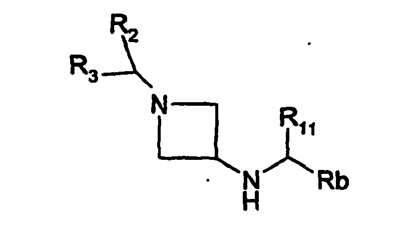 R₂, R₃,R₁₁, R₅ ont les mêmes significations que dans la revendication 6, Hal représente un atome d'halogène et Rb représente un radical Ar et Het, isole le produit et le transforme éventuellement en sel pharmaceutiquement acceptable.

15. Procédé de préparation des composés de formule (I) selon la revendication 6 pour lesquels R₁ représente un radical -N(R₄)R₅ **caractérisé en ce que** l'on fait réagir un dérivé R₅(R₄)NH avec un dérivé de formule : 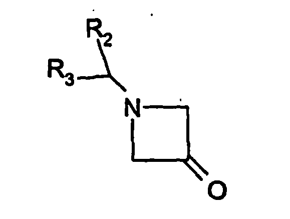 R₂, R₃, R₄, R₅ ont les mêmes significations que dans la revendication 6, isole le produit et le transforme éventuellement en sel pharmaceutiquement acceptable.

16. Procédé de préparation des composés de formule (I) selon la revendication 6 pour lesquels R₁ représente un radical -N(R₄)SO₂R₆ **caractérisé en ce que** l'on fait réagir un dérivé Hal-SO₂R₆ sur un dérivé de formule : 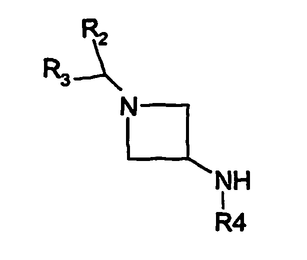 R₂,R₃,R₄ et R₆ ont les mêmes significations que dans la revendication 6 et Hal représente un atome d'halogène, isole le produit et le transforme éventuellement en sel pharmaceutiquement acceptable.

17. Procédé de préparation des composés de formule (I) selon la revendication 6 pour lesquels R₁ représente un radical -N(R₄)COP₅ **caractérisé en ce que** l'on fait réagir un dérivé Hal-COR₆ avec un dérivé de formule : . 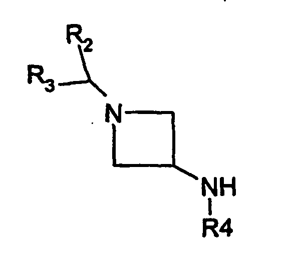 R₂, R₃, R₄ et R₅ ont les mêmes significations que dans la revendication 6 et Hal représente un atome d'halogène, isole le produit et le transforme éventuellement en sel pharmaceutiquement acceptable.

18. Procédé de préparation des composés de formule (I) selon la revendication 6 pour lesquels R₁ représente un radical -N(R₄)-SO₂-R₆, R₄ est un radical Het ou Ar **caractérisé en ce que** l'on fait réagir un dérivé Rd-NH-SO₂-R₆ avec un dérivé de formule : 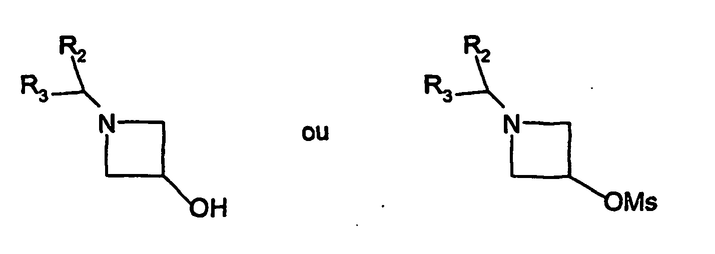 Rd représente un radical Ar ou Het, R₂, R₃ et R₆ ont les mêmes significations que dans la revendication 6 et Ms représente un radical méthylsulfonyloxy, isole le produit et le transforme éventuellement en sel pharmaceutiquement acceptable.

19. Procédé de préparation des composés de formule (I) selon la revendication 6 **caractérisé en ce que** l'on fait réagir un dérivé R₂-CHBr-R₃ avec un dérivé de formule : 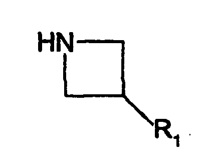 R₁, R₂ et R₃ ont les mêmes significations que dans la revendication 6, isole le produit et le transforme éventuellement en sel pharmaceutiquement acceptable.

20. Procédé de préparation des composés de formule (I) selon la revendication 6 pour lesquels R₁ représente un radical -N(R₄)-SO₂-R₆ pour lequel R₄ est un radical pipérid-4-yle substitué sur l'azote par un radical alkyle **caractérisé en ce que** l'on alkyle un composé de formule (I) correspondant pour lequel R₁ représente un radical -N(R₄)-SO₂-R₆ pour lequel R₄ est un radical pipérid-4-yle, isole le produit et le transforme éventuellement en sel pharmaceutiquement acceptable.

21. Procédé de préparation des composés de formule (I) selon la revendication 6 pour lesquels R₁ représente un radical -N(R₄)-SO₂-R₆ pour
lequel R₄ est un radical phényle substitué par un radical pyrrolid-1-yle varactérisé en ce que l'on fait réagir la pyrrolidine sur un composé de formule (I) correspondant pour lequel R₁ représente un radical -N(R₄)SO₂R₆ pour lequel R₄ est un radical phényle substitué par un atome d'halogène, isole le produit et le transforme éventuellement en sel pharmaceutiquement acceptable.

22. Médicament contenant en tant que principe actif au moins un composé de formule (I) selon l'une des revendications 6 à 11.

## Claims

1. Pharmaceutical composition containing as active ingredient at least one compound of formula: 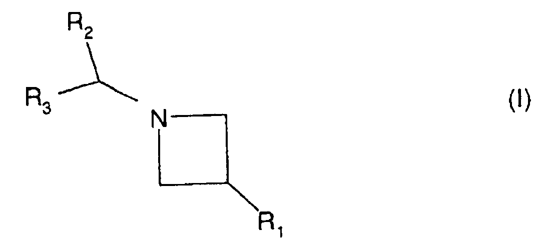 in which
R₁ represents a radical -N(R₄)R₅, -N(R₄)-CO-R₅, -N(R₄)-SO₂R₆,
R₂ and R₃, which are identical or different, represent either an aromatic chosen from phenyl, naphthyl and indenyl, these aromatics being unsubstituted or substituted with one or more halogen atoms or alkyl, alkoxy, formyl, hydroxyl, trifluoromethyl, trifluoromethoxy, -CO-alk, cyano, -COOH, COOalk, -CONR₇R₈, -CO-NH-NR₉R₁₀, alkylsulfanyl, alkylsulfinyl, alkylsulfonyl, alkylsulfanylalkyl, alkylsulfinylalkyl, alkylsulfonylalkyl, hydroxyalkyl or -alk-NR₇R₈ radicals; or a heteroaromatic chosen from benzofuryl, benzothiazolyl, benzothienyl, benzoxazolyl, chromanyl, 2,3-dihydrobenzofuryl, 2,3-dihydrobenzothienyl, furyl, imidazolyl, isochromanyl, isoquinolyl, pyrrolyl, pyridyl, pyrimidyl, quinolyl, 1,2,3,4-tetrahydroisoquinolyl, thiazolyl and thienyl rings, it being possible for these heteroaromatics to be unsubstituted or substituted with a halogen atom or an alkyl, alkoxy, hydroxyl, trifluoromethyl, trifluoromethoxy, cyano, -COOH, COOalk, -CO-NH-NR₉R₁₀, -CONR₇R₈, -alk-NR₉R₁₀, alkylsulfanyl, alkylsulfinyl, alkylsulfonyl, alkylsulfanylalkyl, alkylsulfinylalkyl, alkylsulfonylalkyl or hydroxyalkyl radical,
R₄ represents a radical -C(R₁₁)(R₁₂)-Het, -Het, -(CR₁₁)(R₁₂)-Ar, Ar, cycloalkyl or norbornyl,
R₅ represents a hydrogen atom or a hydroxyalkyl, -alk-COOalk, -alk-CONR₇R₈, -alk-NR₇R₈, alkoxy, Ar, Het, -CH₂Ar, -CH₂Het or alkyl radical optionally substituted with one or more halogen atoms,
R₆ represents a hydroxyalkyl, -alk-COOalk, -alk-CONR₇R₈, -alk-NR₇R₈, alkoxy, Ar, Het, -CH₂Ar, -CH₂Het or alkyl radical optionally substituted with 1 or more halogen atoms,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, -COOalk, cycloalkyl, alkylcycloalkyl, -alk-O-alk or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals,
R₁₁ represents a hydrogen atom or a hydroxyalkyl, -alk-COOalk, -alk-CONR₇R₈, -alk-NR₇R₈, alkoxyalkyl, Ar, Het, -CH₂Ar, -CH₂Het or alkyl radical optionally substituted with one or more halogen atoms,
R₁₂ represents a hydrogen atom or a hydroxyalkyl, -alk-COOalk, -alk-CONR₇R₈, -alk-NR₇R₈, alkoxyalkyl or alkyl radical optionally substituted with one or more halogen atoms,
or alternatively R₁₁ and R₁₂ together form with the carbon atom to which they are attached a 3- to 10-membered saturated mono- or bicyclic ring, optionally containing another heteroatom chosen from oxygen, sulfur and nitrogen and being optionally substituted with one or more alkyl radicals,
Ar represents a phenyl, naphthyl or indenyl radical, these different radicals being optionally substituted with one or more halogen atoms or alkyl, alkoxy, -CO-alk, cyano, -COOH, -COOalk, -CONR₁₃R₁₄, -CO-NH-NR₁₅R₁₆, alkylsulfanyl, alkylsulfinyl, alkylsulfonyl, -alk-NR₁₅R₁₆, -NR₁₅R₁₆, alkylthioalkyl, formyl, CF₃, OCF₃, Het, -O-alk-NH-cycloalkyl, SO₂NH₂, hydroxyl, hydroxyalkyl, -NHCOalk, NHCOOalk radicals or on 2 adjacent carbon atoms with dioxymethylene,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, alkoxycarbonyl, oxo or hydroxyl radicals, the nitrogen-containing heterocycles being optionally in their N-oxidized form,
R₁₃ and R₁₄, which are identical or different, represent a hydrogen atom or an alkyl radical or alternatively R₁₃ and R₁₄ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₅ and R₁₆, which are identical or different, represent a hydrogen atom or an alkyl radical or alternatively R₁₅ and R₁₆ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
alk represents an alkyl or alkylene radical,
the alkyl and alkylene radicals and portions and the alkoxy radicals and portions are in the form of a straight or branched chain and contain 1 to 6 carbon atoms and the cycloalkyl radicals contain 3 to 10 carbon atoms,
the optical isomers of these compounds and their pharmaceutically acceptable salts with an inorganic or organic acid.

2. Pharmaceutical composition according to Claim 1, for which in formula (I) Het is chosen from benzimidazole, benzoxazole, benzothiazole, benzothiophene, cinnoline, thiophene, quinazoline, quinoxaline, quinoline, pyrazole, pyrrole, pyridine, imidazole, indole, isoquinoline, pyrimidine, thiazole, thiadiazole, piperidine, piperazine, triazole, furan, tetrahydroisoquinoline, tetrahydroquinoline, these heterocycles being optionally substituted with one or more halogen atoms or alkyl, alkoxy, alkoxycarbonyl, oxo, hydroxyl, OCF₃ or CF₃ radicals.

3. Pharmaceutical composition according to Claim 1, for which in the compound of formula (I)
R₁ represents a radical -N(R₄)R₅ or -N(R₄)-SO₂R₆,
R₂ represents either a phenyl which is unsubstituted or substituted with one or more halogen atoms or alkyl, alkoxy, trifluoromethyl, trifluoromethoxy, -CO-alk, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₃ represents either a phenyl which is unsubstituted or substituted with one or more halogen atoms or alkyl, alkoxy, trifluoromethyl, trifluoromethoxy, -CO-alk, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₄ represents a radical -C(R₁₁)(R₁₂)-Het, -Het, -C(R₁₁)(R₁₂)-Ar, Ar or norbornyl,
R₅ represents a hydrogen atom or a hydroxyalkyl, -alk-COOalk, -alk-CONR₇R₈, -alk-NR₇R₈, alkoxy, -CH₂Ar, -CH₂Het or alkyl radical,
R₆ represents a hydroxyalkyl, -alk-COOalk, -alk-CONR₇R₈, -alk-NR₇R₈, alkoxy, -CH₂Ar, -CH₂Het or alkyl radical,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, cycloalkyl, alkylcycloalkyl, -alk-O-alk or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, oxo, hydroxyalkyl or -CO-NH₂ radicals,
R₁₁ represents a hydrogen atom or a hydroxyalkyl, -alk-COOalk, -alk-CONR₇R₈, -alk-NR₇R₈, alkoxyalkyl, Ar, Het, -CH₂Ar, -CH₂Het or alkyl radical optionally substituted with one or more halogen atoms,
R₁₂ represents a hydrogen atom or a hydroxyalkyl, -alk-COOalk, -alk-CONR₇R₈, -alk-NR₇R₈, alkoxyalkyl or alkyl radical optionally substituted with one or more halogen atoms,
or alternatively R₁₁ and R₁₂ together form with the carbon atom to which they are attached a 3- to 10-membered saturated mono- or bicyclic ring, optionally containing another heteroatom chosen from oxygen, sulfur and nitrogen and being optionally substituted with one or more alkyl radicals,
Ar represents a phenyl or naphthyl radical, these different radicals being optionally substituted with one or more halogen atoms or alkyl, alkoxy, - CO-alk, cyano, -CONR₁₃R₁₄, alkylsulfonyl, -alk-NR₁₅R₁₆, -NR₁₅R₁₆, CF₃, OCF₃, SO₂NH₂, hydroxyl or hydroxyalkyl radicals or on 2 adjacent carbon atoms with dioxymethylene,
Het represents a heterocycle chosen from benzimidazole, benzoxazole, benzothiazole, benzothiophene, thiophene, quinazoline, quinoxaline, quinoline, pyrrole, pyridine, imidazole, indole, isoquinoline, pyrimidine, thiazole, thiadiazole, furan, tetrahydroisoquinoline and tetrahydroquinoline, these heterocycles being optionally substituted with one or more halogen atoms or alkyl, alkoxy, alkoxycarbonyl, oxo, hydroxyl, OCF₃ or CF₃ radicals,
R₁₃ and R₁₄, which are identical or different, represent a hydrogen atom or an alkyl radical or alternatively R₁₃ and R₁₄ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₅ and R₁₆, which are identical or different, represent a hydrogen atom or an alkyl radical or alternatively R₁₅ and R₁₆ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
the optical isomers of these compounds and their pharmaceutically acceptable salts with an inorganic or organic acid.

4. Pharmaceutical composition according to Claim 1, for which in the compound of formula (I)
R₁ represents a radical -N(R₉)-SO₂R₆,
R₂ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈ or hydroxyalkyl radical,
R₃ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈ or hydroxyalkyl radical,
R₄ represents -Het or Ar,
R₆ represents a hydroxyalkyl or alkyl radical,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
Ar represents a phenyl or naphthyl radical, these different radicals being optionally substituted with one or more halogen atoms or alkyl, alkoxy, -CO-alk, cyano, -CONR₁₃R₁₄, -alk-NR₁₅R₁₆, -NR₁₅R₁₆, CF₃, OCF₃, SO₂NH₂, hydroxyl or hydroxyalkyl radicals,
Het represents represents a heterocycle chosen from benzimidazole, benzoxazole, benzothiazole, benzothiophene, thiophene, quinazoline, quinoxaline, quinoline, pyrrole, pyridine, imidazole, indole, isoquinoline, thiazole, thiadiazole, furan, tetrahydroisoquinoline and tetrahydroquinoline, these heterocycles being optionally substituted with one or more halogen atoms or alkyl, alkoxy, alkoxycarbonyl, oxo, hydroxyl, OCF₃ or CF₃ radicals,
R₁₃ and R₁₄, which are identical or different, represent a hydrogen atom or an alkyl radical or alternatively R₁₃ and R₁₄ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₅ and R₁₆, which are identical or different, represent a hydrogen atom or an alkyl radical or alternatively R₁₅ and R₁₆ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
the optical isomers of these compounds and their pharmaceutically acceptable salts with an inorganic or organic acid.

5. Composition according to Claim 1, for which the compound of formula (I) is chosen from the following compounds:
N-{1-[bis(4-chlorophenyl)methyl]azetidin-3-yl}-N-(6-chloropyrid-2-yl)methylsulfonamide,
N-{1-[bis(4-chlorophenyl)methyl]azetidin-3-yl}-N-(6-ethylpyrid-2-yl)methylsulfonamide,
N-{1-[bis(4-chlorophenyl)methyl]azetidin-3-yl}-N-quinol-6-ylmethylsulfonamide,
N-{1-[bis(4-chlorophenyl)methyl]azetidin-3-yl}-N-quinol-5-ylmethylsulfonamide,
N-{1-[bis(4-chlorophenyl)methyl]azetidin-3-yl}-N-isoquinol-5-ylmethylsulfonamide,
N-{1-[bis(4-chlorophenyl)methyl]azetidin-3-yl}-N-pyrid-3-ylmethylsulfonamide,
N-{1-[bis(4-chlorophenyl)methyl]azetidin-3-yl}-N-(1-oxide-pyrid-3-yl)methylsulfonamide,
N-{1R,2S,4S)-bicyclo[2.2.1]hept-2-yl-N-{1-[bis(4-chlorophenyl)methyl]azetidin-3-yl}methylsulfonamide,
N-{1R,2R,4S)-bicyclo[2.2.1]hept-2-yl-N-{1-[bis(4-chlorophenyl)methyl]azetidin-3-yl}methylsulfonamide,
N-{1-[bis(4-chlorophenyl)methyl]azetidin-3-yl}-N-(3,5-difluorophenyl)methylsulfonamide,
N-{1-[bis(4-chlorophenyl)methyl]azetidin-3-yl}-N-(thiazol-2-yl)methylsulfonamide,
N-{1-[bis(4-chlorophenyl)methyl]azetidin-3-yl}-N-(3-methoxyphenyl)methylsulfonamide,
N-{1-[bis(4-chlorophenyl)methyl]azetidin-3-yl}-N-(3-hydroxyphenyl)methylsulfonamide,
N-{1-[bis(4-chlorophenyl)methyl]azetidin-3-yl}-N-(3-hydroxymethylphenyl)methylsulfonamide,
Ethyl N-{1-[bis(4-chlorophenyl)methyl]azetidin-3-yl}-N-(methylsulfonyl)-3-aminobenzoate,
N-{1-[bis(4-chlorophenyl)methyl]azetidin-3-yl}-N-(1-isobutylpiperid-4-yl)methylsulfonamide,
N-benzyl-N-{1-[bis(4-chlorophenyl)methyl]azetidin-3-yl}amine,
N-{1-[bis(4-chlorophenyl)methyl]azetidin-3-yl}-N-(3,5-difluorobenzyl)amine,
N-{1-[bis(4-chlorophenyl)methyl]azetidin-3-yl}-N-(3,5-difluorobenzyl)methylsulfonamide,
N-{1-[bis(4-chloprophenyl)methyl]azetidin-3-yl}-N-(pyrid-3-ylmethyl)methylsulfonamide,
N-{1-[bis(4-fluorophenyl)methyl]azetidin-3-yl}-N-(3,5-difluorophenyl)methylsulfonamide,
(RS)-N-{1-[(4-chlorophenyl)pyrid-3-ylmethyl]azetidin-3-yl}-N-(3,5-difluorophenyl)methylsulfonamide,
(R)-N-{1-[(4-chlorophenyl)pyrid-3-ylmethyl]azetidin-3-yl}-N-(3,5-difluorophenyl)methylsulfonamide,
(S)-N-{1-[(4-chlorophenyl)pyrid-3-ylmethyl]azetidin-3-yl}-N-(3,5-difluorophenyl)methylsulfonamide,
(RS)-N-{1-[(4-chlorophenyl)pyrid-4-ylmethyl]azetidin-3-yl}-N-(3,5-difluorophenyl)methylsulfonamide,
(R)-N-{1-[(4-chlorophenyl)pyrid-4-ylmethyl]azetidin-3-yl}-N-(3,5-difluorophenyl)methylsulfonamide,
(S)-N-{1-[(4-chlorophenyl)pyrid-4-ylmethyl]azetidin-3-yl}-N-(3,5-difluorophenyl)methylsulfonamide,
(RS)-N-{1-[(4-chlorophenyl)pyrimid-5-ylmethyl]azetidin-3-yl}-N-(3,5-difluorophenyl)methylsulfonamide,
(R)-N-{1-[(4-chlorophenyl)pyrimid-5-ylmethyl]azetidin-3-yl}-N-(3,5-difluorophenyl)methylsulfonamide,
(S)-N-{1-[(4-chlorophenyl)pyrimid-5-ylmethyl]azetidin-3-yl}-N-(3,5-difluorophenyl)methylsulfonamide,
N-{1-[bis(4-chlorophenyl)methyl]azetidin-3-yl}-N-(3,5-difluorophenyl)benzylsulfonamide,
their optical isomers and their pharmaceutically acceptable salts with an inorganic or organic acid.

6. Compound of formula: 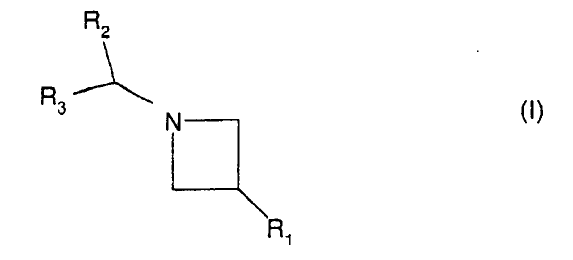 in which
R₁ represents a radical -N(R₄)R₅, -N(R₄)-CO-R₅, -N(R₄)-SO₂R₆,
R₂ and R₃, which are identical or different, represent either an aromatic chosen from phenyl, naphthyl and indenyl, these aromatics being unsubstituted or substituted with one or more halogen atoms or alkyl, alkoxy, formyl, hydroxyl, trifluoromethyl, trifluoromethoxy, -CO-alk, cyano, -COOH, COOalk, -CONR₇R₈, -CO-NH-NR₉R₁₀, alkylsulfanyl, alkylsulfinyl, alkylsulfonyl, alkylsulfanylalkyl, alkylsulfinylalkyl, alkylsulfonylalkyl, hydroxyalkyl or -alk-NR₇R₈ radicals; or a heteroaromatic chosen from benzofuryl, benzothiazolyl, benzothienyl, benzoxazolyl, chromanyl, 2,3-dihydrobenzofuryl, 2,3-dihydrobenzothienyl, furyl, imidazolyl, isochromanyl, isoquinolyl, pyrrolyl, pyridyl, pyrimidyl, quinolyl, 1,2,3,4-tetrahydroisoquinolyl, thiazolyl and thienyl rings, it being possible for these heteroaromatics to be unsubstituted or substituted with a halogen atom or alkyl, alkoxy, hydroxyl, trifluoromethyl, trifluoromethoxy, cyano, -COOH, COOalk, -CO-NH-NR₉R₁₀, -CONR₇R₈, -alk-NR₉R₁₀, alkylsulfanyl, alkylsulfinyl, alkylsulfonyl, alkylsulfanylalkyl, alkylsulfinylalkyl, alkylsulfonylalkyl or hydroxyalkyl radical,
R₄ represents a radical -C(R₁₁)(R₁₂)-Het, -Het, -(CR₁₁) (R₁₂)-Ar, Ar, cycloalkyl or norbornyl,
R₅ represents a hydrogen atom or a hydroxyalkyl, -alk-COOalk, -alk-CONR₇R₈, -alk-NR₇R₈, alkoxy, Ar, Het, -CH₂Ar, -CH₂Het or alkyl radical optionally substituted with one or more halogen atoms,
R₆ represents a hydroxyalkyl, -alk-COOalk, -alk-CONR₇R₈, -alk-NR₇R₈, alkoxy, Ar, Het, -CH₂Ar, -CH₂Het or alkyl radical optionally substituted with 1 or more halogen atoms,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, -COOalk, cycloalkyl, alkylcycloalkyl, -alk-O-alk or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals R₁₁ represents a hydrogen atom or a hydroxyalkyl, -alk-COOalk, -alk-CONR₇R₈, -alk-NR₇R₈, alkoxyalkyl, Ar, Het, -CH₂Ar, -CH₂Het or alkyl radical optionally substituted with one or more halogen atoms,
R₁₂ represents a hydrogen atom or a hydroxyalkyl, -alk-COOalk, -alk-CONR₇R₈, -alk-NR₇R₈, alkoxyalkyl or alkyl radical optionally substituted with one or more halogen atoms,
or alternatively R₁₁ and R₁₂ together form with the carbon atom to which they are attached a 3- to 10-membered saturated mono- or bicyclic ring, optionally containing another heteroatom chosen from oxygen, sulfur and nitrogen and being optionally substituted with one or more alkyl radicals,
Ar represents a phenyl, naphthyl or indenyl radical, these different radicals being optionally substituted with one or more halogen atoms or alkyl, alkoxy, -CO-alk, cyano, -COOH, -COOalk, -CONR₁₃R₁₄, -CO-NH-NR₁₅R₁₆, alkylsulfanyl, alkylsulfinyl, alkylsulfonyl, -alk-NR₁₅R₁₆. -NR₁₅R₁₆, alkylthioalkyl, formyl, CF₃, OCF₃, Het, -O-alk-NH-cycloalkyl, SO₂NH₂, hydroxyl, hydroxyalkyl, -NHCOalk, NHCOOalk radicals or on 2 adjacent carbon atoms with dioxymethylene,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, alkoxycarbonyl, oxo or hydroxyl radicals, the nitrogen-containing heterocycles being optionally in their N-oxidized form,
R₁₃ and R₁₄, which are identical or different, represent a hydrogen atom or an alkyl radical or alternatively R₁₃ and R₁₄ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₅ and R₁₆, which are identical or different, represent a hydrogen atom or an alkyl radical or alternatively R₁₅ and R₁₆ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
alk represents an alkyl or alkylene radical,
the alkyl and alkylene radicals and portions and the alkoxy radicals and portions are in the form of a straight or branched chain and contain 1 to 6 carbon atoms and the cycloalkyl radicals contain 3 to 10 carbon atoms,
the optical isomers of these compounds and their pharmaceutically acceptable salts with an inorganic or organic acid,
with the exception of the compound for which R₂ and R₃ represent phenyl radicals, R₁ represents a radical -N(R₄)SO₂R₆ for which R₄ represents a phenyl radical and R₆ represents a methyl radical.

7. Compound of formula (I) according to Claim 6, for which Het is chosen from benzimidazole, benzoxazole, benzothiazole, benzothiophene, cinnoline, thiophene, quinazoline, quinoxaline, quinoline, pyrazole, pyrrole, pyridine, imidazole, indole, isoquinoline, pyrimidine, thiazole, thiadiazole, piperidine, piperazine, triazole, furan, tetrahydroisoquinoline, tetrahydroquinoline, these heterocycles being optionally substituted with one or more halogen atoms or alkyl, alkoxy, alkoxycarbonyl, oxo, hydroxyl, OCF₃ or CF₃ radicals.

8. Compound of formula (I) according to Claim 6, for which
R₁ represents a radical -N(R₄)R₅ or -N(R₄)-SO₂R₆,
R₂ represents either a phenyl which is unsubstituted or substituted with one or more halogen atoms or alkyl, alkoxy, trifluoromethyl, trifluoromethoxy, -CO-alk, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₃ represents either a phenyl which is unsubstituted or substituted with one or more halogen atoms or alkyl, alkoxy, trifluoromethyl, trifluoromethoxy, -CO-alk, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₄ represents a radical -C(R₁₁)(R₁₂)-Het, -Het, -C(R₁₁)(R₁₂)-Ar, Ar or norbornyl,
R₅ represents a hydrogen atom or a hydroxyalkyl, -alk-COOalk, -alk-CONR₇R₈, -alk-NR₇R₈, alkoxy, -CH₂Ar, -CH₂Het or alkyl radical,
R₆ represents a hydroxyalkyl, -alk-COOalk, -alk-CONR₇R₈, -alk-NR₇R₈, alkoxy, -CH₂Ar, -CH₂Het or alkyl radical,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, cycloalkyl, alkylcycloalkyl, -alk-O-alk or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, oxo, hydroxyalkyl or -CO-NH₂ radicals,
R₁₁ represents a hydrogen atom or a hydroxyalkyl, -alk-COOalk, -alk-CONR₇R₈, -alk-NR₇R₈, alkoxyalkyl, Ar, Het, -CH₂Ar, -CH₂Het or alkyl radical optionally substituted with one or more halogen atoms,
R₁₂ represents a hydrogen atom or a hydroxyalkyl, -alk-COOalk, -alk-CONR₇R₈, -alk-NR₇R₈, alkoxyalkyl or alkyl radical optionally substituted with one or more halogen atoms,
or alternatively R₁₁ and R₁₂ together form with the carbon atom to which they are attached a 3- to 10-membered saturated mono- or bicyclic ring, optionally containing another heteroatom chosen from oxygen, sulfur and nitrogen and being optionally substituted with one or more alkyl radicals,
Ar represents a phenyl or naphthyl radical, these different radicals being optionally substituted with one or more halogen atoms or alkyl, alkoxy, -CO-alk, cyano, -CONR₁₃R₁₄, alkylsulfonyl, -alk-NR₁₅R₁₆, -NR₁₅R₁₆, CF₃, OCF₃, SO₂NH₂, hydroxyl or hydroxyalkyl radicals or on 2 adjacent carbon atoms with dioxymethylene,
Het represents a heterocycle chosen from benzimidazole, benzoxazole, benzothiazole, benzothiophene, thiophene, quinazoline, quinoxaline, quinoline, pyrrole, pyridine, imidazole, indole, isoquinoline, pyrimidine, thiazole, thiadiazole, furan, tetrahydroisoquinoline and tetrahydroquinoline, these heterocycles being optionally substituted with one or more halogen atoms or alkyl, alkoxy, alkoxycarbonyl, oxo, hydroxyl, OCF₃ or CF₃ radicals,
R₁₃ and R₁₄, which are identical or different, represent a hydrogen atom or an alkyl radical or alternatively R₁₃ and R₁₄ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₅ and R₁₆, which are identical or different, represent a hydrogen atom or an alkyl radical or alternatively R₁₅ and R₁₆ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
the optical isomers of these compounds and their pharmaceutically acceptable salts with an inorganic or organic acid,
with the exception of the compound for which R₂ and R₃ represent phenyl radicals, R₁ represents a radical -N(R₄)SO₂R₆ for which R₄ represents a phenyl radical and R₆ represents a methyl radical.

9. Compound of formula (I) according to Claim 6, in which
R₁ represents a radical -N(R₄)-SO₂R₆,
R₂ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈ or hydroxyalkyl radical,
R₃ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈ or hydroxyalkyl radical,
R₄ represents -Het or Ar,
R₆ represents a hydroxyalkyl or alkyl radical,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
Ar represents a phenyl or naphthyl radical, these different radicals being optionally substituted with one or more halogen atoms or alkyl, alkoxy, -CO-alk, cyano, -CONR₁₃R₁₄, -alk-NR₁₅R₁₆. -NR₁₅R₁₆, CF₃, OCF₃, SO₂NH₂, hydroxyl or hydroxyalkyl radicals,
Het represents represents a heterocycle chosen from benzimidazole, benzoxazole, benzothiazole, benzothiophene, thiophene, quinazoline, quinoxaline, quinoline, pyrrole, pyridine, imidazole, indole, isoquinoline, thiazole, thiadiazole, furan, tetrahydroisoquinoline and tetrahydroquinoline, these heterocycles being optionally substituted with one or more halogen atoms or alkyl, alkoxy, alkoxycarbonyl, oxo, hydroxyl, OCF₃ or CF₃ radicals,
R₁₃ and R₁₄, which are identical or different, represent a hydrogen atom or an alkyl radical or alternatively R₁₃ and R₁₄ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₅ and R₁₆, which are identical or different, represent a hydrogen atom or an alkyl radical or alternatively R₁₅ and R₁₆ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
the optical isomers of these compounds and their pharmaceutically acceptable salts with an inorganic or organic acid,
with the exception of the compound for which R₂ and R₃ represent phenyl radicals, R₁ represents a radical -N(R₄)SO₂R₆ for which R₄ represents a phenyl radical and R₆ represents a methyl radical.

10. Compound of formula (I) according to Claim 6, chosen from the following compounds:
N-{1-[bis(4-chlorophenyl)methyl]azetidin-3-yl}-N-(6-chloropyrid-2-yl)methylsulfonamide,
N-{1-[bis(4-chlorophenyl)methyl]azetidin-3-yl}-N-(6-ethylpyrid-2-yl)methylsulfonamide, N-{1-[bis(4-chlorophenyl)methyl]azetidin-3-yl}-N-quinol-6-ylmethylsulfonamide,
N-{1-[bis(4-chlorophenyl)methyl]azetidin-3-yl}-N-quinol-5-ylmethylsulfonamide,
N-{1-[bis(4-chlorophenyl)methyl]azetidin-3-yl}-N-isoquinol-5-ylmethylsulfonamide,
N-{1-[bis(4-chlorophenyl)methyl]azetidin-3-yl}-N-pyrid-3-ylmethylsulfonamide,
N-{1-[bis(4-chlorophenyl)methyl]azetidin-3-yl}-N-(1-oxide-pyrid-3-yl)methylsulfonamide,
N-{1R,2S,4S)-bicyclo[2.2.1]hept-2-yl-N-{1-[bis(4-chlorophenyl)methyl]azetidin-3-yl}methylsulfonamide,
N-{1R,2R,4S)-bicyclo[2.2.1]hept-2-yl-N-{1-[bis(4-chlorophenyl)methyl]azetidin-3-yl}methylsulfonamide,
N-{1-[bis(4-chlorophenyl)methyl]azetidin-3-yl}-N-(3,5-difluorophenyl)methylsulfonamide,
N-{1-[bis(4-chlorophenyl)methyl]azetidin-3-yl}-N-(thiazol-2-yl)methylsulfonamide,
N-{1-[bis(4-chlorophenyl)methyl]azetidin-3-yl}-N-(3-methoxyphenyl)methylsulfonamide,
N-{1-[bis(4-chlorophenyl)methyl]azetidin-3-yl}-N-(3-hydroxyphenyl)methylsulfonamide,
N-{1-[bis(4-chlorophenyl)methyl]azetidin-3-yl}-N-(3-hydroxymethylphenyl)methylsulfonamide,
Ethyl N-{1-[bis(4-chlorophenyl)methyl]azetidin-3-yl}-N-(methylsulfonyl)-3-aminobenzoate,
N-{1-[bis(4-chlorophenyl)methyl]azetidin-3-yl}-N-(1-isobutylpiperid-4-yl)methylsulfonamide,
N-benzyl-N-{1-[bis(4-chlorophenyl)methyl]azetidin-3-yl}amine,
N-{1-[bis(4-chlorophenyl)methyl]azetidin-3-yl}-N-(3,5-difluorobenzyl)amine,
N-{1-[bis(4-chlorophenyl)methyl]azetidin-3-yl}-N-(3,5-difluorobenzyl)methylsulfonamide,
N-{1-[bis(4-chlorophenyl)methyl]azetidin-3-yl}-N-(pyrid-3-ylmethyl)methylsulfonamide,
N-{1-[bis(4-fluorophenyl)methyl]azetidin-3-yl}-N-(3,5-difluorophenyl)methylsulfonamide,
(RS)-N-{1-[(4-chlorophenyl)pyrid-3-ylmethyl]azetidin-3-yl}-N-(3,5-difluorophenyl)methylsulfonamide,
(R)-N-{1-[(4-chlorophenyl)pyrid-3-ylmethyl]azetidin-3-yl}-N-(3,5-difluorophenyl)methylsulfonamide,
(S)-N-{1-[(4-chlorophenyl)pyrid-3-ylmethyl]azetidin-3-yl}-N-(3,5-difluorophenyl)methylsulfonamide,
(RS)-N-{1-[(4-chlorophenyl)pyrid-4-ylmethyl]azetidin-3-yl}-N-(3,5-difluorophenyl)methylsulfonamide,
(R)-N-{1-[(4-chlorophenyl)pyrid-4-ylmethyl]azetidin-3-yl}-N-(3,5-difluorophenyl)methylsulfonamide,
(S)-N-{1-[(4-chlorophenyl)pyrid-4-ylmethyl]azetidin-3-yl}-N-(3,5-difluorophenyl)methylsulfonamide,
(RS)-N-{1-[(4-chlorophenyl)pyrimid-5-ylmethyl]azetidin-3-yl}-N-(3,5-difluorophenyl)methylsulfonamide,
(R)-N-{1-[(4-chlorophenyl)pyrimid-5-ylmethyl]azetidin-3-yl}-N-(3,5-difluorophenyl)methylsulfonamide,
(S)-N-{1-[(4-chlorophenyl)pyrimid-5-ylmethyl]azetidin-3-yl}-N-(3,5-difluorophenyl)methylsulfonamide,
N-{1-[bis(4-chlorophenyl)methyl]azetidin-3-yl}-N-(3,5-difluorophenyl)benzylsulfonamide,
their optical isomers and their pharmaceutically acceptable salts with an inorganic or organic acid.

11. N-{1-[bis(4-Chlorophenyl)methyl)azetidin-3-yl}-N-(3,5-difluorophenyl)benzylsulfonamide, its optical isomers and its pharmaceutically acceptable salts with an inorganic or organic acid.

12. Process for preparing the compounds of formula (I) according to Claim 6, for which R₁ represents a radical -N(R₄)R₅ in which R₅ is a hydrogen atom, R₄ is a radical -CR₁₁R₁₂-Ar or -CR₁₁R₁₂-Het and R₁₂ is a hydrogen atom, **characterized in that** a derivative Rb-COR₁₁ for which R₁₁ has the same meanings as in Claim 6 is reacted with a derivative of formula: 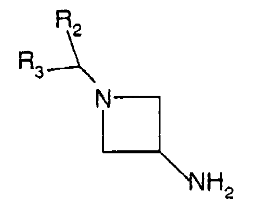 Rb represents a radical Ar or Het, R₂, R₃, R₁₁ Ar and Het have the same meanings as in Claim 6, the product is isolated and it is optionally converted to a pharmaceutically acceptable salt.

13. Process for preparing compounds of formula (I) according to Claim 6, for which R₁ represents a radical -N(R₄)-CO-R₅ in which R₄ is a radical -C(R₁₁)(R₁₂)-Het or -C(R₁₁)(R₁₂)-Ar and R₁₂ is a hydrogen atom, **characterized in that** a derivative Hal-COR₅ is reacted with a derivative of formula: 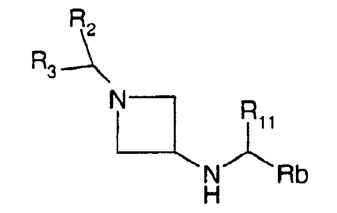 Hal represents a halogen atom, Rb represents a radical Ar or Het, and R₂, R₃, R₅, R₁₁, Ar and Het have the same meanings as in Claim 6, the product is isolated and it is optionally converted to a pharmaceutically acceptable salt.

14. Process for preparing the compounds of formula (I) according to Claim 6, for which R₁ represents a radical -N(R₄)-SO₂R₆ in which R₄ is a radical -C(R₁₁) (R₁₂)-Ar or -C(R₁₁)(R₁₂) -Het and R₁₂ is a hydrogen atom, **characterized in that** a derivative Hal-SO₂R₆ is reacted with a derivative of formula: 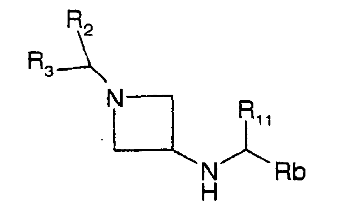 R₂, R₃, R₁₁ and R₅ have the same meanings as in Claim 6, Hal represents a halogen atom and Rb represents a radical Ar and Het, the product is isolated and it is optionally converted to a pharmaceutically acceptable salt.

15. Process for preparing the compounds of formula (I) according to Claim 6 for which R₁ represents a radical -N(R₄)R₅, **characterized in that** a derivative R₅(R₄)NH is reacted with a derivative of formula: 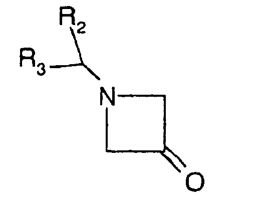 R₂, R₃, R₄ and R₅ have the same meanings as in Claim 6, the product is isolated and it is optionally converted to a pharmaceutically acceptable salt.

16. Process for preparing the compounds of formula (I) according to Claim 6 for which R₁ represents a radical -N (R₄)SO₂R₆, **characterized in that** a derivative Hal-SO₂R₆ is reacted with a derivative of formula: 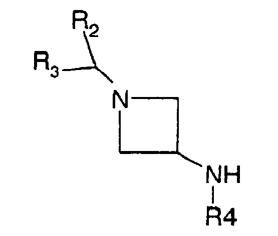 R₂, R₃, R₄ and R₆ have the same meanings as in Claim 6 and Hal represents a halogen atom, the product is isolated and optionally converted to a pharmaceutically acceptable salt.

17. Process for preparing the compounds of formula (I) according to Claim 6 for which R₁ represents a radical -N(R₄)COR₅, **characterized in that** a derivative Hal-COR₆ is reacted with a derivative of formula: 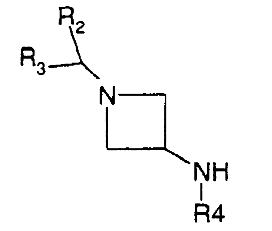 R₂, R₃, R₄ and R₅ have the same meanings as in Claim 6 and Hal represents a halogen atom, the product is isolated and optionally converted to a pharmaceutically acceptable salt.

18. Process for preparing the compounds of formula (I) according to Claim 6 for which R₁ represents a radical -N(R₄)-SO₂-R₆, R₄ is a radical Het or Ar, **characterized in that** a derivative Rd-NH-SO₂-R₆ is reacted with a derivative of formula: 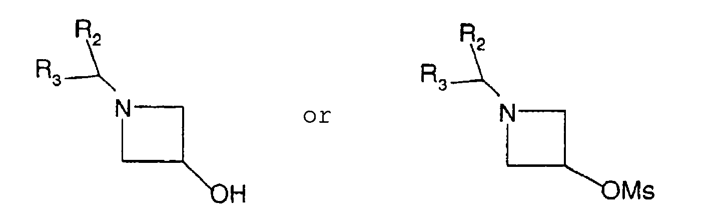 Rd represents a radical Ar or Het, R₂, R₃ and R₆ have the same meanings as in Claim 6 and Ms represents a methylsulfonyloxy radical, the product is isolated and it is optionally converted to a pharmaceutically acceptable salt.

19. Process for preparing the compounds of formula (I) according to Claim 6, **characterized in that** a derivative R₂-CHBr-R₃ is reacted with a derivative of formula: 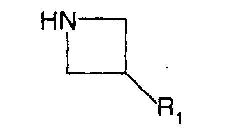 R₁, R₂ and R₃ have the same meanings as in Claim 6, the product is isolated and it is optionally converted to a pharmaceutically acceptable salt.

20. Process for preparing the compounds of formula (I) according to Claim 6 for which R₁ represents a radical -N(R₄)-SO₂-R₆ for which R₄ is a piperid-4-yl radical substituted on the nitrogen with an alkyl radical, **characterized in that** a corresponding compound of formula (I) for which R₁ represents a radical -N(R₄)-SO₂-R₆ for which R₄ is a piperid-4-yl radical is alkylated, the product is isolated and it is optionally converted to a pharmaceutically acceptable salt.

21. Process for preparing the compounds of formula (I) according to Claim 6, for which R₁ represents a radical -N(R₄)-SO₂-R₆ for which R₄ is a phenyl radical substituted with a pyrrolid-1-yl radical, **characterized in that** pyrrolidine is reacted with a corresponding compound of formula (I) for which R₁ represents a radical -N(R₄)SO₂R₆ for which R₄ is a phenyl radical substituted with a halogen atom, the product is isolated and it is optionally converted to a pharmaceutically acceptable salt.

22. Medicament containing as an active ingredient at least one compound of formula (I) according to one of claims 6 to 11.

## Patentansprüche

1. Pharmazeutische Zusammensetzung, die als Wirkstoff mindestens eine Verbindung der folgenden Formel enthält: 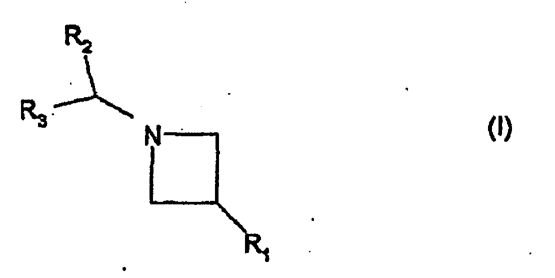 worin
R₁ für einen Rest -N(R₄)R₅, -N(R₄)-CO-R₅, -N(R₄)-SO₂R₆ steht,
R₂ und R₃, die gleich oder verschieden sind, entweder für einen Aromaten stehen, der aus Phenyl, Naphtyl und Indenyl ausgewählt ist, wobei diese Aromaten unsubstituiert oder mit einem oder mehreren Halogen, Alkyl, Alkoxy, Formyl, Hydroxy, Trifluormethyl, Trifluormethoxy, -CO-alk, Cyano, -COOH, COOalk, -CONR₇R₈, -CO-NH-NR₉R₁₀, Alkylsulfanyl, Alkylsulfinyl, Alkylsulfonyl, Alkylsulfanylalkyl, Alkylsulfinylalkyl, Alkylsulfonylalkyl, Hydroxyalkyl oder -alk-NR₇R₈ substituiert sind; oder für einen Heteroaromaten, der aus den Ringen Benzofuryl, Benzothiazolyl, Benzothienyl, Benzoxazolyl, Chromanyl, 2,3-Dihydrobenzofuryl, 2,3-Dihydrobenzothienyl, Furyl, Imidazolyl, Isochromanyl, Isochinolyl, Pyrrolyl, Pyridyl, Pyrimidyl, Chinolyl, 1,2,3,4-Tetrahydroisochinolyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, Cyano, -COOH, COOalk, -CO-NH-NR₉R₁₀, -CONR₇R₈, -alk-NR₉R₁₀, Alkylsulfanyl, Alkylsulfinyl, Alkylsulfonyl, Alkylsulfanylalkyl, Alkylsulfinylalkyl, Alkylsulfonylalkyl oder Hydroxyalkyl substituiert sein können,
R₄ für einen Rest -C(R₁₁)(R₁₂)-Het, -Het, -C(R₁₁)(R₁₂)-Ar, Ar, Cycloalkyl oder Norbornyl steht,
R₅ für ein Wasserstoffatom oder einen Rest Hydroxyalkyl, -alk-COOalk, -alk-CONR₇R₈, -alk-NR₇R₈, Alkoxy, Ar, Het, -CH₂Ar, -CH₂Het oder Alkyl steht, der gegebenenfalls mit einem oder mehreren Halogen substituiert ist,
R₆ für einen Rest Hydroxyalkyl, -alk-COOalk, -alk-CONR₇R₈, -alk-NR₇R₈, Alkoxy, Ar, Het, -CH₂Ar, -CH₂Het oder Alkyl steht, der gegebenenfalls mit 1 oder mehreren Halogen substituiert ist,
R₇ und R₈, die gleich oder verschieden sind, für ein Wasserstoffatom oder einen Alkylrest stehen oder auch R₇ und R₈ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₉ und R₁₀, die gleich oder verschieden sind, für ein Wasserstoffatom oder einen Rest Alkyl, -COOalk, Cycloalkyl, Alkylcycloalkyl, -alk-O-alk, Hydroxyalkyl stehen oder auch R₉ und R₁₀ zusammen mit dem Stickstoffatom, an das sie gebunden sind, einen gesättigten oder ungesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl, -COalk, -COOalk, -CO-NHalk, -CS-NHalk, Oxo, Hydroxyalkyl, -alk-O-alk oder -CO-NH₂ substituiert ist,
R₁₁ für ein Wasserstoffatom oder einen Rest Hydroxyalkyl, -alk-COOalk, -alk-CONR₇R₈, -alk-NR₇R₈, Alkoxyalkyl, Ar, Het, -CH₂Ar, -CH₂Het oder Alkyl steht, der gegebenenfalls mit einem oder mehreren Halogen substituiert ist,
R₁₂ für ein Wasserstoffatom oder einen Rest Hydroxyalkyl, -alk-COOalk, -alk-CONR₇R₈, -alk-NR₇R₈, Alkoxyalkyl oder Alkyl steht, der gegebenenfalls mit einem oder mehreren Halogen substituiert ist,
oder auch R₁₁ und R₁₂ zusammen mit dem Kohlenstoffatom, an das sie gebunden sind, einen gesättigten, mono- oder bicyclischen Ring mit 3 bis 10 Ringgliedern bilden, der gegebenenfalls ein anderes Heteroatom, ausgewählt aus Sauerstoff, Schwefel und Stickstoff, enthält und der gegebenenfalls mit einem oder mehreren Alkyl substituiert ist,
Ar für einen Phenyl-, Naphtyl- oder Indenylrest steht, wobei diese verschiedenen Reste gegebenenfalls mit einem oder mehreren Halogen, Alkyl, Alkoxy, -CO-alk, Cyano, -COOH, -COOalk, -CONR₁₃R₁₄, -CO-NH-NR₁₅R₁₆, Alkylsulfanyl, Alkylsulfinyl, Alkylsulfonyl, -alk-NR₁₅R₁₆, -NR₁₅R₁₆, Alkylthioalkyl, Formyl, CF₃, OCF₃, Het, -O-alk-NH-cycloalkyl, SO₂NH₂, Hydroxy, Hydroxyalkyl, -NHCOalk, NHCOOalk oder an 2 benachbarten Kohlenstoffatomen mit Dioxymethylen substituiert sind,
Het für einen ungesättigten oder gesättigten, mono- oder bicyclischen Heterocyclus steht, der 3 bis 10 Ringglieder besitzt und ein oder mehrere Heteroatome, ausgewählt aus Sauerstoff, Schwefel und Stickstoff, enthält und der gegebenenfalls mit einem oder mehreren Alkyl, Alkoxy, Halogen, Alkoxycarbonyl, Oxo, Hydroxy substituiert ist, wobei die stickstoffhaltigen Heterocyclen gegebenenfalls in ihrer N-oxidierten Form vorliegen,
R₁₃ und R₁₄, die gleich oder verschieden sind, für ein Wasserstoffatom oder einen Alkylrest stehen oder auch R₁₃ und R₁₄ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff enthält und der gegebenenfalls mit einem oder mehreren Alkyl substituiert ist,
R₁₅ und R₁₆, die gleich oder verschieden sind, für ein Wasserstoffatom oder einen Alkylrest stehen oder auch R₁₅ und R₁₆ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
alk für einen Alkyl- oder Alkylenrest steht,
wobei die Alkyl- und Alkylenreste und -anteile und die Alkoxyreste und -anteile geradkettig oder verzweigt sind und 1 bis 6 Kohlenstoffatome enthalten und die Cycloalkylreste 3 bis 10 Kohlenstoffatome enthalten,
die optischen Isomere dieser Verbindungen und ihre pharmazeutisch annehmbaren Salze mit einer anorganischen oder organischen Säure.

2. Pharmazeutische Zusammensetzung nach Anspruch 1, wobei in der Formel (I) Het aus Benzimidazol, Benzoxazol, Benzothiazol, Benzothiophen, Cinnolin, Thiophen, Chinazolin, Chinoxalin, Chinolin, Pyrazol, Pyrrol, Pyridin, Imidazol, Indol, Isochinolin, Pyrimidin, Thiazol, Thiadiazol, Piperidin, Piperazin, Triazol, Furan, Tetrahydroisochinolin, Tetrahydrochinolin ausgewählt ist, wobei diese Heterocyclen gegebenenfalls mit einem oder mehreren Alkyl, Alkoxy, Halogen, Alkoxycarbonyl, Oxo, Hydroxy, OCF₃ oder CF₃ substituiert sind.

3. Pharmazeutische Zusammensetzung nach Anspruch 1, wobei in der Verbindung der Formel (I)
R₁ für einen Rest -N(R₄)R₅, -N(R₄)-SO₂R₆ steht,
R₂ entweder für ein Phenyl steht, das unsubstituiert oder mit einem oder mehreren Halogen, Alkyl, Alkoxy, Trifluormethyl, Trifluormethoxy, -CO-alk, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈, -alk-NR₉R₁₀, Alkylsulfanyl, Alkylsulfinyl, Alkylsulfonyl oder Hydroxyalkyl substituiert sein können,
R₃ entweder für ein Phenyl steht, das unsubstituiert oder mit einem oder mehreren Halogen, Alkyl, Alkoxy, Trifluormethyl, Trifluormethoxy, -CO-alk, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈, -alk-NR₉R₁₀, Alkylsulfanyl, Alkylsulfinyl, Alkylsulfonyl oder Hydroxyalkyl substituiert sein können,
R₄ für einen Rest -C(R₁₁)(R₁₂) -Het, -Het, -C(R₁₁)(R₁₂)-Ar, Ar oder Norbornyl steht,
R₅ für ein Wasserstoffatom oder einen Rest Hydroxyalkyl, -alk-COOalk, -alk-CONR₇R₈, -alk-NR₇R₈, Alkoxy, -CH₂Ar, -CH₂Het oder Alkyl steht,
R₆ für einen Rest Hydroxyalkyl, -alk-COOalk, -alk-CONR₇R₈, -alk-NR₇R₈, Alkoxy, -CH₂Ar, -CH₂Het oder Alkyl steht,
R₇ und R₈, die gleich oder verschieden sind, für ein Wasserstoffatom oder einen Alkylrest stehen oder auch R₇ und R₈ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₉ und R₁₀, die gleich oder verschieden sind, für ein Wasserstoffatom oder einen Rest Alkyl, Cycloalkyl, Alkylcycloalkyl, -alk-O-alk oder Hydroxyalkyl stehen oder auch R₉ und R₁₀ zusammen mit dem Stickstoffatom, an das sie gebunden sind, einen gesättigten oder ungesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl, -COalk, -COOalk, -CO-NHalk, Oxo, Hydroxyalkyl oder -CO-NH₂ substituiert ist,
R₁₁ für ein Wasserstoffatom oder einen Rest Hydroxyalkyl, -alk-COOalk, -alk-CONR₇R₈, -alk-NR₇R₈, Alkoxyalkyl, Ar, Het, -CH₂Ar, -CH₂Het oder Alkyl steht, der gegebenenfalls mit einem oder mehreren Halogen substituiert ist,
R₁₂ für ein Wasserstoffatom oder einen Rest Hydroxyalkyl, -alk-COOalk, -alk-CONR₇R₈, -alk-NR₇R₈, Alkoxyalkyl oder Alkyl steht, der gegebenenfalls mit einem oder mehreren Halogen substituiert ist,
oder auch R₁₁ und R₁₂ zusammen mit dem Kohlenstoffatom, an das sie gebunden sind, einen gesättigten, mono- oder bicyclischen Ring mit 3 bis 10 Ringgliedern bilden, der gegebenenfalls ein anderes Heteroatom, ausgewählt aus Sauerstoff, Schwefel und Stickstoff, enthält und der gegebenenfalls mit einem oder mehreren Alkyl substituiert ist,
Ar für einen Phenyl- oder Naphtylrest steht, wobei diese verschiedenen Reste gegebenenfalls mit einem oder mehreren Halogen, Alkyl, Alkoxy, -CO-alk, Cyano, -CONR₁₃R₁₄, Alkylsulfonyl, -alk-NR₁₅R₁₆, -NR₁₅R₁₆, CF₃, OCF₃, SO₂NH₂, Hydroxy, Hydroxyalkyl oder an 2 benachbarten Kohlenstoffatomen mit Dioxymethylen substituiert sind,
Het für einen Heterocyclus steht, der aus Benzimidazol, Benzoxazol, Benzothiazol, Benzothiophen, Thiophen, Chinazolin, Chinoxalin, Chinolin, Pyrrol, Pyridin, Imidazol, Indol, Isochinolin, Pyrimidin, Thiazol, Thiadiazol, Furan, Tetrahydroisochinolin und Tetrahydrochinolin ausgewählt ist, wobei diese Heterocyclen gegebenenfalls mit einem oder mehreren Alkyl, Alkoxy, Halogen, Alkoxycarbonyl, Oxo, Hydroxy, OCF₃ oder CF₃ substituiert sind,
R₁₃ und R₁₄, die gleich oder verschieden sind, für ein Wasserstoffatom oder einen Alkylrest stehen oder auch R₁₃ und R₁₄ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₁₅ und R₁₆, die gleich oder verschieden sind, für ein Wasserstoffatom oder einen Alkylrest stehen oder auch R₁₅ und R₁₆ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
die optischen Isomere dieser Verbindungen und ihre pharmazeutisch annehmbaren Salze mit einer anorganischen oder organischen Säure.

4. Pharmazeutische Zusammensetzung nach Anspruch 1, wobei in der Verbindung der Formel (I)
R₁ für einen Rest -N(R₄)-SO₂R₆ steht,
R₂ entweder für ein Phenyl steht, das unsubstituiert oder mit einem oder mehreren Halogen, Alkyl, Alkoxy, Trifluormethyl, Trifluormethoxy, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈ oder Hydroxyalkyl substituiert sein können,
R₃ entweder für ein Phenyl steht, das unsubstituiert oder mit einem oder mehreren Halogen, Alkyl, Alkoxy, Trifluormethyl, Trifluormethoxy, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈ oder Hydroxyalkyl substituiert sein können,
R₄ für -Het oder Ar steht,
R₆ für einen Hydroxyalkyl- oder Alkylrest steht,
R₇ und R₈, die gleich oder verschieden sind, für ein Wasserstoffatom oder einen Alkylrest stehen oder auch R₇ und R₈ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
Ar für einen Phenyl- oder Naphtylrest steht, wobei diese verschiedenen Reste gegebenenfalls mit einem oder mehreren Halogen, Alkyl, Alkoxy, -CO-alk, Cyano, -CONR₁₃R₁₄, -alk-NR₁₅R₁₆, -NR₁₅R₁₆, CF₃, OCF₃, SO₂NH₂, Hydroxy oder Hydroxyalkyl substituiert sind,
Het für einen Heterocyclus steht, der aus Benzimidazol, Benzoxazol, Benzothiazol, Benzothiophen, Thiophen, Chinazolin, Chinoxalin, Chinolin, Pyrrol, Pyridin, Imidazol, Indol, Isochinolin, Thiazol, Thiadiazol, Furan, Tetrahydroisochinolin und Tetrahydrochinolin ausgewählt ist, wobei diese Heterocyclen gegebenenfalls mit einem oder mehreren Alkyl, Alkoxy, Halogen, Alkoxycarbonyl, Oxo, Hydroxy, OCF₃ oder CF₃ substituiert sind,
R₁₃ und R₁₄, die gleich oder verschieden sind, für ein Wasserstoffatom oder einen Alkylrest stehen oder auch R₁₃ und R₁₄ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₁₅ und R₁₆, die gleich oder verschieden sind, für ein Wasserstoffatom oder einen Alkylrest stehen oder auch R₁₅ und R₁₆ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
die optischen Isomere dieser Verbindungen und ihre pharmazeutisch annehmbaren Salze mit einer anorganischen oder organischen Säure.

5. Zusammensetzung nach Anspruch 1, wobei die Verbindung der Formel (I) aus folgenden Verbindungen ausgewählt ist:
N-{1-[Bis-(4-chlorphenyl)methyl]-azetidin-3-yl}-N-(6-chlorpyrid-2-yl)-methylsulfonamid,
N-{1-[Bis-(4-chlorphenyl)methyl]azetidin-3-yl}-N-(6-ethylpyrid-2-yl)-methylsulfonamid,
N-{1-[Bis-(4-chlorphenyl)methyl]azetidin-3-yl}-N-chinol-6-yl-methylsulfonamid,
N-{1-[Bis-(4-chlorphenyl)methyl]azetidin-3-yl}-N-chinol-5-yl-methylsulfonamid,
N-{1-[Bis-(4-chlorphenyl)methyl]azetidin-3-yl}-N-isochinol-5-yl-methylsulfonamid,
N-{1-[Bis-(4-chlorphenyl)methyl]azetidin-3-yl}-N-pyrid-3-yl-methylsulfonamid,
N-{1-[Bis-(4-chlorphenyl)methyl]azetidin-3-yl}-N-(1-oxid-pyrid-3-yl)methylsulfonamid,
N-(1R,2S,4S)-Bicyclo[2,2,1]hept-2-yl-N-{1-[bis-(4-chlorphenyl)methyl]azetidin-3-yl}-methylsulfonamid,
N-(1R,2R,4S)-Bicyclo[2,2,1]hept-2-yl-N-{1-[bis-(4-chlorphenyl)methyl]azetidin-3-yl}-methylsulfonamid,
N-{1-[Bis-(4-chlorphenyl)methyl]azetidin-3-yl}-N-(3,5-difluorphenyl)-methylsulfonamid,
N-{1-[Bis-(4-chlorphenyl)methyl]azetidin-3-yl}-N-(thiazol-2-yl)-methylsulfonamid,
N-{1-[Bis-(4-chlorphenyl)-methyl]-azetidin-3-yl}-N-(3-methoxyphenyl)-methylsulfonamid,
N-{1-[Bis-(4-chlorphenyl)-methyl]-azetidin-3-yl}-N-(3-hydroxyphenyl)-methylsulfonamid,
N-{1-[Bis-(4-chlorphenyl)-methyl]-azetidin-3-yl}-N-(3-hydroxymethylphenyl)-methylsulfonamid,
N-{1-[Bis-(4-chlorphenyl)-methyl]-azetidin-3-yl}-N-(methylsulfonyl)-3-ethylaminobenzoat,
N-{1-[Bis-(4-chlorphenyl)methyl]azetidin-3-yl}-N-(1-isobutylpiperid-4-yl)-methylsulfonamid,
N-Benzyl-N-{1-[bis(4-chlorphenyl)methyl]azetidin-3-yl}amin,
N-{1-[Bis(4-chlorphenyl)methyl]azetidin-3-yl}-N-(3,5-difluorbenzyl)amin,
N-{1-[Bis(4-chlorphenyl)methyl]azetidin-3-yl}-N-(3,5-difluorbenzyl)methylsulfonamid,
N-{1-[Bis(4-chlorphenyl)methyl]azetidin-3-yl}-N-(pyrid-3-yl-methyl)methylsulfonamid,
N-{1-[Bis-(4-fluorphenyl)-methyl]-azetidin-3-yl}-N-(3,5-difluorphenyl)methylsulfonamid,
(RS)-N-{1-[(4-Chlorphenyl)-pyrid-3-yl-methyl]-azetidin-3-yl}-N-(3,5-difluorphenyl)-methylsulfonamid,
(R)-N-{1-[(4-Chlorphenyl)-pyrid-3-yl-methyl]-azetidin-3-yl}-N-(3,5-difluorphenyl)-methylsulfonamid,
(S)-N-{1-[(4-Chlorphenyl)-pyrid-3-yl-methyl]-azetidin-3-yl}-N-(3,5-difluorphenyl)-methylsulfonamid,
(RS)-N-{1-[(4-Chlorphenyl)-pyrid-4-yl-methyl]-azetidin-3-yl}-N-(3,5-difluorphenyl)-methylsulfonamid,
(R)-N-{1-[(4-Chlorphenyl)-pyrid-4-yl-methyl]-azetidin-3-yl}-N-(3,5-difluorphenyl)-methylsulfonamid,
(S)-N-{1-[(4-Chlorphenyl)-pyrid-4-yl-methyl]-azetidin-3-yl}-N-(3,5-difluorphenyl)-methylsulfonamid,
(RS)-N-{1-[(4-Chlorphenyl)-pyrimidin-5-yl-methyl]-azetidin-3-yl}-N-(3,5-difluorphenyl)-methylsulfonamid,
(R)-N-{1-[(4-Chlorphenyl)-pyrimidin-5-yl-methyl]-azetidin-3-yl}-N-(3,5-difluorphenyl)-methylsulfonamid,
(S)-N-{1-[(4-Chlorphenyl)-pyrimidin-5-yl-methyl]-azetidin-3-yl}-N-(3,5-difluorphenyl)-methylsulfonamid,
N-{1-[Bis-(4-chlorphenyl)methyl]azetidin-3-yl}-N-(3,5-difluorphenyl)-benzylsulfonamid,
ihre optischen Isomere und ihre pharmazeutisch annehmbaren Salze mit einer anorganischen oder organischen Säure.

6. Verbindung der Formel: 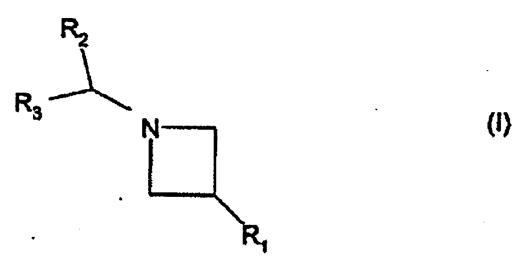 worin
R₁ für einen Rest -N(R₄)R₅, -N(R₄)-CO-R₅, -N(R₄)-SO₂R₆ steht,
R₂ und R₃, die gleich oder verschieden sind, entweder für einen Aromaten stehen, der aus Phenyl, Naphtyl und Indenyl ausgewählt ist, wobei diese Aromaten unsubstituiert oder mit einem oder mehreren Halogen, Alkyl, Alkoxy, Formyl, Hydroxy, Trifluormethyl, Trifluormethoxy, -CO-alk, Cyano, -COOH, COOalk, -CONR₇R₈, -CO-NH-NR₉R₁₀, Alkylsulfanyl, Alkylsulfinyl, Alkylsulfonyl, Alkylsulfanylalkyl, Alkylsulfinylalkyl, Alkylsulfonylalkyl, Hydroxyalkyl oder -alk-NR₇R₈ substituiert sind; oder für einen Heteroaromaten, der aus den Ringen Benzofuryl, Benzothiazolyl, Benzothienyl, Benzoxazolyl, Chromanyl, 2,3-Dihydrobenzofuryl, 2,3-Dihydrobenzothienyl, Furyl, Imidazolyl, Isochromanyl, Isochinolyl, Pyrrolyl, Pyridyl, Pyrimidyl, Chinolyl, 1,2,3,4-Tetrahydroisochinolyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, Cyano, -COOH, COOalk, -CO-NH-NR₉R₁₀, -CONR₇R₈, -alk-NR₉R₁₀, Alkylsulfanyl, Alkylsulfinyl, Alkylsulfonyl, Alkylsulfanylalkyl, Alkylsulfinylalkyl, Alkylsulfonylalkyl oder Hydroxyalkyl substituiert sein können,
R₄ für einen Rest -C(R₁₁) (R₁₂)-Het, -Het, -C(R₁₁)(R₁₂)-Ar, Ar, Cycloalkyl oder Norbornyl steht,
R₅ für ein Wasserstoffatom oder einen Rest Hydroxyalkyl, -alk-COOalk, -alk-CONR₇R₈, -alk-NR₇R₈, Alkoxy, Ar, Het, -CH₂Ar, -CH₂Het oder Alkyl steht, der gegebenenfalls mit einem oder mehreren Halogen substituiert ist,
R₆ für einen Rest Hydroxyalkyl, -alk-COOalk, -alk-CONR₇R₈, -alk-NR₇R₈, Alkoxy, Ar, Het, -CH₂Ar, -CH₂Het oder Alkyl steht, der gegebenenfalls mit 1 oder mehreren Halogen substituiert ist,
R₇ und R₈, die gleich oder verschieden sind, für ein Wasserstoffatom oder einen Alkylrest stehen oder auch R₇ und R₈ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₉ und R₁₀, die gleich oder verschieden sind, für ein Wasserstoffatom oder einen Rest Alkyl, -COOalk, Cycloalkyl, Alkylcycloalkyl, -alk-O-alk, Hydroxyalkyl stehen oder auch R₉ und R₁₀ zusammen mit dem Stickstoffatom, an das sie gebunden sind, einen gesättigten oder ungesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl, -COalk, -COOalk, -CO-NHalk, -CS-NHalk, Oxo, Hydroxyalkyl, -alk-O-alk oder -CO-NH₂ substituiert ist,
R₁₁ für ein Wasserstoffatom oder einen Rest Hydroxyalkyl, -alk-COOalk, -alk-CONR₇R₈, -alk-NR₇R₈, Alkoxyalkyl, Ar, Het, -CH₂Ar, -CH₂Het oder Alkyl steht, der gegebenenfalls mit einem oder mehreren Halogen substituiert ist,
R₁₂ für ein Wasserstoffatom oder einen Rest Hydroxyalkyl, -alk-COOalk, -alk-CONR₇R₈, -alk-NR₇R₈, Alkoxyalkyl oder Alkyl steht, der gegebenenfalls mit einem oder mehreren Halogen substituiert ist,
oder auch R₁₁ und R₁₂ zusammen mit dem Kohlenstoffatom, an das sie gebunden sind, einen gesättigten mono- oder bicyclischen Ring mit 3 bis 10 Ringgliedern bilden, der gegebenenfalls ein anderes Heteroatom, ausgewählt aus Sauerstoff, Schwefel und Stickstoff, enthält und der gegebenenfalls mit einem oder mehreren Alkyl substituiert ist,
Ar für einen Phenyl-, Naphtyl- oder Indenylrest steht, wobei diese verschiedenen Reste gegebenenfalls mit einem oder mehreren Halogen, Alkyl, Alkoxy, -CO-alk, Cyano, -COOH, -COOalk, -CONR₁₃R₁₄. -CO-NH-NR₁₅R₁₆, Alkylsulfanyl, Alkylsulfinyl, Alkylsulfonyl, -alk-NR₁₅R₁₆, -NR₁₅R₁₆, Alkylthioalkyl, Formyl, CF₃, OCF₃, Het, -O-alk-NH-cycloalkyl, SO₂NH₂, Hydroxy, Hydroxyalkyl, -NHCOalk, NHCOOalk oder an 2 benachbarten Kohlenstoffatomen mit Dioxymethylen substituiert sind,
Het für einen ungesättigten oder gesättigten, mono- oder bicyclischen Heterocyclus steht, der 3 bis 10 Ringglieder besitzt und ein oder mehrere Heteroatome, ausgewählt aus Sauerstoff, Schwefel und Stickstoff, enthält und der gegebenenfalls mit einem oder mehreren Alkyl, Alkoxy, Halogen, Alkoxycarbonyl, Oxo, Hydroxy substituiert ist, wobei die stickstoffhaltigen Heterocyclen gegebenenfalls in ihrer N-oxidierten Form vorliegen,
R₁₃ und R₁₄, die gleich oder verschieden sind, für ein Wasserstoffatom oder einen Alkylrest stehen oder auch R₁₃ und R₁₄ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff enthält und der gegebenenfalls mit einem oder mehreren Alkyl substituiert ist,
R₁₅ und R₁₆, die gleich oder verschieden sind, für ein Wasserstoffatom oder einen Alkylrest stehen oder auch R₁₅ und R₁₆ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
alk für einen Alkyl- oder Alkylenrest steht,
wobei die Alkyl- und Alkylenreste und -anteile und die Alkoxyreste und -anteile geradkettig oder verzweigt sind und 1 bis 6 Kohlenstoffatome enthalten und die Cycloalkylreste 3 bis 10 Kohlenstoffatome enthalten,
die optischen Isomere dieser Verbindungen und ihre pharmazeutisch annehmbaren Salze mit einer anorganischen oder organischen Säure,
mit Ausnahme der Verbindung, worin R₂ und R₃ für Phenylreste stehen, R₁ für einen Rest -N(R₄)SO₂R₆ steht, worin R₄ für einen Phenylrest steht und R₆ für einen Methylrest steht.

7. Verbindung der Formel (I) nach Anspruch 6, worin Het aus Benzimidazol, Benzoxazol, Benzothiazol, Benzothiophen, Cinnolin, Thiophen, Chinazolin, Chinoxalin, Chinolin, Pyrazol, Pyrrol, Pyridin, Imidazol, Indol, Isochinolin, Pyrimidin, Thiazol, Thiadiazol, Piperidin, Piperazin, Triazol, Furan, Tetrahydroisochinolin, Tetrahydrochinolin ausgewählt ist, wobei diese Heterocyclen gegebenenfalls mit einem oder mehreren Alkyl, Alkoxy, Halogen, Alkoxycarbonyl, Oxo, Hydroxy, OCF₃ oder CF₃ substituiert sind.

8. Verbindung der Formel (I) nach Anspruch 6, worin
R₁ für einen Rest -N(R₄)R₅, -N(R₄))-SO₂R₆ steht,
R₂ entweder für ein Phenyl steht, das unsubstituiert oder mit einem oder mehreren Halogen, Alkyl, Alkoxy, Trifluormethyl, Trifluormethoxy, -CO-alk, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈, -alk-NR₉R₁₀, Alkylsulfanyl, Alkylsulfinyl, Alkylsulfonyl oder Hydroxyalkyl substituiert sein können,
R₃ entweder für ein Phenyl steht, das unsubstituiert oder mit einem oder mehreren Halogen, Alkyl, Alkoxy, Trifluormethyl, Trifluormethoxy, -CO-alk, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈, -alk-NR₉R₁₀, Alkylsulfanyl, Alkylsulfinyl, Alkylsulfonyl oder Hydroxyalkyl substituiert sein können,
R₄ für einen Rest -C(R₁₁)(R₁₂)-Het, -Het, -C(R₁₁)(R₁₂)-Ar, Ar oder Norbornyl steht,
R₅ für ein Wasserstoffatom oder einen Rest Hydroxyalkyl, -alk-COOalk, -alk-CONR₇R₈, -alk-NR₇R₈, Alkoxy, -CH₂Ar, -CH₂Het oder Alkyl steht,
R₆ für einen Rest Hydroxyalkyl, -alk-COOalk, -alk-CONR₇R₈, -alk-NR₇R₈, Alkoxy, -CH₂Ar, -CH₂Het oder Alkyl steht,
R₇ und R₈, die gleich oder verschieden sind, für ein Wasserstoffatom oder einen Alkylrest stehen oder auch R₇ und R₈ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₉ und R₁₀, die gleich oder verschieden sind, für ein Wasserstoffatom oder einen Rest Alkyl, Cycloalkyl, Alkylcycloalkyl, -alk-O-alk oder Hydroxyalkyl stehen oder auch R₉ und R₁₀ zusammen mit dem Stickstoffatom, an das sie gebunden sind, einen gesättigten oder ungesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl, -COalk, -COOalk, -CO-NHalk, Oxo, Hydroxyalkyl oder -CO-NH₂ substituiert ist,
R₁₁ für ein Wasserstoffatom oder einen Rest Hydroxyalkyl, -alk-COOalk, -alk-CONR₇R₈, -alk-NR₇R₈, Alkoxyalkyl, Ar, Het, -CH₂Ar, -CH₂Het oder Alkyl steht, der gegebenenfalls mit einem oder mehreren Halogen substituiert ist,
R₁₂ für ein Wasserstoffatom oder einen Rest Hydroxyalkyl, -alk-COOalk, -alk-CONR₇R₈, -alk-NR₇R₈, Alkoxyalkyl oder Alkyl steht, der gegebenenfalls mit einem oder mehreren Halogen substituiert ist,
oder auch R₁₁ und R₁₂ zusammen mit dem Kohlenstoffatom, an das sie gebunden sind, einen gesättigten, mono- oder bicyclischen Ring mit 3 bis 10 Ringgliedern bilden, der gegebenenfalls ein anderes Heteroatom, ausgewählt aus Sauerstoff, Schwefel und Stickstoff, enthält und der gegebenenfalls mit einem oder mehreren Alkyl substituiert ist,
Ar für einen Phenyl- oder Naphtylrest steht, wobei diese verschiedenen Reste gegebenenfalls mit einem oder mehreren Halogen, Alkyl, Alkoxy, -CO-alk, Cyano, -CONR₁₃R₁₄, Alkylsulfonyl, -alk-NR₁₅R₁₆, -NR₁₅R₁₆, CF₃, OCF₃, SO₂NH₂, Hydroxy, Hydroxyalkyl oder an 2 benachbarten Kohlenstoffatomen mit Dioxymethylen substituiert sind,
Het für einen Heterocyclus steht, der aus Benzimidazol, Benzoxazol, Benzothiazol, Benzothiophen, Thiophen, Chinazolin, Chinoxalin, Chinolin, Pyrrol, Pyridin, Imidazol, Indol, Isochinolin, Pyrimidin, Thiazol, Thiadiazol, Furan, Tetrahydroisochinolin und Tetrahydrochinolin ausgewählt ist, wobei diese Heterocyclen gegebenenfalls mit einem oder mehreren Alkyl, Alkoxy, Halogen, Alkoxycarbonyl, Oxo, Hydroxy, OCF₃ oder CF₃ substituiert sind,
R₁₃ und R₁₄, die gleich oder verschieden sind, für ein Wasserstoffatom oder einen Alkylrest stehen oder auch R₁₃ und R₁₄ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₁₅ und R₁₆, die gleich oder verschieden sind, für ein Wasserstoffatom oder einen Alkylrest stehen oder auch R₁₅ und R₁₆ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
die optischen Isomere dieser Verbindungen und ihre pharmazeutisch annehmbaren Salze mit einer anorganischen oder organischen Säure,
mit Ausnahme der Verbindung, worin R₂ und R₃ für Phenylreste stehen, R₁ für einen Rest -N(R₄)SO₂R₆ steht, worin R₄ für einen Phenylrest steht und R₆ für einen Methylrest steht.

9. Verbindung der Formel (I) nach Anspruch 6, worin
R₁ für einen Rest -N(R₄)-SO₂R₆ steht,
R₂ entweder für ein Phenyl steht, das unsubstituiert oder mit einem oder mehreren Halogen, Alkyl, Alkoxy, Trifluormethyl, Trifluormethoxy, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈ oder Hydroxyalkyl substituiert sein können,
R₃ entweder für ein Phenyl steht, das unsubstituiert oder mit einem oder mehreren Halogen, Alkyl, Alkoxy, Trifluormethyl, Trifluormethoxy, Cyano, -CONR₇R₈, Hydroxyalkyl oder -alk-NR₇R₈ substituiert ist; oder für einen Heteroaromaten, der aus den Ringen Pyridyl, Pyrimidyl, Thiazolyl und Thienyl ausgewählt ist, wobei diese Heteroaromaten unsubstituiert oder mit einem Halogen, Alkyl, Alkoxy, Hydroxy, Trifluormethyl, Trifluormethoxy, -CONR₇R₈ oder Hydroxyalkyl substituiert sein können,
R₄ für -Het oder Ar steht,
R₆ für einen Hydroxyalkyl- oder Alkylrest steht,
R₇ und R₈, die gleich oder verschieden sind, für ein Wasserstoffatom oder einen Alkylrest stehen oder auch R₇ und R₈ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
Ar für einen Phenyl- oder Naphtylrest steht, wobei diese verschiedenen Reste gegebenenfalls mit einem oder mehreren Halogen, Alkyl, Alkoxy, -CO-alk, Cyano, -CONR₁₃R_{14,} -alk-NR₁₅R₁₆, -NR₁₅R₁₆, CF₃, OCF₃, SO₂NH₂, Hydroxy oder Hydroxyalkyl substituiert sind,
Het für einen Heterocyclus steht, der aus Benzimidazol, Benzoxazol, Benzothiazol, Benzothiophen, Thiophen, Chinazolin, Chinoxalin, Chinolin, Pyrrol, Pyridin, Imidazol, Indol, Isochinolin, Thiazol, Thiadiazol, Furan, Tetrahydroisochinolin und Tetrahydrochinolin ausgewählt ist, wobei diese Heterocyclen gegebenenfalls mit einem oder mehreren Alkyl, Alkoxy, Halogen, Alkoxycarbonyl, Oxo, Hydroxy, OCF₃ oder CF₃ substituiert sind,
R₁₃ und R₁₄, die gleich oder verschieden sind, für ein Wasserstoffatom oder einen Alkylrest stehen oder auch R₁₃ und R₁₄ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
R₁₅ und R₁₆, die gleich oder verschieden sind, für ein Wasserstoffatom oder einen Alkylrest stehen oder auch R₁₅ und R₁₆ zusammen mit dem Stickstoffatom, an das sie gebunden sind, einen gesättigten, mono- oder bicyclischen Heterocyclus mit 3 bis 10 Ringgliedern bilden, der gegebenenfalls ein weiteres Heteroatom, ausgewählt aus Sauerstoff, Schwefel und Stickstoff, enthält und der gegebenenfalls mit einem oder mehreren Alkyl substituiert ist,
die optischen Isomere dieser Verbindungen und ihre pharmazeutisch annehmbaren Salze mit einer anorganischen oder organischen Säure,
mit Ausnahme der Verbindung, worin R₂ und R₃ für Phenylreste stehen, R₁ für einen Rest -N(R₄)SO₂R₆ steht, worin R₄ für einen Phenylrest steht und R₆ für einen Methylrest steht.

10. Verbindung der Formel (I) nach Anspruch 6, die aus folgenden Verbindungen ausgewählt ist:
N-{1-[Bis-(4-chlorphenyl)methyl]-azetidin-3-yl}-N-(6-chlorpyrid-2-yl)-methylsulfonamid,
N-{1-[Bis-(4-chlorphenyl)methyl]azetidin-3-yl}-N-(6-ethylpyrid-2-yl)-methylsulfonamid,
N-{1-[Bis-(4-chlorphenyl)methyl]azetidin-3-yl}-N-chinol-6-yl-methylsulfonamid,
N-{1-[Bis-(4-chlorphenyl)methyl]azetidin-3-yl}-N-chinol-5-yl-methylsulfonamid,
N-{1-[Bis-(4-chlorphenyl)methyl]azetidin-3-yl}-N-isochinol-5-yl-methylsulfonamid,
N-{1-[Bis-(4-chlorphenyl)methyl]azetidin-3-yl}-N-pyrid-3-yl-methylsulfonamid,
N-{1-[Bis-(4-chlorphenyl)methyl]azetidin-3-yl}-N-(1-oxid-pyrid-3-yl)methylsulfonamid,
N-(1R,2S,4S)-Bicyclo[2,2,1]hept-2-yl-N-{1-[bis-(4-chlorphenyl)methyl]azetidin-3-yl}-methylsulfonamid,
N-(1R,2R,4S)-Bicyclo[2,2,1]hept-2-yl-N-{1-[bis-(4-chlorphenyl)methyl]azetidin-3-yl}-methylsulfonamid,
N-{1-[Bis-(4-chlorphenyl)methyl]azetidin-3-yl}-N-(3,5-difluorphenyl)-methylsulfonamid,
N-{1-[Bis-(4-chlorphenyl)methyl]azetidin-3-yl}-N-(thiazol-2-yl)-methylsulfonamid,
N-{1-[Bis-(4-chlorphenyl)-methyl]-azetidin-3-yl}-N-(3-methoxyphenyl)-methylsulfonamid,
N-{1-[Bis-(4-chlorphenyl)-methyl]-azetidin-3-yl}-N-(3-hydroxyphenyl)-methylsulfonamid,
N-{1-[Bis-(4-chlorphenyl)-methyl]-azetidin-3-yl}-N-(3-hydroxymethylphenyl)-methylsulfonamid,
N-{1-[Bis-(4-chlorphenyl)-methyl]-azetidin-3-yl}-N-(methylsulfonyl)-3-ethylaminobenzoat,
N-{1-[Bis-(4-chlorphenyl)methyl]azetidin-3-yl}-N-(1-isobutyl-piperid-4-yl)-methylsulfonamid,
N-Benzyl-N-{1-[bis(4-chlorphenyl)methyl]azetidin-3-yl}amin,
N-{1-[Bis(4-chlorphenyl)methyl]azetidin-3-yl}-N-(3,5-difluorbenzyl)amin,
N-{1-[Bis(4-chlorphenyl)methyl]azetidin-3-yl}-N-(3,5-difluorbenzyl)methylsulfonamid,
N-{1-[Bis(4-chlorphenyl)methyl]azetidin-3-yl}-N-(pyrid-3-yl-methyl)methylsulfonamid,
N-{1-[Bis-(4-fluorphenyl)-methyl]-azetidin-3-yl}-N-(3,5-difluorphenyl)methylsulfonamid,
(RS)-N-{1-[(4-Chlorphenyl)-pyrid-3-yl-methyl]-azetidin-3-yl}-N-(3,5-difluorphenyl)-methylsulfonamid,
(R)-N-{1-[(4-Chlorphenyl)-pyrid-3-yl-methyl]-azetidin-3-yl}-N-(3,5-difluorphenyl)-methylsulfonamid,
(S)-N-{1-[(4-Chlorphenyl)-pyrid-3-yl-methyl]-azetidin-3-yl}-N-(3,5-difluorphenyl)-methylsulfonamid,
(RS)-N-{1-[(4-Chlorphenyl)-pyrid-4-yl-methyl]-azetidin-3-yl}-N-(3,5-difluorphenyl)-methylsulfonamid,
(R)-N-{1-[(4-Chlorphenyl)-pyrid-4-yl-methyl]-azetidin-3-yl}-N-(3,5-difluorphenyl)-methylsulfonamid,
(S)-N-{1-[(4-Chlorphenyl)-pyrid-4-yl-methyl]-azetidin-3-yl}-N-(3,5-difluorphenyl)-methylsulfonamid,
(RS)-N-{1-[(4-Chlorphenyl)-pyrimidin-5-yl-methyl]-azetidin-3-yl}-N-(3,5-difluorphenyl)-methylsulfonamid,
(R)-N-{1-[(4-Chlorphenyl)-pyrimidin-5-yl-methyl]-azetidin-3-yl}-N-(3,5-difluorphenyl)-methylsulfonamid,
(S)-N-{1-[(4-Chlorphenyl)-pyrimidin-5-yl-methyl]-azetidin-3-yl}-N-(3,5-difluorphenyl)-methylsulfonamid,
N-{1-[Bis-(4-chlorphenyl)methyl]azetidin-3-yl}-N-(3,5-difluorphenyl)-benzylsulfonamid,
ihre optischen Isomere und ihre pharmazeutisch annehmbaren Salze mit einer anorganischen oder organischen Säure.

11. N-{1-[Bis-(4-chlorphenyl)methyl]azetidin-3-yl}-N-(3,5-difluorphenyl)-methylsulfonamid, seine optischen Isomere und seine pharmazeutisch annehmbaren Salze mit einer anorganischen oder organischen Säure.

12. Verfahren zur Herstellung der Verbindungen der Formel (I) nach Anspruch 6, worin R₁ für einen Rest -N(R₄)R₅ steht, worin R₅ ein Wasserstoffatom ist, R₄ ein Rest -CR₁₁R₁₂-Ar oder -CR₁₁R₁₂-Het ist und R₁₂ ein Wasserstoffatom ist, **dadurch gekennzeichnet, dass** man ein Derivat Rb-COR₁₁, worin R₁₁ die gleichen Bedeutungen wie im Anspruch 6 hat, mit einem Derivat der folgenden Formel umsetzt: 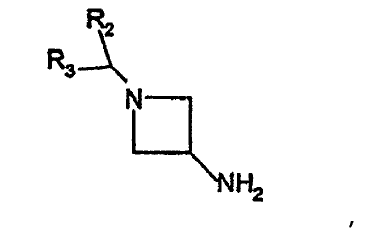 wobei Rb für einen Rest Ar oder Het steht, R₂, R₃, R₁₁, Ar und Het die gleichen Bedeutungen wie im Anspruch 6 haben, das Produkt isoliert und es gegebenenfalls in ein pharmazeutisch annehmbares Salz umwandelt.

13. Verfahren zur Herstellung der Verbindungen der Formel (I) nach Anspruch 6, worin R₁ für einen Rest -N(R₄)-CO-R₅ steht, worin R₄ ein Rest -C(R₁₁)(R₁₂)-Het oder -C(R₁₁)(R₁₂)-Ar ist und R₁₂ ein Wasserstoffatom ist, **dadurch gekennzeichnet, dass** man ein Derivat Hal-COR₅ mit einem Derivat der folgenden Formel umsetzt: 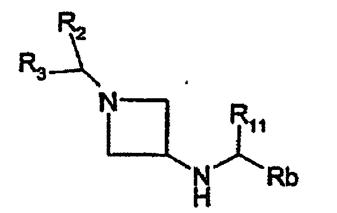 wobei Hal für ein Halogenatom steht, Rb für einen Rest Ar oder Het steht und R₂, R₃, R₅, R₁₁, Ar und Het die gleichen Bedeutungen wie im Anspruch 6 haben, das Produkt isoliert und es gegebenenfalls in ein pharmazeutisch annehmbares Salz umwandelt.

14. Verfahren zur Herstellung der Verbindungen der Formel (I) nach Anspruch 6, worin R₁ für einen Rest -N(R₄)-SO₂R₆ steht, worin R₄ ein Rest -C(R₁₁)(R₁₂)-Ar oder -C(R₁₁)(R₁₂)-Het ist und R₁₂ ein Wasserstoffatom ist, **dadurch gekennzeichnet, dass** man ein Derivat Hal-SO₂R₆ mit einem Derivat der folgenden Formel umsetzt: 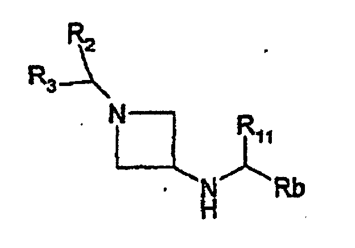 wobei R₂, R₃, R₁₁, R₆ die gleichen Bedeutungen wie im Anspruch 6 haben, Hal für ein Halogenatom steht und Rb für einen Rest Ar oder Het steht, das Produkt isoliert und es gegebenenfalls in ein pharmazeutisch annehmbares Salz umwandelt.

15. Verfahren zur Herstellung der Verbindungen der Formel (I) nach Anspruch 6, worin R₁ für einen Rest -N(R₄)R₅ steht, **dadurch gekennzeichnet, dass** man ein Derivat R₅(R₄)NH mit einem Derivat der folgenden Formel umsetzt: 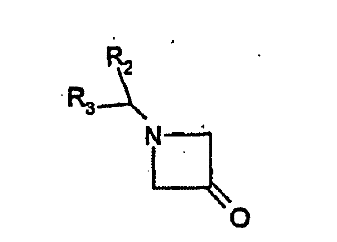 wobei R₂, R₃, R₄, R₅ die gleichen Bedeutungen wie im Anspruch 6 haben, das Produkt isoliert und es gegebenenfalls in ein pharmazeutisch annehmbares Salz umwandelt.

16. Verfahren zur Herstellung der Verbindungen der Formel (I) nach Anspruch 6, worin R₁ für einen Rest -N(R₄)SO₂R₆ steht, **dadurch gekennzeichnet, dass** man ein Derivat Hal-SO₂R₆ an einem Derivat der folgenden Formel umsetzt: 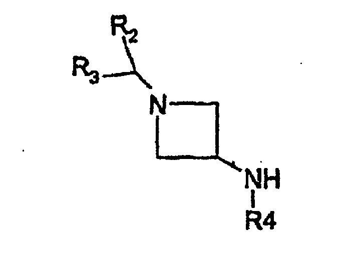 wobei R₂, R₃, R₄ und R₆ die gleichen Bedeutungen wie im Anspruch 6 haben und Hal für ein Halogenatom steht, das Produkt isoliert und es gegebenenfalls in ein pharmazeutisch annehmbares Salz umwandelt.

17. Verfahren zur Herstellung der Verbindungen der Formel (I) nach Anspruch 6, worin R₁ für einen Rest -N(R₄)COR₅ steht, **dadurch gekennzeichnet, dass** man ein Derivat Hal-COR₆ mit einem Derivat der folgenden Formel umsetzt: 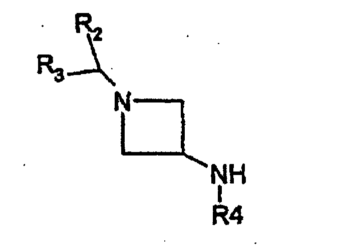 wobei R₂, R₃, R₄ und R₅ die gleichen Bedeutungen wie im Anspruch 6 haben und Hal für ein Halogenatom steht, das Produkt isoliert und es gegebenenfalls in ein pharmazeutisch annehmbares Salz umwandelt.

18. Verfahren zur Herstellung der Verbindungen der Formel (I) nach Anspruch 6, worin R₁ für einen Rest -N(R₄)-SO₂-R₆ steht, R₄ ein Rest Het oder Ar ist, **dadurch gekennzeichnet, dass** man ein Derivat Rd-NH-SO₂-R₆ mit einem Derivat der folgenden Formel umsetzt: 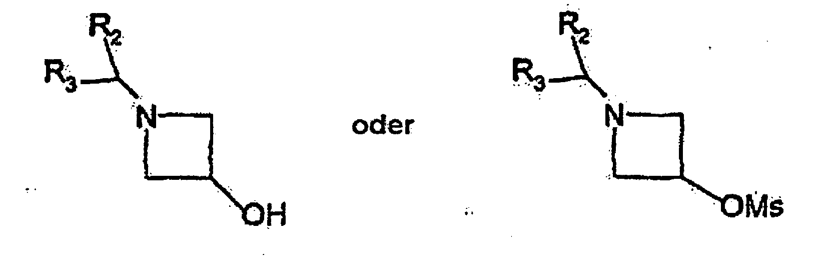 wobei Rd für einen Rest Ar oder Het steht, R₂, R₃ und R₆ die gleichen Bedeutungen wie im Anspruch 6 haben und Ms für einen Methylsulfonyloxyrest steht, das Produkt isoliert und es gegebenenfalls in ein pharmazeutisch annehmbares Salz umwandelt.

19. Verfahren zur Herstellung der Verbindungen der Formel (I) nach Anspruch 6, **dadurch gekennzeichnet, dass** man ein Derivat R₂-CHBr-R₃ mit einem Derivat der folgenden Formel umsetzt: 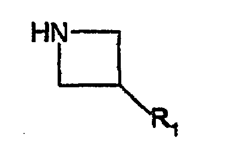 wobei R₁, R₂ und R₃ die gleichen Bedeutungen wie im Anspruch 6 haben, das Produkt isoliert und es gegebenenfalls in ein pharmazeutisch annehmbares Salz umwandelt.

20. Verfahren zur Herstellung der Verbindungen der Formel (I) nach Anspruch 6, worin R₁ für einen Rest -N(R₄)-SO₂-R₆ steht, worin R₄ ein Piperid-4-yl-Rest ist, der am Stickstoff mit einem Alkylrest substituiert ist, **dadurch gekennzeichnet, dass** man eine entsprechende Verbindung der Formel (I), worin R₁ für einen Rest -N(R₄)-SO₂-R₆ steht, worin R₄ ein Piperid-4-yl-Rest ist, alkyliert, das Produkt isoliert und es gegebenenfalls in ein pharmazeutisch annehmbares Salz umwandelt.

21. Verfahren zur Herstellung der Verbindungen der Formel (I) nach Anspruch 6, worin R₁ für einen Rest -N(R₄)-SO₂-R₆ steht, worin R₄ ein Phenylrest ist, der mit einem Pyrrolid-1-yl-Rest substituiert ist, **dadurch gekennzeichnet, dass** man Pyrrolidin an einer entsprechenden Verbindung der Formel (I), worin R₁ für einen Rest -N(R₄)SO₂R₆ steht, worin R₄ ein Phenylrest ist, der mit einem Halogenatom substituiert ist, umsetzt, das Produkt isoliert und es gegebenenfalls in ein pharmazeutisch annehmbares Salz umwandelt.

22. Arzneimittel, das als Wirkstoff mindestens eine Verbindung der Formel (I) nach einem der Ansprüche 6 bis 11 enthält.
